# EUROPEAN PATENT SPECIFICATION

(11) **EP 3 653 639 B1**
(45) Date of publication and mention of the grant of the patent: **09.11.2022**
(21) Application number: 18784747.0
(22) Date of filing: 09.04.2018
(51) Int. Cl.: C07K 14/415, A61K 38/16, A61P 3/10, A61P 3/06, A61K 31/198, A61K 45/06

(54) **POLYPEPTIDE AND COMPOSITION THEREOF FOR TREATING DISEASES OF METABOLIC SYSTEM**
POLYPEPTID UND ZUSAMMENSETZUNG DAVON ZUR BEHANDLUNG VON STOFFWECHSELKRANKHEITEN
POLYPEPTIDE ET COMPOSITION DE CELUI-CI POUR LE TRAITEMENT DE MALADIES DU SYSTÈME MÉTABOLIQUE

(30) Priority: 10.04.2017 CN 201710230019
(43) Date of publication of application: 20.05.2020
(73) Proprietor: Wuhan Grectech Co., Ltd., Wuhan, Hubei 430056 (CN)
(72) Inventor: ZHANG, Yufei, Wuhan Hubei 430056 (CN)
(74) Representative: Gille Hrabal Partnerschaftsgesellschaft mbB Patentanwälte
(86) International application number: PCT/CN2018/082401
(87) International publication number: WO 2018/188565

(56) References cited:
- CN-A- 1 762 485
- CN-A- 102 030 820
- US-A1- 2008 260 840
- US-B1- 6 277 619
- VANESSA EYRAUD ET AL: "Expression and Biological Activity of the Cystine Knot Bioinsecticide PA1b (Pea Albumin 1 Subunit b)", PLOS ONE, vol. 8, no. 12, 11 December 2013 (2013-12-11), page e81619, XP055747303, DOI: 10.1371/journal.pone.0081619

## Description

### FIELD OF THE INVENTION

The invention belongs to the field of biomedicine, and relates to a polypeptide and a composition thereof, in particular to a polypeptide and a composition thereof for treating diseases of metabolic system.

### BACKGROUND OF THE INVENTION

Diabetes is a comprehensive metabolic disease involving a disorder of the metabolism of carbohydrate, protein and fat, which is resulted from relative or absolute insufficiency of insulin secretion *in vivo,* the inability of insulin to play its normal physiological role in target cells, and/or excessive glucagon secretion, and characterized by long-term hyperglycemia in patients.

The main harm of diabetes is the complications. Long-term diabetes can damage multiple systems of the body, especially the cardiovascular system, leading to chronic progressive pathological changes in tissues including eyes, kidneys, nerves, heart, blood vessels, etc., causing functional defects and failures, such as cardiovascular and cerebrovascular atherosclerosis, retinal and renal microangiopathy, neuropathy and lower limb gangrene, etc. Diabetic retinopathy (DR) and diabetic nephropathy (DN) are common microvascular complications of diabetes.

According to the statistics from the World Health Organization, diabetes is a main cause of blindness, kidney failure, heart attack, stroke and lower limb amputation. At present, diabetes has become the third leading cause of death in the elderly besides heart disease and cancer.

Currently, the main anti-diabetic agents are insulin, oral hypoglycemic agents, incretin, etc. The existing agents have obvious drawbacks; for example, metformin easily leads to lacticacidemia, thiazolidinediones have side-effects such as hepatotoxicity and bone loss, etc., sulfonylureas when taken for long term causes hypoglycemia easily, DD-IV inhibitors when taken for long term induces cough easily, GLP-1 easily causes nausea and vomit in patients, and insulin has a series of problems such as incapable oral administration, inconvenient storage and use, hypoglycemic reaction in patients, and insulin resistance and lipatrophy when taken for long term.

At present, all kinds of hypoglycemic agents used in clinic have various defects, and cannot cure diabetes completely, and the patients need lifelong medication. Therefore, it is urgent to develop new, safe and effective hypoglycemic agents, especially convenient for oral administration, so as to meet the needs of increasing patients with diabetes worldwide.

Meanwhile, as diabetes is a metabolic disease involving a disorder of three major metabolic systems of carbohydrates, fats and proteins caused by absolute or relative insufficiency of insulin secretion, patients with diabetes are often accompanied with hyperlipidemia, characterized by relatively high levels of cholesterol, triglyceride and low-density lipoprotein, or a relatively low level of high-density lipoprotein.

Diabetes is closely associated with hyperlipidemia. On the one hand, when insulin is insufficient, the activity of lipase in diabetic patients is also reduced and blood lipids are elevated; moreover, besides glucose metabolic disorder, diabetes is also accompanied by disorders of fats, proteins, water and electrolytes, and thus free fatty acids are often released from adipose depots, thereby increasing the triglyceride and free fatty acid levels in the blood; furthermore, people with type 2 diabetes eat too much, which promotes the synthesis of lipids in the body and also increases the blood lipids. On the other hand, as obese individuals with hyperlipidemia have relatively decreased numbers of insulin receptors, they have insulin resistance, which in turn easily induces diabetes.

Hyperlipidemia is a secondary disease of diabetes, which can easily lead to cardiovascular and cerebrovascular complications. According to statistics, about 40% of diabetic patients have hyperlipidemia, and diabetes combined with hyperlipidemia is more likely to develop stroke, coronary heart disease, limb necrosis, fundus diseases, kidney diseases, neuropathy and the like. Therefore, it is necessary to develop new medicaments to lower both blood glucose and blood lipids in patients with diabetes.

The publication "Expression and Biological Activity of the Cystine Knot Bioinsecticide PAlb (Pea Albumin 1 Subunit b)", by Vanessa Eyraud et al, which was published on December 11, 2013 (2013-12-11) in PLoS ONE, vol. 8, not 12, page e81619 discloses polypeptides falling within formula (I) claimed in the present application. This publication also discloses their biological activity as insectides.

The publication US 2008/260840 A discloses a suspension formulation comprising (i) a particle formulation comprising the insulinotropic peptide GLP-1, a disaccharide, methionine, and a buffer, and (ii) a non-aqueous, single-phase suspension vehicle comprising one or more pyrrolidone polymer and one or more solvent selected from the group consisting of lauryl lactate, lauryl alcohol, benzyl benzoate, and mixtures thereof, wrerein the suspension vehicle exhibits viscous fluid characteristics, and the particle formulation is dispersed in the vehicle, for use in the treatment of diabetes and hyperlipidemia

The publication CN 102 030 820 A discloses polypeptides similar to the ones of the current invention for use in the treatment of diabetes and disorders of lipid metabolism.

### SUMMARY OF THE INVENTION

The invention provides a polypeptide and a composition thereof, which have excellent hypoglycemic and hypolipidemic activities and are resistance to enzymatic hydrolysis by pepsin and trypsin at the same time, so that they can be administered either by injection or orally. The polypeptide and the composition thereof also have the activity of protecting and repairing the function of pancreatic β cells, without causing hypoglycemic reaction and insulin resistance in patients, have no toxic side-effects, are convenient and safe to be administered, have long-term storage stability, and are a new type of hypoglycemic and hypolipidemic agents.

The inventor of the invention has found that natural polypeptides composed of multiple amino acids extracted from leguminous plants and the polypeptides with similar structures have excellent hypoglycemic activities. Further studies have revealed that the polypeptides also have excellent hypolipidemic activity, and recent studies have further revealed that better hypoglycemic and hypolipidemic effects can be achieved when these polypeptides were used in combination with the amino acid methionine. Thus, the invention was completed.

According to one aspect of the invention, the invention relates to a method for lowering blood glucose and/or blood lipid level in a mammal, characterized in that a therapeutically effective amount of one polypeptide of the sequence as shown in the following general formula (I) or a mixture of two or more polypeptides of the sequences as shown in the following general formula (I) in any proportion is administered to the mammal:

X₁SCNGVCSPFX₂X₃PPCGX₄X₅X₆CRCIPX₇GLX₈X₉GX₁₀CRNPX₁₁G (I)

wherein, X₁-X₁₁ is independently selected from the group consisting of A, C, D, E, F, G, H, I, K, L, M, N, P, Q, R, S, T, V, W, Y and Mₒₓ.

According to another aspect of the invention, the invention relates to a method for preventing and/or treating diabetes and/or hyperlipidemia in a mammal, characterized in that a therapeutically effective amount of one polypeptide of the sequence as shown in general formula (I) or a mixture of two or more polypeptides of the sequences as shown in general formula (I) in any proportion is administered to the mammal.

According to another aspect of the invention, the invention relates to a method for lowering blood glucose and/or blood lipid level in a mammal, characterized in that a therapeutically effective amount of one polypeptide of the sequence as shown in general formula (I) or a mixture of two or more polypeptides of the sequences as shown in general formula (I) in any proportion is administered to the mammal in combination with other hypoglycemic agents and/or hypolipidemic agents.

According to another aspect of the invention, the invention relates to a method for preventing and/or treating diabetes and/or hyperlipidemia in a mammal, characterized in that a therapeutically effective amount of one polypeptide of the sequence as shown in general formula (I) or a mixture of two or more polypeptides of the sequences as shown in general formula (I) in any proportion is administered to the mammal in combination with other hypoglycemic agents and/or hypolipidemic agents.

According to another aspect of the invention, the invention relates to the use of one polypeptide of the sequence as shown in general formula (I) or a mixture of two or more polypeptides of the sequences as shown in general formula (I) in any proportion in the manufacture of a medicament, food, health food or formula food for special medical purposes, for lowering blood glucose and/or blood lipid level in a mammal.

According to another aspect of the invention, the invention relates to the use of one polypeptide of the sequence as shown in general formula (I) or a mixture of two or more polypeptides of the sequences as shown in general formula (I) in any proportion in the manufacture of a medicament for preventing and/or treating diabetes and/or hyperlipidemia in a mammal.

According to another aspect of the invention, the invention relates to the use of one polypeptide of the sequence as shown in general formula (I) or a mixture of two or more polypeptides of the sequences as shown in general formula (I) in any proportion in the manufacture of a medicament, food, health food or formula food for special medical purposes, for lowering blood glucose and/or blood lipid level in a mammal in combination with other hypoglycemic agents and/or hypolipidemic agents.

According to another aspect of the invention, the invention relates to the use of one polypeptide of the sequence as shown in general formula (I) or a mixture of two or more polypeptides of the sequences as shown in general formula (I) in any proportion in the manufacture of a medicament for preventing and/or treating diabetes and/or hyperlipidemia in a mammal in combination with other hypoglycemic agents and/or hypolipidemic agents.

According to another aspect of the invention, the invention relates to an oral pharmaceutical composition or preparation, food, health food or formula food for special medical purposes, comprising one polypeptide of the sequence as shown in general formula (I) or a mixture of two or more polypeptides of the sequences as shown in general formula (I) in any proportion and pharmaceutically acceptable carriers.

According to another aspect of the invention, the invention relates to a pharmaceutical composition or preparation for intravenous, intramuscular or subcutaneous injection, comprising one polypeptide of the sequence as shown in general formula (I) or a mixture of two or more polypeptides of the sequences as shown in general formula (I) in any proportion and pharmaceutically acceptable carriers.

According to another aspect of the invention, the invention relates to a composition, comprising one polypeptide of the sequence as shown in general formula (I) or a mixture of two or more polypeptides of the sequences as shown in general formula (I) in any proportion and at least the amino acid methionine.

According to one aspect of the invention, the invention relates to a method for lowering blood glucose and/or blood lipid level in a mammal, characterized in that a therapeutically effective amount of the above composition is administered to the mammal.

According to another aspect of the invention, the invention relates to a method for preventing and/or treating diabetes and/or hyperlipidemia in a mammal, characterized in that a therapeutically effective amount of the above composition is administered to the mammal.

According to one aspect of the invention, the invention relates to a method for lowering blood glucose and/or blood lipid level in a mammal, characterized in that a therapeutically effective amount of the above composition is administered to the mammal in combination with other hypoglycemic agents and/or hypolipidemic agents.

According to another aspect of the invention, the invention relates to a method for preventing and/or treating diabetes and/or hyperlipidemia in a mammal, characterized in that a therapeutically effective amount of the above composition is administered to the mammal in combination with other hypoglycemic agents and/or hypolipidemic agents.

According to another aspect of the invention, the invention relates to the use of the above composition in the manufacture of a medicament, food, health food or formula food for special medical purposes, for lowering blood glucose and/or blood lipid level in a mammal.

According to another aspect of the invention, the invention relates to the use of the above composition in the manufacture of a medicament for preventing and/or treating diabetes and/or hyperlipidemia in a mammal.

According to another aspect of the invention, the invention relates to the use of the above composition in the manufacture of a medicament, food, health food or formula food for special medical purposes, for lowering blood glucose and/or blood lipid level in a mammal in combination with other hypoglycemic agents and/or hypolipidemic agents.

According to another aspect of the invention, the invention relates to the use of the above composition in the manufacture of a medicament for preventing and/or treating diabetes and/or hyperlipidemia in a mammal in combination with other hypoglycemic agents and/or hypolipidemic agents.

Preferably, the mammal, such as a diabetic patient has a glycosylated hemoglobin value >7.0%, preferably >7.5% before the administration of the polypeptide or the composition thereof of the invention, and has glycosylated hemoglobin value <7.5%, preferably <7.0% after the administration of the polypeptide or the composition thereof of the invention.

According to another aspect of the invention, the invention relates to an oral pharmaceutical preparation, food, health food or formula food for special medical purposes, comprising the above composition and pharmaceutically acceptable carriers.

According to another aspect of the invention, the invention relates to a pharmaceutical composition or preparation for intravenous, intramuscular or subcutaneous injection, comprising the above composition and pharmaceutically acceptable carriers.

In a preferred embodiment, the polypeptide of the invention has the sequences as shown in general formula (I). Preferably, X₁ is a hydrophobic amino acid, selected from the group consisting of A, L, V, I, P, F, M, W, and Mₒₓ; X₂ is an acidic amino acid, selected from E, and D; X₃ is a hydrophobic amino acid, selected from the group consisting of A, L, V, I, P, F, M, W, and Mₒₓ; X₄ is a polar neutral amino acid except cysteine, selected from the group consisting of G, S, T, Y, N, and Q; X₅ is a neutral amino acid except cysteine, selected from the group consisting of G, S, T, Y, N, Q, A, L, V, I, P, F, M, and W; X₆ is a hydrophobic amino acid, selected from the group consisting of A, L, V, I, P, F, M, W, and Mₒₓ; X₇ is a neutral amino acid except cysteine, selected from the group consisting of G, S, T, Y, N, Q, A, L, V, I, P, F, M, and W; X₈ is a hydrophobic amino acid, selected from the group consisting of A, L, V, I, P, F, M, W, and Mₒₓ; X₉ is a hydrophobic amino acid, selected from the group consisting of A, L, V, I, P, F, M, W, and Mₒₓ; X₁₀ is selected from Y, K, and N; and X₁₁ is a polar neutral amino acid except cysteine, selected from the group consisting of G, S, T, Y, N, and Q. More preferably, X₁ is A, I or V; X₂ is E or D; X₃ is M, Mₒₓ or I; X₄ is S or T; X₅ is S or P; X₆ is A or L; X₇ is V, A or Y; X₈ is V, F or L; X₉ is V or I; X₁₀ is Y, K or N; and X₁₁ is S, T or Y. More preferably, X₂X₃ is one selected from the group consisting of DI, EI, DM, EM, DMₒₓ, and EMₒₓ; X₄X₅X₆ is one selected from the group consisting of SPL, SPA, SSL, SSA, TPL, TPA, TSL and TSA; and X₈X₉ is one selected from the group consisting of VI, VV, FI, FV, LI, and LV. More preferably, the polypeptide of the invention is one having the following sequence or a mixture of two or more polypeptides having the following sequences in any proportion:
SEQ ID No.1: ASCNGVCSPFEMPPCGTSACRCIPVGLVVGYCRNPSG,
SEQ ID No.2: ASCNGVCSPFEMₒₓPPCGTSACRCIPVGLVVGYCRNPSG,
SEQ ID No.3: ASCNGVCSPFEMPPCGTSACRCIPVGLVIGYCRNPSG,
SEQ ID No.4: ASCNGVCSPFEMₒₓPPCGTSACRCIPVGLVIGYCRNPSG,
SEQ ID No.5: ASCNGVCSPFEMPPCGSSACRCIPVGLLIGYCRNPSG,
SEQ ID No.6: ASCNGVCSPFEMₒₓPPCGSSACRCIPVGLLIGYCRNPSG,
SEQ ID No.7: ASCNGVCSPFEMPPCGTSACRCIPVGLFIGYCRNPSG,
SEQ ID No.8: ASCNGVCSPFEMₒₓPPCGTSACRCIPVGLFIGYCRNPSG,
SEQ ID No.9: ASCNGVCSPFEMPPCGTSACRCIPYGLFIGYCRNPSG,
SEQ ID No.10: ASCNGVCSPFDIPPCGSPLCRCIPVGLVIGKCRNPYG,
SEQ ID No.11: ISCNGVCSPFDIPPCGSPLCRCIPAGLVIGNCRNPYG,
SEQ ID No.12: VSCNGVCSPFDIPPCGSPLCRCIPAGLVIGKCRNPYG,
SEQ ID No.13: ASCNGVCSPFDIPPCGTPLCRCIPVGLVIGNCRNPYG,
SEQ ID No.14: ASCNGVCSPFDMPPCGSSACRCIPVGLFIGNCRNPYG,
SEQ ID No.15: ASCNGVCSPFDMPPCGSSACRCIPVGLVIGYCRNPYG,
SEQ ID No.16: ASCNGVCSPFDMPPCGSSLCRCIPAGLVVGYCRNPSG,
SEQ ID No.17: ASCNGVCSPFDMPPCGTSACRCIPAGLFIGKCRNPYG,
SEQ ID No.18: ASCNGVCSPFDMPPCGTSACRCIPVGLVVGYCRNPSG,
SEQ ID No.19: ASCNGVCSPFDMPPCGTSLCRCIPAGLFIGYCRNPSG,
SEQ ID No.20: ASCNGVCSPFDMPPCGTSLCRCIPAGLVIGYCRNPYG,
SEQ ID No.21: ASCNGVCSPFEIPPCGSPLCRCIPVGLVIGNCRNPYG,
SEQ ID No.22: ASCNGVCSPFEIPPCGTPLCRCIPAGLVIGKCRNPYG,
SEQ ID No.23: ASCNGVCSPFEIPPCGTPLCRCIPVGLFIGKCRNPSG,
SEQ ID No.24: ASCNGVCSPFEMPPCGSSACRCIPAGLFIGKCRNPYG,
SEQ ID No.25: ASCNGVCSPFEMPPCGSSACRCIPVGLFVGYCRNPYG,
SEQ ID No.26: ASCNGVCSPFEMPPCGSSACRCIPVGLVVGYCRNPSG,
SEQ ID No.27: ASCNGVCSPFEMPPCGSSLCRCIPAGLFIGYCRNPSG,
SEQ ID No.28: ASCNGVCSPFEMPPCGSSLCRCIPAGLVIGYCRNPSG,
SEQ ID No.29: ASCNGVCSPFEMPPCGTSACRCIPAGLFIGNCRNPYG,
SEQ ID No.30: ASCNGVCSPFEMPPCGTSACRCIPAGLVIGYCRNPYG,
SEQ ID No.31: ISCNGVCSPFDIPPCGSPACRCIPVGLVIGKCRNPYG,
SEQ ID No.32: ISCNGVCSPFDIPPCGSPLCRCIPAGLVIGNCRNPSG,
SEQ ID No.33: ISCNGVCSPFDIPPCGSPLCRCIPVGLFIGNCRNPSG,
SEQ ID No.34: ISCNGVCSPFDIPPCGSPLCRCIPVGLVIGYCRNPSG,
SEQ ID No.35: ISCNGVCSPFDIPPCGTPACRCIPVGLVIGNCRNPYG,
SEQ ID No.36: ISCNGVCSPFDIPPCGTPLCRCIPAGLFVGKCRNPYG,
SEQ ID No.37: ISCNGVCSPFDIPPCGTPLCRCIPAGLFIGKCRNPSG,
SEQ ID No.38: ISCNGVCSPFDMPPCGSPACRCIPAGLLIGYCRNPSG,
SEQ ID No.39: ISCNGVCSPFDMPPCGTSACRCIPAGLVIGYCRNPSG,
SEQ ID No.40: ISCNGVCSPFEIPPCGSPLCRCIPAGLFIGKCRNPSG,
SEQ ID No.41: ISCNGVCSPFEIPPCGTPACRCIPVGLFIGKCRNPSG,
SEQ ID No.42: ISCNGVCSPFEIPPCGTPACRCIPAGLVIGKCRNPYG,
SEQ ID No.43: ISCNGVCSPFEIPPCGSPACRCIPVGLVIGNCRNPYG,
SEQ ID No.44: ISCNGVCSPFEIPPCGSSLCRCIPAGLLVGKCRNPSG,
SEQ ID No.45: ISCNGVCSPFEIPPCGTPLCRCIPAGLFIGNCRNPSG,
SEQ ID No.46: ISCNGVCSPFEIPPCGTPLCRCIPAGLVIGYCRNPSG,
SEQ ID No.47: ISCNGVCSPFEIPPCGTSACRCIPAGLVIGYCRNPSG,
SEQ ID No.48: ISCNGVCSPFEMPPCGSSACRCIPAGLVIGYCRNPSG,
SEQ ID No.49: ISCNGVCSPFEMPPCGSSACRCIPAGLFIGYCRNPSG,
SEQ ID No.50: ISCNGVCSPFEMPPCGTSLCRCIPAGLVVGYCRNPSG,
SEQ ID No.51: VSCNGVCSPFDIPPCGTPLCRCIPYGLFVGNCRNPYG,
SEQ ID No.52: VSCNGVCSPFDIPPCGTPACRCIPYGLFVGNCRNPYG,
SEQ ID No.53: VSCNGVCSPFDIPPCGTPLCRCIPAGLVIGNCRNPYG,
SEQ ID No.54: VSCNGVCSPFDIPPCGTPLCRCIPVGLFIGNCRNPSG,
SEQ ID No.55: VSCNGVCSPFDIPPCGTPLCRCIPVGLVIGYCRNPSG,
SEQ ID No.56: VSCNGVCSPFDIPPCGSPLCRCIPVGLFIGKCRNPSG,
SEQ ID No.57: VSCNGVCSPFDMPPCGSSACRCIPAGLFIGNCRNPYG,
SEQ ID No.58: VSCNGVCSPFDMPPCGSSACRCIPAGLVIGYCRNPYG,
SEQ ID No.59: VSCNGVCSPFDMPPCGSSACRCIPVGLVIGYCRNPSG,
SEQ ID No.60: VSCNGVCSPFDMPPCGSSACRCIPVGLFIGYCRNPSG,
SEQ ID No.61: VSCNGVCSPFDMPPCGTSACRCIPAGLFIGYCRNPSG,
SEQ ID No.62: VSCNGVCSPFEIPPCGSPACRCIPVGLVIGKCRNPYG,
SEQ ID No.63: VSCNGVCSPFEIPPCGSPLCRCIPAGLVIGNCRNPYG,
SEQ ID No.64: VSCNGVCSPFEIPPCGSPLCRCIPVGLVIGYCRNPSG,
SEQ ID No.65: VSCNGVCSPFEIPPCGTPACRCIPVGLVIGNCRNPYG,
SEQ ID No.66: VSCNGVCSPFEIPPCGSPLCRCIPVGLFIGNCRNPSG,
SEQ ID No.67: VSCNGVCSPFEIPPCGTPLCRCIPAGLFIGKCRNPSG,
SEQ ID No.68: VSCNGVCSPFEMPPCGSSACRCIPYGLVVGNCRNPSG,
SEQ ID No.69: VSCNGVCSPFEMPPCGTPLCRCIPYGLLIGKCRNPYG,
SEQ ID No.70: VSCNGVCSPFEMPPCGTSACRCIPAGLVIGYCRNPSG,
SEQ ID No.71: VSCNGVCSPFEMPPCGTSACRCIPAGLFIGYCRNPSG.

More preferably, the polypeptide of the invention is one having the above mentioned SEQ ID No.1 - SEQ ID No.12 or a mixture of two or more polypeptides having the above mentioned SEQ ID No.1 - SEQ ID No.12 in any proportion.

The polypeptide of the invention comprises six cysteines located at positions 3, 7, 15, 20, 22 and 32, respectively. The six cysteines form three pairs of disulfide bonds in the polypeptide chain, namely Cys3-Cys20, Cys7-Cys22 and Cys15-Cys32, and thus form "cystine knot motif". In the motif, the Cys3-Cys20 and Cys7-Cys22 disulfide bonds form the backbone and the Cys15-Cys32 disulfide bond threads the backbone. The motif belongs to the inhibitor cystine knot (ICK) structure. The polypeptide of the invention further comprises 14 to 16 hydrophobic amino acids. Among them, the amino acids at positions 1, 6, 9, 10, 12, 13, 14, 19, 23, 24, 27, 28, 29 and 35 are all hydrophobic amino acids, and the positions of these hydrophobic amino acids in the amino acid sequences are highly consistent. Owing to the above structural characterization, the polypeptides of the invention are resistant to enzymatic hydrolysis by pepsin and trypsin and have excellent hypoglycemic and hypolipidemic activities, regardless of being administered orally or injected subcutaneously, intramuscularly or intravenously.

In a preferred embodiment, the polypeptide and the mixture thereof of the invention are derived from an extract of seeds of a leguminous plant or the by-product of the seeds after processing - bean meal. The leguminous plant includes, but is not limited to, soybean, faba bean, pea, red bean, mung bean, cowpea, green bean, lentil, etc., and is one of them or a mixture of two or more of them in any proportion; preferably, the leguminous plant is one selected from pea, soybean, and mung bean or a mixture of two or more selected from them in any proportion. Preferably, the extract includes the above-described polypeptides of SEQ ID No.1 - SEQ ID No.12. Preferably, the total content of the 12 polypeptides is at least 5%, preferably at least 8%, more preferably at least 10%, more preferably at least 20%, more preferably at least 30%, more preferably at least 50%, more preferably at least 80%, further preferably at least 90%, further preferably at least 95%, further preferably at least 97%, and most preferably at least 98%. The inventor has found by research that the extract would have good hypoglycemic and hypolipidemic activities even of low purity, for example but not limited to a content of 10% for the 12 polypeptides.

According to the invention, the extract is an organic solvent extract. The organic solvent is one selected from the group consisting of n-butanol, isopropanol, ethanol, methanol, formic acid and acetic acid, or a mixture of two or more organic solvents selected from the group in any proportion; and prefereably the organic solvent is n-butanol. Preferably, the extraction method includes the following steps:
(1) pulverizing the seeds of a leguminous plant or the by-product of the seeds after processing - bean meal, adding an organic solvent, extracting at low or room temperature, centrifuging and filtering, and collecting the supernatant to give crude polypeptides;
(2) purifying the crude polypeptides by reversed-phase HPLC to yield the polypeptide extract.

Preferably, before extraction with the organic solvent in step (1), the pulverized seeds of the leguminous plant or the by-product of the seeds after processing - bean meal are decolorized and degreased. More preferably, one organic solvent selected from the group consisting of chloroform, cyclohexane and petroleum ether or a mixture of two or more organic solvents selected from the group in any proportion is used for the decolorizing and degreasing; and most preferably, chloroform is used for the decolorizing and degreasing.

Preferably, the extraction temperature is 0-30 °C, more preferably 10 °C.

Preferably, the extraction time is 1-40 hours, more preferably 20-30 hours.

In the invention, the amino acid is methionine. As those skilled in the art will understand, methionine can be in D-form, L-form or DL-form, preferably L-form.

Preferably, in the invention, the polypeptide and the amino acid methionine are at a weight ration in the range of 100:1-1:100, more preferably 50:1-1:50, further preferably 50:1-1:2, most preferably 20:1-10:1.

In the invention, the composition comprising the polypeptide of the invention and the composition comprising the polypeptide of the invention and the amino acid methionine can be a liquid composition or solid composition. Preferably, in the invention, the composition comprising the polypeptide of the invention and the composition comprising the polypeptide of the invention and the amino acid methionine can further comprise recombinant human serum albumin and/or bovine serum albumin for improving the stability of the polypeptide of the invention, with the content of the recombinant human serum albumin and/or bovine serum albumin in the range of 0.5-50 mg/ml (in a liquid composition), more preferably 1-20 mg/ml, most preferably 5-10 mg/ml, or 0.5-50 mg/g (in a solid composition), more preferably 1-20 mg/g, most preferably 5-10 mg/g.

Preferably, in the invention, the composition comprising the polypeptide of the invention and the composition comprising the polypeptide of the invention and the amino acid methionine can further comprise sodium chloride for increasing ionic strength and preventing the aggregation of polypeptide, with the content of sodium chloride in the range of 1-20 mg/ml (in a liquid composition), more preferably 5-15 mg/ml, most preferably 8-12 mg/ml, or 1-20 mg/g (in a solid composition), more preferably 5-15 mg/g, most preferably 8-12 mg/g.

Preferably, in the invention, the composition comprising the polypeptide of the invention and the composition comprising the polypeptide of the invention and the amino acid methionine can further comprise ethylenediaminetetraacetic acid disodium salt for chelating metal ions and preventing the oxidation of the polypeptide, with the content of ethylenediaminetetraacetic acid disodium salt in the range of 0.05-0.1 mg/ml (in a liquid composition) or 0.05-0.2mg/g (in a solid composition).

Preferably, in the invention, the composition comprising the polypeptide of the invention and the composition comprising the polypeptide of the invention and the amino acid methionine can further comprise sodium acetate, acetic acid for adjusting pH and ionic strength and increasing the solubility of the polypeptide in water. The content of sodium acetate is in the range of 0.5-3 mg/ml (in a liquid composition), more preferably 1-2.5 mg/ml, most preferably 1.5-2 mg/ml.

Preferably, in the invention, the composition comprising the polypeptide of the invention and the composition comprising the polypeptide of the invention and the amino acid methionine can further comprise one, two or more of glycerol, mannitol and sorbitol as excipients for improving the stability of the polypeptide, with the content thereof in the range of 0.5-100 mg/ml (in a liquid composition), more preferably 1-50 mg/ml, most preferably 8-15 mg/ml, or 0.5-100 mg/g (in a solid composition), more preferably 1-50 mg/g, most perferably 8-15 mg/g.

Preferably, when the composition comprising the polypeptide of the invention and the composition comprising the polypeptide of the invention and the amino acid methionine are liquid compositions, the pH values are in the range of 3.5-6.5, more preferably 4-5, most preferably 4.5; preferably, when the composition comprising the polypeptide of the invention and the composition comprising the polypeptide of the invention and the amino acid methionine are solid compositions, the pH values of the 5% solutions of the compositions are in the range of 3.5-6.5, more preferably 4-5, most preferably 4.5.

In a preferred embodiment, the composition comprising the polypeptide of the invention and the amino acid methionine is a liquid composition, comprising:
(a) one polypeptide of the sequence as shown in general formula (I) or a mixture of two or more polypeptides of the sequences as shown in general formula (I) in any proportion;
(b) at least one amino acid methionine;
(c) sodium acetate;
(d) acetic acid;
(e) glycerol; and
(f) water for injection.

In a preferred embodiment, the composition comprising the polypeptide of the invention and the amino acid methionine is a solid composition, comprising:
(a) one polypeptide of the sequence as shown in general formula (I) or a mixture of two or more polypeptides of the sequences as shown in general formula (I) in any proportion;
(b) at least one amino acid methionine;
(c) D-mannitol; and
(d) ethylenediaminetetraacetic acid disodium salt. Preferably, the composition further comprises sodium chloride.

In the invention, preferably, the mammal is a human being.

The polypeptide and the composition thereof of the invention can reduce the increased blood glucose and/or blood lipid level caused by various reasons. The increased blood glucose occurs in but not limited to type 1 and/or type 2 diabetes, preferably type 2 diabetes, and the increased blood lipid level occurs in but not limited to hyperlipidemia.

As those skilled in the art will understand, the polypeptide and the composition thereof of the invention can be made into pharmaceutical composition or preparation, food, health food or formula food for special medical purposes.

As those skilled in the art will understand, the therapeutically effective amount of the polypeptide or the composition thereof the invention can be determined by a limited number of routine experiments. The therapeutically effective amount of the polypeptide or the mixture thereof the invention may be, for example, 1-20 mg/(kg.d); and the therapeutically effective amount of the composition comprising the polypeptide of the invention and the amino acid methionine may be, for example, 1-30 mg/(kg.d).

As those skilled in the art will understand, the polypeptide or the composition thereof of the invention can be used in combination with other hypoglycemic agents and/or hypolipidemic agents. For instance, other hypoglycemic agents include but are not limited to insulin and the derivatives thereof, such as long-acting insulin, oral hypoglycemic agents, incretin, etc., and any combination thereof. As those skilled in the art will understand, various oral hypoglycemic agents can be used in the invention, including but not limited to biguanides such as metformin, sulfonylurea secretion enhancers, thiazolidinediones such as glitazone, α-glucodidase inhibitors such as acarbose, etc., and any combination thereof. For example, other hypolipidemic agents include but are not limited to lovastatin, simvastatin, pravastatin, fenofibrate, gemfibrozil, bezafibrate, alginic sodium diester, etc. As those skilled in the art will understand, the polypeptide or the composition thereof of the invention and other hypoglycemic agents and/or hypolipidemic agents may be administered simultaneously as a single composition, or may be administered simultaneously in separate compositions, or may be administered sequentially in separate compositions. As those skilled in the art will understand, the sequence of the sequential administration does not affect the therapeutic effects of the polypeptide or the composition thereof of the invention and other hypoglycemic agents and/or hypolipidemic agents. The polypeptide or the composition thereof of the invention can be prepared into various dosage forms suitable for oral administration, intravenous injection, intramuscular injection or subcutaneous injection, including but not limited to tablets suitable for oral administration (including but not limited to various coated tablets, sustained-release or controlled-release tablets), lozenges, capsules (including soft capsules and hard capsules), granules, dispersible powders, aqueous or oily suspensions, emulsions, elixirs or syrups, etc.; injections suitable for intravenous injection, intramuscular injection or subcutaneous injection, powder for injection, etc.

When a pharmaceutical preparation is made from the polypeptide or the composition thereof of the invention, it can further comprise pharmaceutically acceptable carriers, including but not limited to various organic or inorganic drug carriers, such as excipients, lubricants, binders, disintegrants, water-soluble polymers, inorganic salts, solvents, dissolution auxiliary agents, suspensions, isotonic agents, buffers, preservatives, antioxidants, coloring agents, sweeteners, acidity regulators, foaming agents and flavoring agents, etc.

As those skilled in the art will understand, the polypeptide of the invention can be purchased commercially or obtained by various methods known in the art, including but not limited to extracting from a leguminous plant, chemical synthesis (e.g. solid phase synthesis), method for obtaining recombinant proteins by recombinant DNA technology, etc., preferably the method of extracting from a leguminous plant.

As those skilled in the art will understand, the composition, pharmaceutical preparation, food, health food or formula food for special medical purposes of the invention can be prepared by various methods known in the art. In a preferred embodiment, the polypeptide or the composition thereof, pharmaceutical preparation, food, health food or formula food for special medical purposes of the invention are stored under the protection of inert gas, preferably under nitrogen protection.

In the invention, according to the internationally accepted conventions for amino acid abbreviations, in the amino acid sequence, A represents alanine, C represents cysteine, D represents aspartic acid, E represents glutamic acid, F represents phenylalanine, G represents glycine, H represents histidine, I represents isoleucine, K represents lysine, L represents leucine, M represents methionine, N represents asparagine, P represents proline, Q represents glutamine, R represents arginine, S represents serine, T represents threonine, V represents valine, W represents tryptophan, and Y represents tyrosine. In the invention, Mₒₓ represents methionine sulfoxide.

The invention has the following advantages and beneficial effects compared with the prior art:
The polypeptide of the invention has excellent hypoglycemic and hypolipidemic activities, and when combined with an amino acid, it not only improves the stability, but also increases the hypoglycemic and hypolipidemic activities. The polypeptide of the invention and the composition thereof with an amino acid are resistant to enzymatic hydrolysis by pepsin and trypsinase, so that they can be administered orally, intravenously, intramuscularly or subcutaneously, making them rare agents that can lower blood glucose and blood lipid level by four administration modes. They have flexible adiminstration modes, also have the activity of protecting and repairing the function of pancreatic β cells, do not cause hypoglycemic reaction and insulin resistance in patients, have no toxic side-effects, are convenient and safe to be administered, have long-term storage stability, can effectively prevent and treat type 2 diabetes and hyperlipidemia, reduce glycosylated hemoglobin (HbAlc) level in diabetic patients more effectively compared with the existing hypoglycemic agents, have additional cardiovascular benefits, and reduce complications of diabetes. In sum, they have obvious advantages, and can achieve effective control of blood glucose and glycosylated haemoglobin especially in those patients who cannot effectively control blood glucose level with metformin, sulfonylurea secretion enhancers, thiazolidinediones, α-glucodidase inhibitors, insulin or its derivatives, insulin or its derivatives or combinations, the polypeptide and the composition thereof.

### BRIEF DESCRIPTION OF THE FIGURES

Fig. 1 shows the HPLC chromatogram of the polypeptide of the invention extracted from a leguminous plant;
Fig. 2 shows the amino acid sequence of the polypeptide with an amino aicd sequence of SEQ ID No.7;
Fig. 3 shows the amino acid sequence of the polypeptide with an amino aicd sequence of SEQ ID No.8;
Fig. 4 shows the amino acid sequence of the polypeptide with an amino aicd sequence of SEQ ID No.3;
Fig. 5. shows the amino acid sequence of the polypeptide with an amino aicd sequence of SEQ ID No.10;
Fig. 6 shows the HPLC chromatograms of enzymatic hydrolysis of the polypeptide of the invention.
Fig. 7 shows the HPLC chromatograms of enzymatic hydrolysis of albumin.
Fig. 8 shows the HPLC chromatograms of stability studies of the polypeptide of the invention.
Fig. 9 shows the images of H&E staining and immunohistochemistry of pancreas.

### DETAILED DESCRIPTION OF THE INVENTION

Hereinafter, the invention is further described in detail with reference to the specific examples. It should be understood that the examples are not intended to limit the scope of the invention. In addition, it should be understood that after reading the contents of the invention, a person skilled in the art can make various changes and modifications to the invention, and these equivalents are also deemed to fall within the scope of the invention.

### Example 1 Polypeptide of the invention extracted from a leguminous plant

The polypeptides and the mixture thereof of the invention can be extracted from seeds of a leguminous plant or the by-product of the seeds after processing -- bean meal. The specific extraction process was performed as follows:
10 kg of commercial pea meal was pulverized by a pulverizer, sieved to give particles with an average diameter of 100 mesh. The particles were then mixed with 50 L of chloroform, stirred for 1 hour, centrifuged and filtered. Subsequently, the filter cake was washed with chloroform, and dried to give the decolorized and degreased bean meal.

The above decolorized and degreased bean meal was mixed with 100 L of n-butanol, homogenized in a homogenizer for 30 minutes, connected to a freezer to control the working temperature at 10 °C. Then the mixture was transferred to a jacket extraction tank, stirred at 10 °C for 24 hours, centrifuged and filtered to give the supernatant. The filter cake was washed with n-butanol, and the supernatant was collected and combined.

The supernatant was then concentrated 10-fold at 50 °C by vacuum distillation, and the concentrated solution was freeze dried to yield 95.6 g of crude polypeptides.

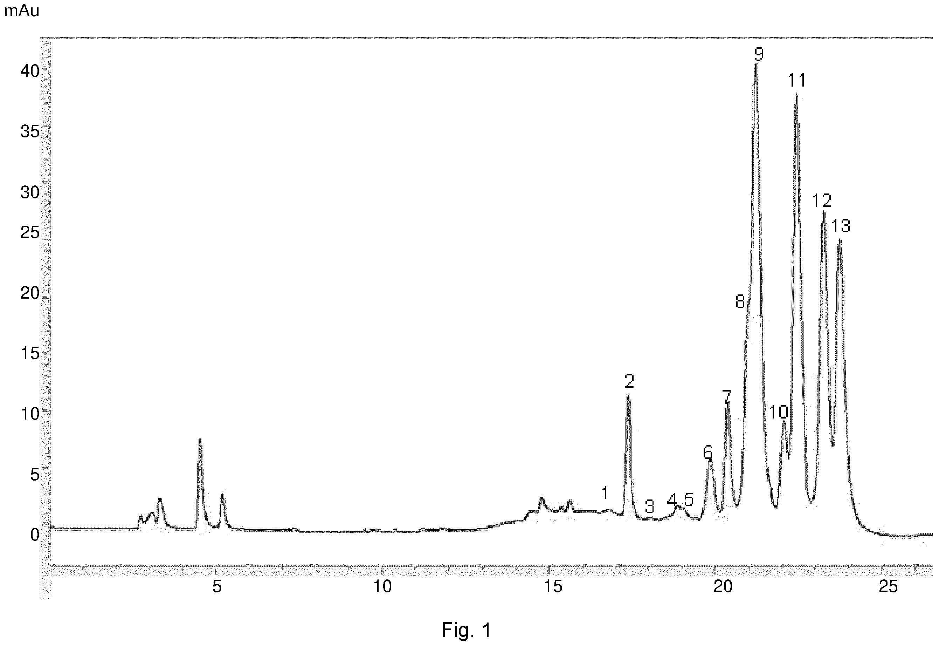

Purification and identification of the polypetides:
The above crude polypeptides were separated and purified by reversed-phase liquid chromatography. A appropriate amount of the crude polypeptides was dissolved in chromatographic solution A (water + 0.1% trifluoroacetic acid), filtered through a 0.22 □m membrane filter, and the filtrate was loaded to the liquid chromatograph. Agilent 1260 high-performance liquid chromatography was used under the following conditions: C18 reversed-phase column (Wateres Corporation, 4.6 x 250 mm, 300SB, 5 □m), mobile phase: water (containing 0.1% trifluoroacetic acid) + acetonitrile (containing 0.1% trifluoroacetic acid), gradient elution with 15% to 50% acetonitrile, over 25 min, at a flow rate of 1 ml/min, detection at 214 nm. 13 peaks were arised from 16 to 25 min (see Fig. 1). The peaks with retention times of 16.9 min, 17.5 min, 18.1 min, 18.9 min, 19.1 min, 19.9 min, 20.4 min, 21.0 min, 21.2 min, 22.1 min, 22.4 min, 23.3 min and 23.7 min were collected, respectively. After the confirmation by MALDI-TOF and Edman degradation, a total of 13 polypeptides were identified with the following the amino acid sequences:
SEQ ID No.1: ASCNGVCSPFEMPPCGTSACRCIPVGLVVGYCRNPSG,
SEQ ID No.2: ASCNGVCSPFEMₒₓPPCGTSACRCIPVGLVVGYCRNPSG,
SEQ ID No.3: ASCNGVCSPFEMPPCGTSACRCIPVGLVIGYCRNPSG,
SEQ ID No.4: ASCNGVCSPFEMₒₓPPCGTSACRCIPVGLVIGYCRNPSG,
SEQ ID No.5: ASCNGVCSPFEMPPCGSSACRCIPVGLLIGYCRNPSG,
SEQ ID No.6: ASCNGVCSPFEMₒₓPPCGSSACRCIPVGLLIGYCRNPSG,
SEQ ID No.7: ASCNGVCSPFEMPPCGTSACRCIPVGLFIGYCRNPSG,
SEQ ID No.8: ASCNGVCSPFEMₒₓPPCGTSACRCIPVGLFIGYCRNPSG,
SEQ ID No.9: ASCNGVCSPFEMPPCGTSACRCIPYGLFIGYCRNPSG,
SEQ ID No.10: ASCNGVCSPFDIPPCGSPLCRCIPVGLVIGKCRNPYG,
SEQ ID No.11: ISCNGVCSPFDIPPCGSPLCRCIPAGLVIGNCRNPYG,
SEQ ID No.12: VSCNGVCSPFDIPPCGSPLCRCIPAGLVIGKCRNPYG, and
SEQ ID No.72: ASCNGVCSPFEMPPCGSSACRCIPVGLVVGYCRHPSG.

### Example 2 Synthesis of polypeptides of the invention by solid-phase polypeptide synthesis method

The polypeptides of the invention can also be synthesized by the solid-phase polypeptide synthesis method. The specific process was performed as follows:
An ABI 433A polypeptide synthesizer from Applied Biosystems was used, a Fmoc/tBu method and a NMP solvent system was adopted, and HBTU/HOBt was utilized as condensing agents. The following protecting groups was used for protecting the amino acid side-chain respectively: *tert*-butyl (*t*Bu) group for Ser, Thr and Tyr, *tert*-butyl ester group for Asp and Glu, triphenylmethyl (Trt) group for Asn, Gln and Cys, 2,2,4,6,7-pentamethyl-dihydrobenzofuran-5-sulfonyl (Pbf) group for Arg, and 2-chlorobenzyl-oxycarbonyl for Lys. The synthesis scale was 0.5 mmol. The amounts of amino acids with protective groups and condensation agents were 10 times excess. The program was automatically completed after setting. After the reaction was completed, the resin was washed three times with DCM and dried under vacuum. Then, a mixture of TFA/TIS/H₂O/Phenol (the addition of phenol prevented the formation of disulfide bond via the oxidation of cysteine ) was added to the polypeptide solid-phase reactor to react sufficiently with the resin for 2 hours. Subsequently, most of the solvent was removed with nitrogen, frozen methyl *tert*-butyl ether was added to allow precipitation, the supernatant was removed by centrifugation, and the precipitate was washed with frozen methyl *tert*-butyl ether and freeze dried to yield crude polypeptides in reduced form.

In 100 ml of 50% isopropanol solution, 10 mg of the crude reduced polypeptides, EDTA, Tris-HCl, GSH and GSSG at concentrations of 2 mM, 200 mM, 1 mM and 100 µM, respectively, were added, at pH 8.6, and reacted at 4 °C for 48 hours. The reaction was terminated by adding TFA to pH 4. Isopropanol was removed by vacuum concentration, and the concentrated solution was freeze dried to yield crude polypeptides. The crude products were dissolved in 60% methanol, separated and purified by semi-preparative HPLC, with a gradient elution of 30-45% acetonitrile, and then freeze dried to yield the product, about 60% yield, ≥98% HPLC purity.

The polypeptides with the following amino acid sequences were synthesized respectively by the solid-phase polypeptide synthesis method:
SEQ ID No.1: ASCNGVCSPFEMPPCGTSACRCIPVGLVVGYCRNPSG,
SEQ ID No.13: ASCNGVCSPFDIPPCGTPLCRCIPVGLVIGNCRNPYG,
SEQ ID No.17: ASCNGVCSPFDMPPCGTSACRCIPAGLFIGKCRNPYG,
SEQ ID No.21: ASCNGVCSPFEIPPCGSPLCRCIPVGLVIGNCRNPYG,
SEQ ID No.26: ASCNGVCSPFEMPPCGSSACRCIPVGLVVGYCRNPSG,
SEQ ID No.36: ISCNGVCSPFDIPPCGTPLCRCIPAGLFVGKCRNPYG,
SEQ ID No.38: ISCNGVCSPFDMPPCGSPACRCIPAGLLIGYCRNPSG,
SEQ ID No.44: ISCNGVCSPFEIPPCGSSLCRCIPAGLLVGKCRNPSG,
SEQ ID No.50: ISCNGVCSPFEMPPCGTSLCRCIPAGLVVGYCRNPSG,
SEQ ID No.52: VSCNGVCSPFDIPPCGTPACRCIPYGLFVGNCRNPYG,
SEQ ID No.61: VSCNGVCSPFDMPPCGTSACRCIPAGLFIGYCRNPSG,
SEQ ID No.63: VSCNGVCSPFEIPPCGSPLCRCIPAGLVIGNCRNPYG, and
SEQ ID No.68: VSCNGVCSPFEMPPCGSSACRCIPYGLVVGNCRNPSG.

### Example 3 Experiment of hypoglycemic effect of the polypeptide of the invention on normal mice after tail vein injection

### 1. Materials and Instruments

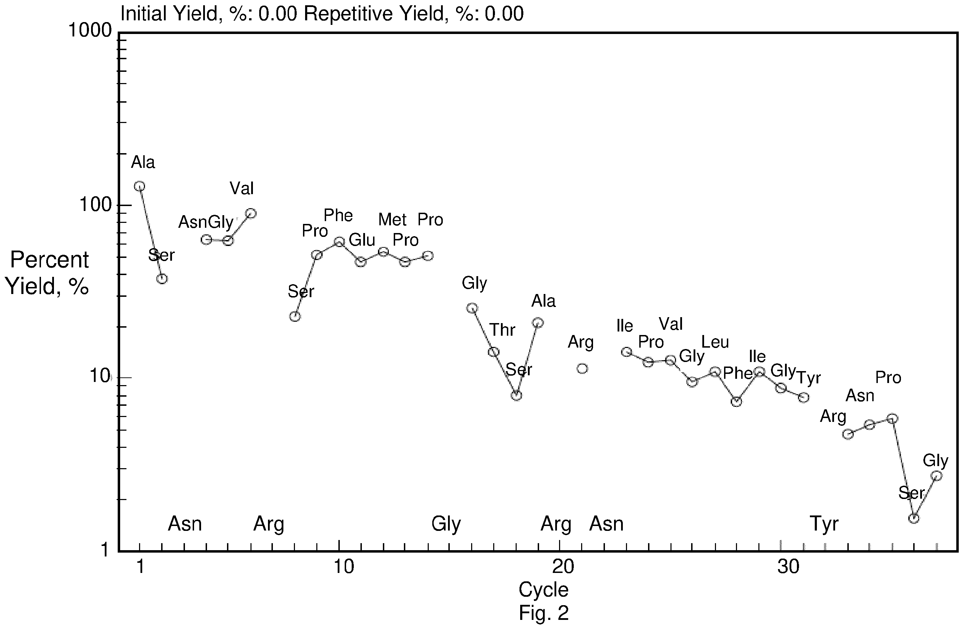

Polypeptide: the polypeptide with an amino aicd sequence of SEQ ID No.7 in Example 1, with an average molecular weight of 3789, an amino acid sequence of ASCNGVCSPFEMPPCGTSACRCIPVGLFIGYCRNPSG (see Fig. 2), HPLC purity of ≥98%, was prepared into 1 mg/mL solution with normal saline for use.

Kunming white mice: Hubei Epidemic Prevention Station, 40 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into four groups, 10 mice in each group. Each of the mice was stained with picric acid as a marker and detected the blood glucose level by collecting tail vein blood. The mice in the blank control group were intravenously injected once with normal saline at a dose of 0.05 ml/10g into the tail vein, mice in Group A (high-dose polypeptide hypoglycemic group) were intravenously injected once with 1 mg/mL polypeptide at a dose of 0.10ml/10g into the tail vein, mice in Group B (medium-dose polypeptide hypoglycemic group) were intravenously injected once with 1 mg/mL polypeptide at a dose of 0.05ml/10g into the tail vein, and mice in Group C (low-dose polypeptide hypoglycemic group) were intravenously injected once with 1 mg/mL polypeptide at a dose of 0.025ml/10g into the tail vein. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 1 below:

**Table 1. Hypoglycemic effect of the polypeptide on normal mice after intravenous injection into tail vein**

| Mice group | Blood glucose level prior to administration | Blood glucose level at 15 min after administration | Blood glucose level at 30 min after administration | Blood glucose level at 45 min after administration | Blood glucose level at 45 min after administration |
|---|---|---|---|---|---|
| Blank control group | 7.1±0.4 | 7.9±0.5 | 7.4±0.3 | 6.8±0.2 | 6.3±0.3 |
| Group A | 7.1±0.5 | 3.3±0.3^{∗∗} | 2.1±0.3^{∗∗} | 1.7±0.2^{∗∗} | 1.5±0.2^{∗∗} |
| Group B | 7.9±0.6 | 4.3±0.4^{∗∗} | 3.3±0.3^{∗∗} | 2.9±0.4^{∗∗} | 2.7±0.3^{∗∗} |
| Group C | 6.9±0.5 | 5.7±0.3 | 4.3±0.3^{∗∗} | 3.9±0.4^{∗∗} | 4.9±0.5^{∗} |

| | | | | | |
|---|---|---|---|---|---|
| Note: as compared with blank control group, ^{∗}p<0.05, ^{∗∗}p<0.01. | | | | | |

### 4. Conclusion

From the above experimental results, it could be seen that 15 minutes after normal mice were tail vein injected with the polypeptide with an amino aicd sequence of SEQ ID No.7 of the invention, the blood glucose level began to drop, and the hypoglycemic effect was in the following order: high-dose Group A > medium-dose Group B > low-dose Group C, showing an obvious dose-effect relationship. The results indicated that the polypeptide with an amino aicd sequence of SEQ ID No.7 of the invention could lower blood glucose level of normal mice by intravenous injection, and thus demonstrated that the polypeptide had explicit hypoglycemic activity.

### Example 4 Experiment of hypoglycemic effect of the polypeptide of the invention on normal mice after subcutaneous injection

### 1. Materials and Instruments

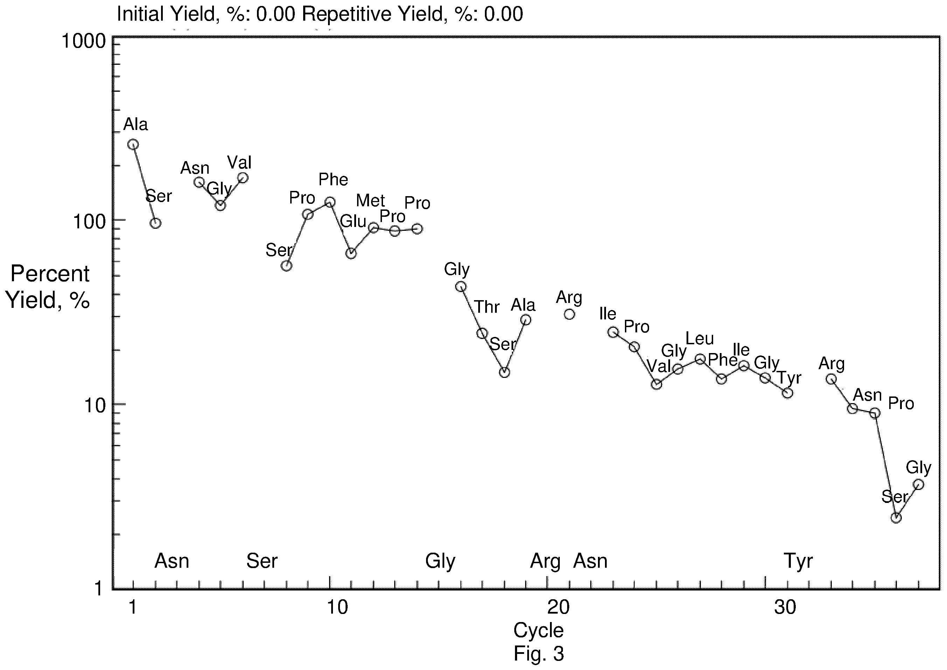

Polypeptide: the polypeptide with an amino aicd sequence of SEQ ID No.8 in Example 1, with an average molecular weight of 3805, an amino acid sequence of ASCNGVCSPFEMₒₓPPCGTSACRCIPVGLFIGYCRNPSG (see Fig. 3), HPLC purity of ≥98% by, was prepared into 1 mg/mL solution with normal saline for use.

Kunming white mice: Hubei Epidemic Prevention Station, 50 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into four groups, 10 mice in each group. Each of the mice was stained with picric acid as a marker and detected blood glucose level by collecting tail vein blood. The mice in the blank control group were subcutaneously injected once with normal saline at a dose of 0.05ml/10g on the back, mice in Group A (high-dose polypeptide hypoglycemic group) were subcutaneously injected once with 1 mg/mL polypeptide at a dose of 0.10ml/10g on the back, mice in Group B (medium-dose polypeptide hypoglycemic group) were subcutaneously injected once with 1 mg/mL polypeptide at a dose of 0.05ml/10g on the back, and mice in Group C (low-dose polypeptide hypoglycemic group) were subcutaneously injected once with 1 mg/mL polypeptide at a dose of 0.025ml/10g on the back. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 2 below:

**Table 2. Hypoglycemic effect of the polypeptide on normal mice after subcutaneous injection on the back**

| Mice group | Blood glucose level prior to administratio n | Blood glucose level at 15 min after administration | Blood glucose level at 30 min after administration | Blood glucose level at 45 min after administration | Blood glucose level at 60 min after administration |
|---|---|---|---|---|---|
| Blank control group | 6.7±0.6 | 8.1±0.5 | 7.5±0.4 | 6.7±0.3 | 6.1±0.4 |
| Group A | 7.4±0.5 | 5.1±0.4^{∗∗} | 3.0±0.4^{∗∗} | 2.2±0.2^{∗∗} | 1.7±0.1^{∗∗} |
| Group B | 7.1±0.6 | 5.2±0.4^{∗∗} | 4.3±0.5^{∗∗} | 3.2±0.3^{∗∗} | 2.6±0.2^{∗∗} |
| Group C | 7.3±0.4 | 6.2±0.3^{∗∗} | 4.9±0.3^{∗∗} | 3.8±0.2^{∗∗} | 4.6±0.2^{∗∗} |

| | | | | | |
|---|---|---|---|---|---|
| Note: as compared with blank control group, ^{∗}p<0.05, ^{∗∗}p<0.01. | | | | | |

### 4. Conclusion

From the above experimental results, it could be seen that 15 minutes after normal mice were subcutaneously injected with the polypeptide with an amino aicd sequence of SEQ ID No.7 of the invention on the back, the blood glucose level began to drop, and the hypoglycemic effect was in the following order: high-dose Group A > medium-dose Group B > low-dose Group C, showing an obvious dose-effect relationship. The results indicated that the polypeptide with an amino aicd sequence of SEQ ID No.8 of the invention could also lower blood glucose level of normal mice, and thus demonstrated that the polypeptide had explicit hypoglycemic activity. Moreover, as compared with the polypeptide with an amino aicd sequence of SEQ ID No.7, the oxidation methionine of into methionine sulfoxide did not affect the hypoglycemic activity

### Example 5 Experiment of hypoglycemic effect of the polypeptide of the invention on normal mice after subcutaneous injection

### 1. Materials and Instruments

Polypeptide: the polypeptide with an amino aicd sequence of SEQ ID No.72 in Example 1, with an average molecular weight of 3736, an amino acid sequence of ASCNGVCSPFEMPPCGSSACRCIPVGLVVGYCRHPSG, HPLC purity of ≥98%, was prepared into 1 mg/mL solution with normal saline for use.

The polypeptide with an amino aicd sequence of SEQ ID No.26 in Example 2, with an average molecular weight of 3716, an amino acid sequence of ASCNGVCSPFEMPPCGSSACRCIPVGLVVGYCRNPSG, HPLC purity of ≥98%, was prepared into 1 mg/mL solution with normal saline for use.

Kunming white mice: Hubei Epidemic Prevention Station, 30 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into three groups, 10 mice in each group. Each of the mice was stained with picric acid as a marker and detected the blood glucose level by collecting tail vein blood. The mice in the blank control group were subcutaneously injected once with normal saline at a dose of 0.05ml/10g on the back, mice in Group A (polypeptide of SEQ ID No.72) were subcutaneously injected once with 1 mg/mL polypeptide at a dose of 0.05ml/10g on the back, and mice in Group B (polypeptide of SEQ ID No.26) were subcutaneously injected once with 1 mg/mL polypeptide at a dose of 0.05ml/10g on the back. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 3 below:

**Table 3. Hypoglycemic effect of the polypeptide on normal mice after subcutaneous injection on the back**

| Mice group | Blood glucose level prior to administratio n | Blood glucose level at 15 min after administration | Blood glucose level at 30 min after administration | Blood glucose level at 45 min after administration | Blood glucose level at 45 min after administration |
|---|---|---|---|---|---|
| Blank control group | 6.9±0.4 | 7.6±0.5 | 7.2±0.6 | 6.8±0.7 | 6.4±0.9 |
| Group A | 7.3±0.6 | 8.91±0.7 | 10.4±0.8^{∗∗} | 11.1±0.9^{∗∗} | 10.2±0.7^{∗∗} |
| Group B | 7.1±0.5 | 5.3±0.4^{∗∗} | 4.4±0.3^{∗∗} | 3.6±0.3^{∗∗} | 2.2±0.1^{∗∗} |

| | | | | | |
|---|---|---|---|---|---|
| Note: as compared with blank control group, ^{∗}p<0.05, ^{∗∗}p<0.01. | | | | | |

### 4. Conclusion

The polypeptide with an amino aicd sequence of ASCNGVCSPFEMPPCGSSACRCIPVGLVGYCRNPSG could lower blood glucose level of mice by subcutaneous injection and had hypoglycemic activity. However, the polypeptide with an amino aicd sequence of ASCNGVCSPFEMPPCGSSACRCIPVGLVGYCRHPSG raised blood glucose level of mice by subcutaneous injection, and had an opposite effect to the former. The two merely differed in the structure of the amino acid at position 34, with the former having a neutral amino acid-asparagine (Asn) and the latter having a basic amino acid-histidine (His). The possible reason for the different bioactivity between the two might be that the latter had strong hydrophilicity, which could be confirmed by the shortest retention time in the C18 reversed-phase chromatography. More particularly, the polypeptide of amino acid sequence of SEQ ID No.72 was assigned to peak No.1 in Fig. 1 with a retention time of 16.9 min. The histidine contained in the polypeptide of SEQ ID No.72 was protonated in physiological environment, became more hydrophilic, and consequently changed the spatial conformation of the polypeptide and the way it bound to protein, which resulted in no hypoglycemic activity of the polypeptide.

It could also be seen from the current example that the amino acid at position 34 of the polypeptide was the key site to the hypoglycemic activity, and if it was H (histidine), the corresponding polypeptide had no hypoglycemic activity.

### Example 6 Comparative experiment of hypoglycemic effect of polypeptides from natural extracts and solid-phase synthetic polypeptides of the invention on normal mice after subcutaneous injection

### 1. Materials and Instruments

Polypeptide A: the polypeptide with an amino aicd sequence of SEQ ID No.1 in Example 1, with an average molecular weight of 3727, an amino acid sequence of ASCNGVCSPFEMPPCGTSACRCIPVGLVVGYCRNPSG, HPLC purity of ≥98%, was prepared into 1 mg/mL solution with normal saline for use.

Polypeptide B: the polypeptide with an amino aicd sequence of SEQ ID No.1 in Example 2, with an average molecular weight of 3727, an amino acid sequence of ASCNGVCSPFEMPPCGTSACRCIPVGLVVGYCRNPSG, HPLC purity of ≥98%, was prepared into 1 mg/mL solution with normal saline for use.

Kunming white mice: Hubei Epidemic Prevention Station, 30 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into three groups, 10 mice in each group. Each of the mice was stained with picric acid as a marker and detected the blood glucose level by collecting tail vein blood. The mice in the blank control group were subcutaneously injected once with normal saline at a dose of 0.05ml/10g on the back, mice in Group A (polypeptide A) were subcutaneously injected once with 1 mg/mL polypeptide at a dose of 0.05ml/10g on the back, mice in Group B (polypeptide B) were subcutaneously injected once with 1 mg/mL polypeptide at a dose of 0.05ml/10g on the back. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 4 below:

**Table 4. Hypoglycemic effect of the polypeptide on normal mice after subcutaneous injection on the back**

| Mice group | Blood glucose level prior to administrat ion | Blood glucose level at 15 min after administration | Blood glucose level at 30 min after administration | Blood glucose level at 45 min after administration | Blood glucose level at 60 min after administration |
|---|---|---|---|---|---|
| Blank control group | 7.0±0.6 | 7.6±0.5 | 8.2±0.6 | 7.8±0.5 | 7.4±0.6 |
| Group A | 6.8±0.5 | 5.2±0.4^{∗} | 4.4±0.5^{∗∗} | 3.6±0.3^{∗∗} | 2.2±0.2^{∗∗} |
| Group B | 7.2±0.6 | 5.5±0.3^{∗} | 4.6±0.2^{∗∗} | 3.3±0.2^{∗∗} | 2.4±0.1^{∗∗} |

| | | | | | |
|---|---|---|---|---|---|
| Note: as compared with blank control group, ^{∗}p<0.05, ^{∗∗}p<0.01. | | | | | |

### 4. Conclusion

From the above experimental results, it could be seen that there was no significant difference between the two polypeptides in hypoglycemic activity. It was thus clear that the polypeptides of the invention derived from different sources but with the same structures had the same hypoglycemic activity.

### Example 7 Comparative experiment of hypoglycemic effect of polypeptide of the invention on normal mice after tail vein injection, subcutaneous injection and intramuscular injection

### 1. Materials and Instruments

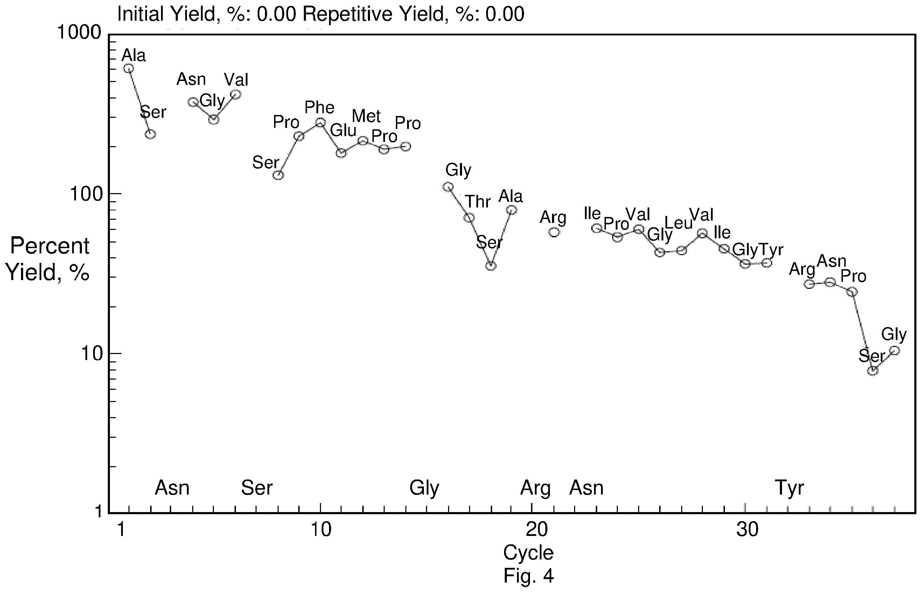

Polypeptide: the polypeptide with an amino aicd sequence of SEQ ID No.3 in Example 1, with an average molecular weight of 3741, an amino acid sequence of ASCNGVCSPFEMPPCGTSACRCIPVGLVIGYCRNPSG (see Fig. 4), HPLC purity of ≥98%, was prepared into 1 mg/mL solution with normal saline for use.

Kunming white mice: Hubei Epidemic Prevention Station, 40 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into four groups, 10 mice in each group. Each of the mice was stained with picric acid as a marker and detected the blood glucose level by collecting tail vein blood. The mice in the blank control group were subcutaneously injected once with normal saline at a dose of 0.05ml/10g on the back, mice in Group A were injected once with 1 mg/mL polypeptide at a dose of 0.05ml/10g into the tail vein of the mice, mice in Group B were subcutaneously injected once with 1 mg/mL polypeptide at a dose of 0.05ml/10g on the back, and mice in Group C were intramuscularly injected once with 1 mg/mL polypeptide at a dose of 0.05ml/10g in the lateral thigh. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 5 below:

**Table 5. Hypoglycemic effect of the polypeptide on normal mice after tail vein injection, subcutaneous injection and intramuscular injection**

| Mice group | Blood glucose level prior to administration | Blood glucose level at 15 min after administration | Blood glucose level at 30 min after administration | Blood glucose level at 45 min after administration | Blood glucose level at 60 min after administration |
|---|---|---|---|---|---|
| Blank control group | 6.8±0.4 | 7.4±0.3 | 8.1±0.4 | 7.6±0.5 | 7.2±0.2 |
| Group A | 7.3±0.5 | 5.6±0.3^{∗∗} | 4.5±0.2^{∗∗} | 3.2±0.1^{∗∗} | 2.4±0.2^{∗∗} |
| Group B | 6.2±0.3 | 5.2±0.2^{∗∗} | 3.5±0.3^{∗∗} | 2.8±0.2^{∗∗} | 2.1±0.1^{∗∗} |
| Group C | 8.1±0.7 | 6.3±0.5 | 4.8±0.4^{∗∗} | 3.5±0.3^{∗∗} | 2.6±0.2^{∗∗} |

| | | | | | |
|---|---|---|---|---|---|
| Note: as compared with blank control group, ^{∗}p<0.05, ^{∗∗}p<0.01. | | | | | |

### 4. Conclusion

From the above experimental results, it could be seen that the polypeptide administered in three ways (intravenous, subcutaneous and intramuscular injections) all had significant hypoglycemic effects, with no significant difference in hypoglycemic effect. As the subcutaneous injection was relatively convenient to operate, it could be adopted in the screening method of the polypeptide and the composition thereof of the invention for the hypoglycemic activity.

### Example 8 Experiment of hypoglycemic effect of the polypeptides of the invention on normal mice after subcutaneous injection

### 1. Materials and Instruments

Polypeptides: the polypeptides with amino aicd sequences of SEQ ID No.13, 17, 21, 26, 36, 38, 44, 50, 52, 61, 63, and 68 in Example 2, totally 12, with HPLC purity of ≥98%, were prepared into 1 mg/mL solutions with normal saline for use.

Kunming white mice: Hubei Epidemic Prevention Station, 130 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into 13 groups, 10 mice in each group. Each of the mice was stained with picric acid as a marker and detected the blood glucose level by collecting tail vein blood. The mice in the blank control group were subcutaneously injected once with normal saline at a dose of 0.05ml/10g on the back, and mice in the other groups were subcutaneously injected once with 1 mg/mL polypeptide at a dose of 0.05ml/10g on the back, respectively. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 6 below:

**Table 6. Hypoglycemic effect of the polypeptides on normal mice after subcutaneous injection on the back**

| Mice group | Blood glucose level prior to administration | Blood glucose level at 15 min after administration | Blood glucose level at 30 min after administration | Blood glucose level at 45 min after administration | Blood glucose level at 60 min after administration |
|---|---|---|---|---|---|
| Blank control group | 6.5±0.4 | 7.4±0.3 | 8.1±0.5 | 7.8±0.4 | 7.2±0.3 |
| SEQ ID No.13 | 7.4±0.6 | 6.1±0.3 | 4.4±0.4^{∗∗} | 3.5±0.3^{∗∗} | 2.6±0.3^{∗∗} |
| SEQ ID No.17 | 8.2±0.7 | 6.6±0.5 | 4.9±0.4^{∗∗} | 3.6±0.3^{∗∗} | 3.0±0.2^{∗∗} |
| SEQ ID No.21 | 7.8±0.4 | 6.7±0.4 | 5.2±0.3^{∗∗} | 4.3±0.2^{∗∗} | 3.6±0.1^{∗∗} |
| SEQ ID No.26 | 6.8±0.3 | 5.3±0.4^{∗∗} | 4.6±0.3^{∗∗} | 3.2±0.2^{∗∗} | 2.1±0.2^{∗∗} |
| SEQ ID No.36 | 7.1±0.5 | 6.2±0.4 | 4.3±0.3^{∗∗} | 3.5±0.2^{∗∗} | 2.8±0.1^{∗∗} |
| SEQ ID No.38 | 6.3±0.5 | 4.9±0.3^{∗} | 3.6±0.4^{∗∗} | 3.1±0.2^{∗∗} | 3.5±0.3^{∗∗} |
| SEQ ID No.44 | 7.2±0.3 | 6.3±0.3^{∗} | 5.5±0.2^{∗∗} | 3.8±0.3^{∗∗} | 2.6±0.2^{∗∗} |
| SEQ ID No.50 | 7.3±0.6 | 6.2±0.5^{∗} | 5.3±0.2^{∗∗} | 4.6±0.3^{∗∗} | 3.2±0.3^{∗∗} |
| SEQ ID No.52 | 7.1±0.3 | 6.4±0.4 | 5.4±0.5^{∗∗} | 4.3±0.3^{∗∗} | 2.9±0.3^{∗∗} |
| SEQ ID No.61 | 6.9±0.4 | 5.6±0.4^{∗∗} | 4.3±0.3^{∗∗} | 3.6±0.2^{∗∗} | 2.3±0.1^{∗∗} |
| SEQ ID No.63 | 6.6±0.6 | 5.9±0.5^{∗} | 4.6±0.4^{∗∗} | 3.2±0.3^{∗∗} | 3.5±0.2^{∗∗} |
| SEQ ID No.68 | 6.7±0.6 | 5.5±0.4^{∗} | 4.2±0.3^{∗∗} | 3.4±0.4^{∗∗} | 2.2±0.1^{∗∗} |

| | | | | | |
|---|---|---|---|---|---|
| Note: as compared with blank control group, ^{∗}p<0.05, ^{∗∗}p<0.01. | | | | | |

### 4. Conclusion

In the polypeptides with amino acid sequences X₁SCNGVCSPFX₂X₃PPCGX₄X₅X₆CRCIPX₇GLX₈X₉GX₁₀CRNPX₁₁G, X₁ is A, V, I; X₂X₃ is EI, EM, DI, DM; X₄X₅X₆X₆ is SPA, SPL, SSA, SSL, TPA, TPL, TSA, TSL; X₇ is A, V, Y; X₈X₉ is FI, LI, VI, FV, LV, VV; X₁₀ is K, N, Y; and X₁₁ is S,Y. All the polypeptides formed by the combination of the above candidates have explicit hypoglycemic activity. Therefore, all the polypeptides having the above general structure have hypoglycemic activity.

### Example 9 Comparative experiment of hypoglycemic effect of the polypeptide of the invention and insulin on normal mice after tail vein injection

### 1. Materials and Instruments

Insulin: Jiangsu Wanbang Biochemical Pharmaceutical Co., Ltd;
Polypeptide: the polypeptide with an amino aicd sequence of SEQ ID No.11 in Example 1, with an average molecular weight of 3788, amino acid sequence ISCNGVCSPFDIPPCGSPLCRCIPAGLVIGNCRNPYG, HPLC purity of ≥98%, was prepared into 2 mg/mL solution with normal saline for use;
Kunming white mice: Hubei Epidemic Prevention Station, 30 mice, weighing 20 ± 2 g, male.
Blood glucose meter and test strips: Beijing Yicheng Bioelectronics Technology Co., Ltd.

### 2. Experimental method

After being fasted for 4 hours (free access to water), the above mentioned white mice were randomly divided into three groups, 10 mice in each group. Each of the mice was stained with picric acid as a marker and detected the blood glucose level by collecting tail vein blood. The mice in the blank control group were intravenously injected once with normal saline at a dose of 0.05ml/10g into the tail vein, mice in Group A (high-dose polypeptide hypoglycemic group) were intravenously injected once with 2mg/mL polypeptide at a dose of 0.05ml/10g into the tail vein, mice in Group B (insulin hypoglycemic control group) were intravenously injected once with 0.5U/mL polypeptide at a dose of 0.05U/10g into the tail vein. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 7 below:

**Table 7. Hypoglycemic effect of the polypeptide and insulin on normal mice after tail vein injection**

| Mice group | Blood glucose level prior to administratio n | Blood glucose level at 15 min after administration | Blood glucose level at 30 min after administration | Blood glucose level at 45 min after administration | Blood glucose level at 60 min after administration |
|---|---|---|---|---|---|
| Blank control group | 6.9±0.5 | 7.8±0.6 | 7.7±0.5 | 7.5±0.6 | 7.5±0.4 |
| Group A | 8.2±0.5 | 4.1±0.3^{∗∗} | 2.8±0.2^{∗∗} | 2.2±0.1^{∗∗} | 2.0±0.1^{∗∗} |
| Group B | 7.7±0.6 | 5.2±0.4^{∗∗} | 3.6±0.3^{∗∗} | 2.7±0.4^{∗∗} | 2.2±0.2^{∗∗} |

| | | | | | |
|---|---|---|---|---|---|
| Note: as compared with blank control group, ^{∗}p<0.05, ^{∗∗}p<0.01. | | | | | |

### 4. Conclusion

From the above experimental results, it could be seen that the polypeptide with an amino acid sequence of SEQ ID No.11 had the same hypoglycemic effect on normal mice as insulin after tail vein injection. The polypeptide injected at a dose of 10mg/kg body weight produced the same hypoglycemic effect on mice as that of insulin injected at a dose of 2.5U/kg body weight. Therefore, the polypeptide had explicit hypoglycemic activity.

### Example 10 Experiment of hypoglycemic effect of the polypeptides of the invention on normal mice after administeration by gavage

### 1. Materials and Instruments

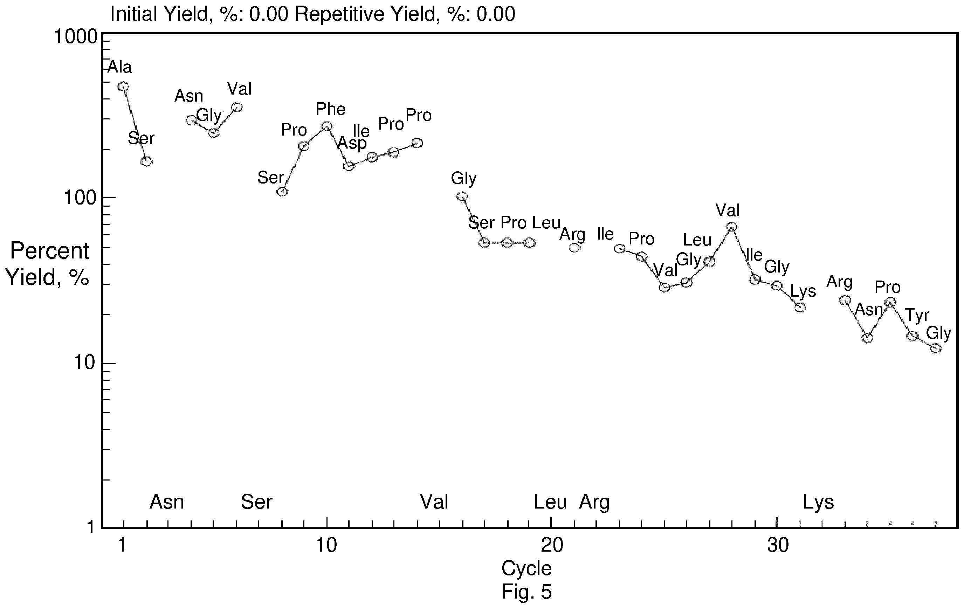

Polypeptide: the polypeptide with an amino aicd sequence of SEQ ID No.10 in Example 1, with an average molecular weight of 3788, an amino acid sequence of ASCNGVCSPFDIPPCGSPLCRCIPVGLVIGKCRNPYG (see Fig. 5), HPLC purity of ≥98%, was prepared into 1 mg/mL solutions with normal saline and 10% glucose solution, respectively, for use.

Kunming white mice: Hubei Epidemic Prevention Station, 30 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into three groups, 10 mice in each group. Each of the mice was stained with picric acid as a marker and detected the blood glucose level by collecting tail vein blood. Mice in Group A were administered by gavage with 10% glucose solution at a dose of 0.10ml/10g, mice in Group B (polypeptide group) were administered by gavage with 1mg/mL polypeptide in saline solution at a dose of 0.10ml/10g, and mice in group C (polypeptide + glucose group) were administered by gavage with 1mg/mL polypeptide in glucose solution (10%) at a dose of 0.10ml/10g. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 8 below:

**Table 8. Hypoglycemic effect of the polypeptides on normal mice after administration by gavage**

| Mice group | Blood glucose level prior to administrati on | Blood glucose level at 15 min after administration | Blood glucose level at 30 min after administration | Blood glucose level at 45 min after administration | Blood glucose level at 60 min after administration |
|---|---|---|---|---|---|
| Group A | 7.1±0.5 | 9.9±0.8 | 9.3±0.6 | 9.0±0.5 | 8.5±0.3 |
| Group B | 6.7±0.6 | 6.9±0.3^{∗} | 6.4±0.2^{∗} | 6.1±0.3^{∗∗} | 6.1±0.2^{∗} |
| Group C | 6.8±0.4 | 7.1±0.3 | 6.9±0.2^{∗} | 6.6±0.3^{∗} | 6.2±0.2^{∗} |

| | | | | | |
|---|---|---|---|---|---|
| Note: as compared with Group A, ^{∗}p<0.05, ^{∗∗}p<0.01. | | | | | |

### 4. Conclusion

From the above experimental results, it could be seen that the polypeptides of the invention after administration by gavage to normal mice would not cause hypoglycemic reaction in the normal mice, and when the normal mice were administered with glucose and the polypeptides of the invention at the same time by gavage, the blood glucoseglucose level of mice was not elevated. It indicated that the polypeptides of the invention could inhibit the increase of blood glucose level caused by glucose uptake in mice. Therefore, the polypeptides of the invention by oral administration before meal could stabilize postprandial blood glucose level in normal range, indirectly proving the hypoglycemic activity thereof.

### Example 11 Hypoglycemic activity of the polypeptides of the invention on normal mice after subcutaneous injection

### 1. Materials and Instruments

Polypeptide mixture A: the polypeptide with an amino aicd sequence of SEQ ID No.3 and the polypeptide with an amino aicd sequence of SEQ ID No.12 in Example 1 were homogeneously mixed at 1:1 mass ratio to give polypeptide mixture A.

Polypeptide mixture B: the polypeptide with an amino aicd sequence of SEQ ID No.1 and the polypeptide with an amino aicd sequence of SEQ ID No.9 in Example 1 were homogeneously mixed at 8:1 mass ratio to give polypeptide mixture B.

Polypeptide mixture C: the polypeptide with an amino aicd sequence of SEQ ID No.36 and the polypeptide with an amino aicd sequence of SEQ ID No.68 in Example 2 were homogeneously mixed at mass ratio of 1:6 to give polypeptide mixture C.

Polypeptide mixture D: the fractions between 17.5 and 25 min in Example 1 were collected and freeze dried to give polypeptide mixture D, in which the total content of the 12 polypeptides with amino aicd sequences of SEQ ID No.1 to SEQ ID No.12 was ≥98%.

Each of the above mixture was prepared into 1 mg/mL solution with normal saline for use, respectively.

Kunming white mice: Hubei Epidemic Prevention Station, 50 mice, weighing 20 ± 2 g, male.

Blood glucose meter and test strips: Beijing Yicheng Bioelectronics Technology Co., Ltd.

### 2. Experimental method

After being fasted for 4 hours (free access to water), the above mentioned white mice were randomly divided into five groups, 10 mice in each group. Each of the mice was stained with picric acid as a marker and detected the blood glucose level by collecting tail vein blood. The mice in the blank control group were subcutaneously injected once with normal saline at a dose of 0.05ml/10g on the back, mice in Group A (polypeptide mixture A) were subcutaneously injected once with 1 mg/mL of polypeptide mixture A at a dose of 0.05ml/10g, mice in Group B (polypeptide mixture B) were subcutaneously injected once with 1 mg/mL of polypeptide mixture B at a dose of 0.05ml/10g, mice in Group C (polypeptide mixture C) were subcutaneously injected once with 1 mg/mL of polypeptide mixture C at a dose of 0.05ml/10g, and mice in Group D (polypeptide mixture D) were subcutaneously injected once with 1 mg/mL of polypeptide mixture D at a dose of 0.05ml/10g. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 9 below:

**Table 9. Hypoglycemic activity of the polypeptide mixtures on normal mice after subcutaneous injection**

| Mice group | Blood glucose level prior to administratio n | Blood glucose level at 15 min after administration | Blood glucose level at 30 min after administration | Blood glucose level at 45 min after administration | Blood glucose level at 60 min after administration |
|---|---|---|---|---|---|
| Blank control group | 7.3±0.5 | 7.9±0.3 | 8.7±0.5 | 8.1±0.6 | 7.6±0.3 |
| A | 6.9±0.6 | 5.2±0.2^{∗∗} | 4.3±0.4^{∗∗} | 3.7±0.4^{∗∗} | 2.6±0.2^{∗∗} |
| B | 7.8±0.4 | 6.5±0.4^{∗} | 5.2±0.3^{∗∗} | 3.4±0.3^{∗∗} | 2.8±0.1^{∗∗} |
| C | 7.2±0.8 | 5.8±0.3^{∗∗} | 4.5±0.5^{∗∗} | 3.2±0.2^{∗∗} | 2.5±0.3^{∗∗} |
| D | 8.3±0.5 | 6.4±0.2^{∗∗} | 4.9±0.3^{∗∗} | 3.6±0.2^{∗∗} | 2.7±0.2^{∗∗} |

| | | | | | |
|---|---|---|---|---|---|
| Note: as compared with blank control group, ^{∗}p<0.05, ^{∗∗}p<0.01. | | | | | |

### 4. Conclusion

From the above experimental results, it could be seen that the polypeptides of the invention and the compositions comprising the polypeptides in any proportion also had excellent hypoglycemic activity, and the combination of the polypeptides did not affect the hypoglycemic activity. Especially the polypeptide mixture (polypeptide mixture D) derived from natural extract had significant hypoglycemic activity, meaning that the polypeptide mixture extracted from a leguminous plant (the collection of fractions between 17.5 and 25 min from HPLC) could be directly used as hypoglycemic agents without further purification, which would greatly increase yield and reduce production costs.

### Example 12 Comparative experiment of hypoglycemic effect of polypeptide mixtures of the invention and insulin on mouse models of type 2 diabetes after tail vein injection

### 1. Materials and Instruments

Insulin: Jiangsu Wanbang Biochemical Pharmaceutical Co., Ltd, 400 U/10mL.

Polypeptide: the fractions between 17.5 and 25 min in Example 1 were collected and freeze dried to give a polypeptide mixture, and the mixture was then prepared into 2 mg/mL solution with normal saline for use.

Mouse models of type 2 diabetes: KK mice, Beijing Huafukang Biological Technology Co., Ltd., 30 mice, weighing 25 ± 2.7 g, male, had been fed with a high-fat and high-sugar diet for one month.

Blood glucose meter and test strips: Beijing Yicheng Bioelectronics Technology Co., Ltd.

### 2. Experimental method

After being fasted for 4 hours (free access to water), the above mentioned mouse models of type 2 diabetes were randomly divided into three groups, 10 mice in each group. Each of the mice was stained with picric acid as a marker and detected the blood glucose level by collecting tail vein blood. The mice in the model control group were intravenously injected once with normal saline at a dose of 0.05 ml/10g into the tail vein, mice in Group A (polypeptide hypoglycemic group) were intravenously injected once with 2 mg/mL polypeptide at a dose of 0.05 ml/10g into the tail vein, and mice in Group B (insulin hypoglycemic control group) were intravenously injected once with 0.5 U/mL insulin at a dose of 0.05 ml/10g into the tail vein. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 10 below:

**Table 10. Hypoglycemic effect of the polypeptide and insulin on mouse models of type 2 diabetes after tail vein injection**

| Mice group | Blood glucose level prior to administrati on | Blood glucose level at 15 min after administratio n | Blood glucose level at 30 min after administratio n | Blood glucose level at 45 min after administration | Blood glucose level at 60 min after administration |
|---|---|---|---|---|---|
| Model control group | 13.2±0.8 | 13.8±1.1 | 13.6±0.9 | 13.1±0.8 | 12.8±0.9 |
| Group A | 13.8±0.7 | 9.3±0.6^{∗∗} | 8.2±0.4^{∗∗} | 7.7±0.5^{∗∗} | 6.5±0.5^{∗∗} |
| Group B | 14.3±0.9 | 8.5±0.7^{∗∗} | 7.5±0.4^{∗∗} | 6.1±0.4^{∗∗} | 4.8±0.3^{∗∗} |

| | | | | | |
|---|---|---|---|---|---|
| Note: as compared with model control group, ^{∗}p<0.05, ^{∗∗}p<0.01. | | | | | |

### 4. Conclusion

From the above experimental results, it could be seen that the polypeptide mixture could lower the blood glucose level of the mouse models of type 2 diabetes after intravenous injection, exhibiting significant hypoglycemic activity -- an effect similar to that of insulin, but it would not cause hypoglycemia in the mouse models of type 2 diabetes.

### Example 13 Comparative experiment of hypoglycemic effect of polypeptide mixtures of the invention and insulin on mouse models of type 2 diabetes after subcutaneous injection

### 1. Materials and Instruments

Insulin: Jiangsu Wanbang Biochemical Pharmaceutical Co., Ltd, 400 U/10mL.

Polypeptide: the fractions between 17.5 and 25 min in Example 1 were collected and freeze dried to give a polypeptide mixture, and the mixture was then prepared into 2 mg/mL solution with normal saline for use.

Mouse models of type 2 diabetes: KK mice, Beijing Huafukang Biological Technology Co., Ltd., 30 mice, weighing 25 ± 2.7 g, male, had been fed with a high-fat and high-sugar diet for one month.

Blood glucose meter and test strips: Beijing Yicheng Bioelectronics Technology Co., Ltd.

### 2. Experimental method

After being fasted for 4 hours (free access to water), the above mentioned mouse models of type 2 diabetes were randomly divided into three groups, 10 mice in each group. Each of the mice was stained with picric acid as a marker and detected the blood glucose level by collecting tail vein blood. The mice in the model control group were subcutaneously injected once with normal saline at a dose of 0.05ml/10g on the back, mice in Group A (polypeptide hypoglycemic group) were subcutaneously injected once with 2 mg/mL polypeptide at a dose of 0.05ml/10g on the back, and mice in Group B (insulin hypoglycemic control group) were subcutaneously injected once with 0.5U/mL insulin at a dose of 0.05ml/10g on the back. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 11 below:

**Table 11. Hypoglycemic effect of the polypeptide and insulin on mouse models of type 2 diabetes after subcutaneous injection**

| Mice group | Blood glucose level prior to administrat ion | Blood glucose level at 15 min after administration | Blood glucose level at 30 min after administration | Blood glucose level at 45 min after administratio n | Blood glucose level at 60 min after administration |
|---|---|---|---|---|---|
| Model control group | 12.9±1.2 | 13.6±0.9 | 12.9±1.1 | 12.8±0.7 | 12.8±0.5 |
| Group A | 10.5±0.8 | 9.6±0.4 | 8.2±0.6^{∗} | 7.5±0.5^{∗∗} | 6.2±0.3^{∗∗} |
| Group B | 11.6±1.1 | 8.8±0.8^{∗} | 7.1±0.7^{∗∗} | 6.4±0.5^{∗∗} | 5.0±0.4^{∗∗} |
| | | | | | |

| | | | | | |
|---|---|---|---|---|---|
| Note: as compared with model control group, ^{∗}p<0.05, ^{∗∗}p<0.01. | | | | | |

### 4. Conclusion

From the above experimental results, it could be seen that the polypeptide mixture could lower the blood glucose level of the mouse models of type 2 diabetes after subcutaneous injection, exhibiting significant hypoglycemic activity -- an effect similar to that of insulin, but it would not cause hypoglycemia in the mouse models of type 2 diabetes.

### Example 14 Comparative experiment of hypoglycemic effect of polypeptide mixtures of the invention and insulin on mouse models of type 2 diabetes after intramuscular injection

### 1. Materials and Instruments

Insulin: Jiangsu Wanbang Biochemical Pharmaceutical Co., Ltd, 400 U/10mL.

Polypeptide: the fractions between 17.5 and 25 min in Example 1 were collected and freeze dried to give a polypeptide mixture, and the mixture was then prepared into 2 mg/mL solution with normal saline for use.

Mouse models of type 2 diabetes: KK mice, Beijing Huafukang Biological Technology Co., Ltd., 30 mice, weighing 25 ± 2.7 g, male, had been fed with a high-fat and high-sugar diet for one month.

Blood glucose meter and test strips: Beijing Yicheng Bioelectronics Technology Co., Ltd.

### 2. Experimental method

The above mentioned white mice were randomly divided into three groups, 10 mice in each group. Each of the mice was stained with picric acid as a marker and detected the blood glucose level by collecting tail vein blood. The mice in the model control group were intramuscularly injected once with normal saline at a dose of 0.05ml/10g in the lateral thigh, mice in Group A (polypeptide hypoglycemic group) were intramuscularly injected once with 2mg/mL polypeptide at a dose of 0.05ml/10g in the lateral thigh, and mice in Group B (insulin hypoglycemic control group) were intramuscularly injected once with 0.5U/mL insulin at a dose of 0.05ml/10g in the lateral thigh. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 12 below:

**Table 12. Hypoglycemic effect of the polypeptide and insulin on mouse models of type 2 diabetes after intramuscular injection**

| Mice group | Blood glucose level prior to administratio n | Blood glucose level at 15 min after administration | Blood glucose level at 30 min after administration | Blood glucose level at 45 min after administration | Blood glucose level at 60 min after administration |
|---|---|---|---|---|---|
| Model control group | 14.5±1.3 | 14.9±1.2 | 14.6±0.9 | 13.8±1.1 | 13.2±1.9 |
| Group A | 12.6±0.9 | 11.2±1.3 | 9.8±0.9^{∗} | 8.3±0.7^{∗∗} | 6.6±0.5^{∗∗} |
| Group B | 13.4±1.1 | 11.1±0.9 | 9.6±0.7^{∗} | 7.3±0.8^{∗∗} | 5.2±0.4^{∗∗} |

| | | | | | |
|---|---|---|---|---|---|
| Note: as compared with model control group, ^{∗}p<0.05, ^{∗∗}p<0.01. | | | | | |

### 4. Conclusion

From the above experimental results, it could be seen that the polypeptide mixtures of the invention could lower the blood glucose level of the mouse models of type 2 diabetes after intramuscular injection, exhibiting significant hypoglycemic activity -- an effect was similar to that of insulin, but it would not cause hypoglycemia in the mouse models of type 2 diabetes.

### Example 15 Stability test of the polypeptide mixture of the invention against pepsin and trypsin

### 1. Materials and Instruments

Polypeptide: the fractions between 15 and 25 min in Example 1 were collected and freeze dried to give a polypeptide composition;
Pepsin: Sigma-Aldrich Company Ltd.;
Trypsin: Sigma-Aldrich Company Ltd.;
Albumin: Serum albumin (bovine) standard;
High-performance liquid chromatography: Agilent 1260 high-performance liquid chromatography, Waters Corporation C18 reversed-phase column (4.6 x 250 mm, 300SB, 5 □m).

### 2. Experiment method

Polypeptides were subject to enzymatic hydrolysis in simulated gastric and simulated intestinal fluids, respectively, to verify whether the polypeptides were resistance to the enzymatic hydrolysis of digestive enzymes. Control experiments were performed with albumin.

Preparation of simulated gastric fluid: water was added to diluted hydrochloric acid with a concentration of 1 mol/L, to adjust the pH to 1.5. 1 g pepsin per 100 mL solution was added, and after homogeneously mixing, the solution was filtered through a 0.22 □m sterile filter for use.

Preparation of simulated intestinal fluid: 6.8 g of potassium dihydrogen phosphate was dissolved in 500 mL water, 0.4% (w/w) NaOH was added to adjust the pH to 6.8, and water was added to make up 1L. 1 g trypsin per 100 mL solution was added, and after homogeneously mixing, the solution was filtered through a 0.22 □m sterile filter for use.

10 □L of the polypeptide solution (10 mg/mL) was added to the simulated gastric fluid (1mL) and simulated intestinal fluid (1mL), respectively, and after homogeneously mixing, the mixtures were incubated in a water bath at 37 °C for 2 hours.

1 mg of dried albumin powder was added to the simulated gastric fluid (1mL) and simulated intestinal fluid (1mL), respectively, and after homogeneously mixing, the mixtures were incubateed in a water bath at 37 °C for 2 hours.

The above enzymatic mixtures were detected by using HPLC.

Chromatographic conditions: mobile phase, water (containing 0.1% TFA) + acetonitrile (containing 0.1% TFA), gradient elution from 15% to 50% acetonitrile for 25 min, flow rate of 1 mL/min, detection wavelength of 214 nm, injection volume of 20 ul.

### 3. Experimental results

The HPLC chromatograms of enzymatic hydrolysis of the polypeptides and albumin were shown in the attached figures.

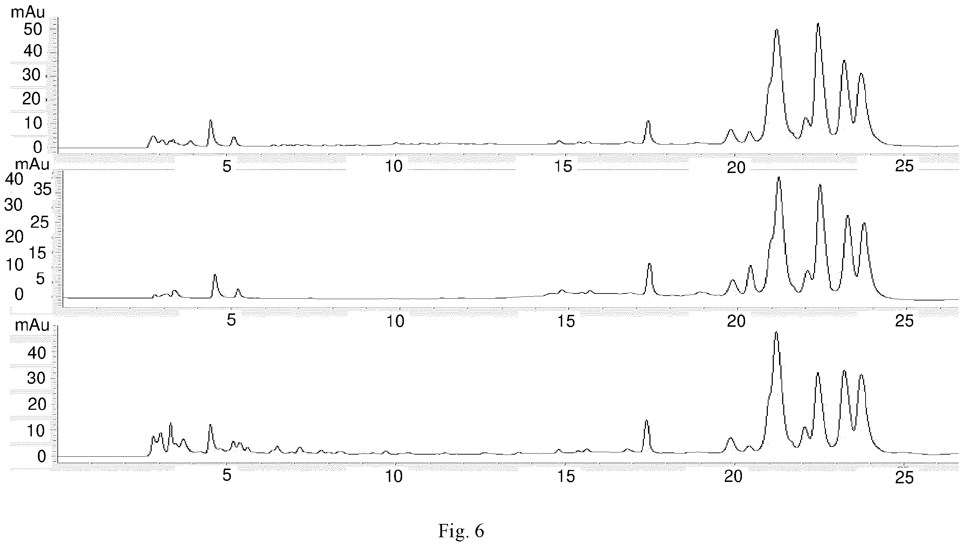

In the HPLC chromatograms of enzymatic hydrolysis of the polypeptides (see Fig. 6), the chromatogram at the top showed the HPLC detection result of the polypeptide reference, the chromatogram in the middle showed the HPLC detection result of enzymatic hydrolysis of the polypeptides in simulated gastric fluid for 2 hours, and the chromatogram at the bottom showed the HPLC detection result of enzymatic hydrolysis of the polypeptides in simulated intestinal fluid for 2 hours.

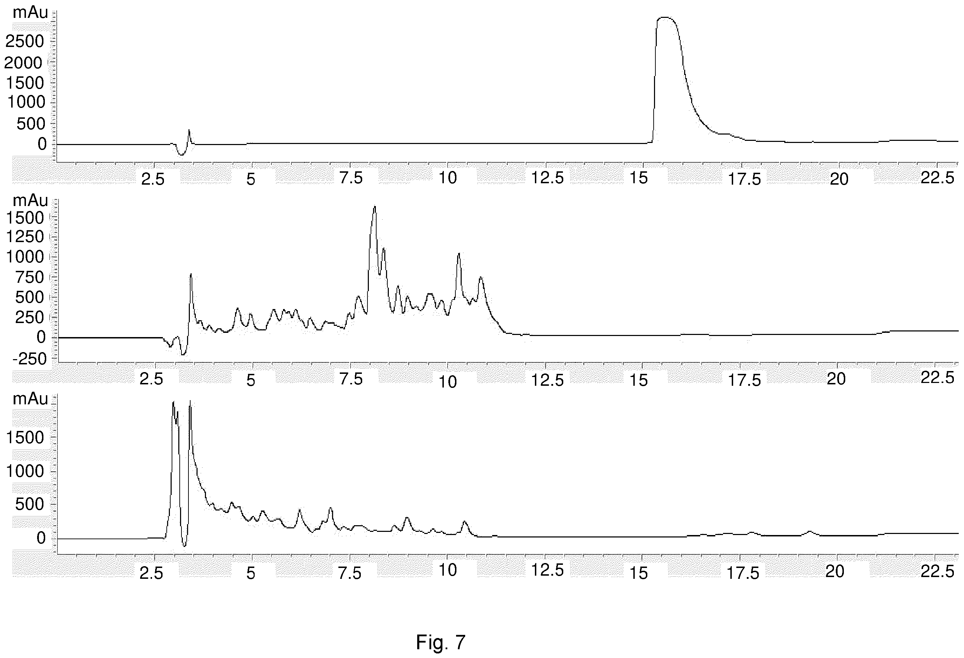

In the HPLC chromatograms of enzymatic hydrolysis of albumin (see Fig. 7), the chromatogram at the top showed the HPLC detection result of the albumin reference, the chromatogram in the middle showed the HPLC detection result of enzymatic hydrolysis of albumin in simulated gastric fluid for 2 hours, and the chromatogram at the bottom showed the HPLC result of enzymatic hydrolysis of albumin in simulated intestinal fluid for 2 hours.

### 4. Conclusion

As shown in the figure, the polypeptides of the invention could resist the enzymatic hydrolysis of both simulated gastric fluid and intestinal fluid, but albumin was completely enzymatically hydrolyzed in the gastric fluid and intestinal fluid, indicating that the polypeptides of the invention were resistant to enzymatic hydrolysis of digestive enzymes in gastrointestinal tract and could be administered orally.

### Example 16 Comparative experiment of hypoglycemic effect of polypeptide mixtures of the invention and metformin on mouse models of type 2 diabetes after administration by gavage

### 1. Materials and Instruments

Polypeptide: the fractions between 17.5 and 25 min in Example 1 were collected and freeze dried to give a polypeptide mixture, and the mixture was then prepared into 2 mg/mL solution with normal saline for use.

Metformin hydrochloride: GLUMETZA metformin hydrochloride tablets, 0.5g/Tablet, Sino-American Shanghai Squibb Pharmaceutical Co., Ltd., was prepared into 20 mg/mL solution with normal saline for use.

Mouse models of type 2 diabetes: KK mice, Beijing Huafukang Biological Technology Co., Ltd., 30 mice, weighing 25 ± 2.7 g, male, had been fed with a high-fat and high-sugar diet for one month.

Blood glucose meter and test strips: Beijing Yicheng Bioelectronics Technology Co., Ltd.

### 2. Experimental method

The above mentioned white mice were randomly divided into three groups, 10 mice in each group. Each of the mice was stained with picric acid as a marker and detected the blood glucose level by collecting tail vein blood. The mice in the model control group were administered once by gavage with normal saline at a dose of 0.05ml/10g, mice in Group A (polypeptide hypoglycemic group) were administered once by gavage with polypeptide at a dose of 0.05ml/10g, and mice in Group B (metformin control group) were administered once by gavage with metformin at a dose of 0.05ml/10g. Blood samples were collected from the tail vein at 15 min, 30 min, 45 min and 60 min after injection, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 13 below:

**Table 13. Hypoglycemic effect of the polypeptides and metformin on mouse models of type 2 diabetes after administration by gavage**

| Mice group | Blood glucose level prior to administratio n | Blood glucose level at 15 min after administration | Blood glucose level at 30 min after administration | Blood glucose level at 45 min after administration | Blood glucose level at 60 min after administration |
|---|---|---|---|---|---|
| Model control group | 13.7±1.5 | 12.8±1.2 | 11.3±0.9 | 10.8±1.1 | 10.2±0.9 |
| Group A | 12.4±1.3 | 11.3±0.9 | 9.9±0.8^{∗} | 7.2±0.6^{∗∗} | 6.7±0.4^{∗∗} |
| Group B | 14.2±1.1 | 12.6±1.2 | 10.4±0.9^{∗} | 9.3±0.8^{∗} | 8.2±0.5^{∗∗} |

| | | | | | |
|---|---|---|---|---|---|
| Note: as compared with model control group, ^{∗}p<0.05, ^{∗∗}p<0.01. | | | | | |

### 4. Conclusion

From the above experimental results, it could be seen that the polypeptide mixtures of the invention could lower the blood glucose level of the mouse models of type 2 diabetes after administration by gavage, exhibiting significant hypoglycemic activity, without causing hypoglycemia in the mouse models of type 2 diabetes. Moreover, the effect of the polypeptide mixtures of the invention was better than that of metformin.

### Example 17 Accelerated stability test of the polypeptide mixtures of the invention

### 1. Materials and Instruments

Polypeptide: the fractions between 15 and 25 min in Example 1 were collected and freeze dried to give a polypeptide composition.

High-performance liquid chromatography: Agilent 1260 high-performance liquid chromatography, Waters Corporation C18 reversed-phase column (4.6 x 250 mm, 300SB, 5 □m).
2. Experiment method: the polypeptides were distributed into cillin bottles, and then the cillin bottles were placed in a chamber at constant temperature of 60 °C and constant humidity of 70% RH to carry out accelerated tests. The polypeptides were weekly sampled and detected by high performance liquid chromatography (HPLC) to determine the stability of the polypeptides.

### 3. Experimental results

The HPLC chromatograms for the stability studies of the polypeptides were shown in the attached figures.

### 4. Conclusion

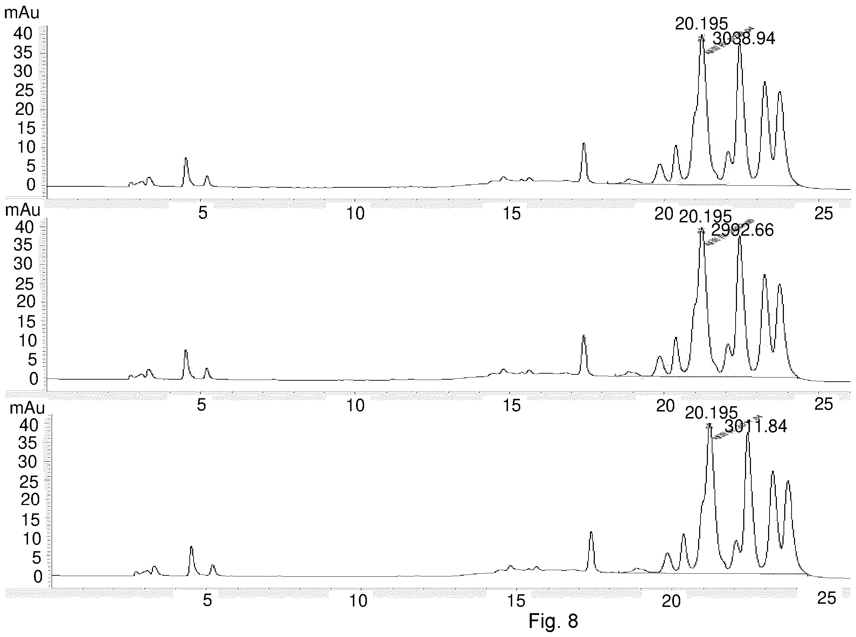

The HPLC chromatograms for the stability studies of the polypeptides (see Fig. 8) were three HPLC detection results of the polypeptide stability experiments. The chromatogram at the top was for the initial reference, having an integral area of the polypeptide mixture of about 3036.94. The chromatogram in the middle showed the HPLC dectection result after 1 week under the accelerated conditions; more particularly, the retention time of the main peak was unchanged, and the integral area of the polypeptide mixture was about 2992.66. The chromatogram at the bottom showed the HPLC detection result after 2 weeks under accelerated conditions; more particularly, the retention time of the main peak was unchanged, and the integral area of the polypeptide mixture was about 3011.84. The results indicated that the polypeptides were stable during accelerated testing, could be stored at room temperature, and were suitable for being developed into a hypoglycemic agent.

### Example 18 Experiment of therapeutic effects of the polypeptide mixtures on mouse models of type 2 diabetes after injection

### 1. Materials and Instruments

Polypeptide: the fractions between 17.5 and 25 min were collected and freeze dried to give a polypeptide mixture, and the mixture was then prepared into 1 mg/mL solution with normal saline to obtain an injection solution of the polypeptide mixture.

Mouse models of type 2 diabetes: KK mice, Beijing Huafukang Biological Technology Co., Ltd., 40 mice, weighing 25 ± 2.7 g, male, had been fed with a high-fat and high-sugar diet for one month.

Blood glucose meter and test strips: Beijing Yicheng Bioelectronics Technology Co., Ltd.

### 2. Experimental method

The above mentioned white mice were randomly divided into four groups, 10 mice in each group. Each of the mice was stained with picric acid as a marker and detected the blood glucose level by collecting tail vein blood. The mice in the model control group were intravenously injected with normal saline at a dose of 0.05ml/10g into the tail vein, mice in Group A (intravenous injection group) were intravenously injected with an injection containing 1 mg/mL polypeptide at a dose of 0.05ml/10g into the tail vein, mice in Group B (subcutaneous injection group) were subcutaneously injected with an injection containing 1 mg/mL polypeptide at a dose of 0.05ml/10g on the back, and mice in Group C (intramuscular injection group) were intramuscularly injected with an injection containing 1 mg/mL polypeptide at a dose of 0.05ml/10g in the lateral thigh. The mice were administered every other day for 4 consecutive weeks, blood samples were collected from the tail vein weekly at regular intervals, respectively, and blood glucose level in mmol/L was determined with the blood glucose meter.

The experimental data was expressed in X̅±s, and the differences between groups were determined by t-test, with p<0.05 indicating a significant difference.

### 3. The experimental results are shown in Table 14 below:

**Table 14. Therapeutic effects of the polypeptide mixtures on mouse models of type 2 diabetes after intravenouse, subcutaneous and intramuscular injection**

| Mice | Blood glucose level | Blood glucose level | Blood glucose level | Blood glucose level |
|---|---|---|---|---|
| group | after one week | after two weeks | after three weeks | after four weeks |
| Model control group | 9.8±0.7 | 10.5±1.1 | 11.6±1.3 | 13.7±1.2 |
| A | 6.3±0.7^{∗} | 6.5±0.5^{∗∗} | 6.4±0.4^{∗∗} | 6.2±0.3^{∗∗} |
| B | 6.7±0.5^{∗} | 6.4±0.4^{∗∗} | 6.5±0.3^{∗∗} | 6.3±0.3^{∗∗} |
| C | 6.4±0.7^{∗} | 6.1±0.8^{∗∗} | 6.3±0.4^{∗∗} | 6.2±0.5^{∗∗} |

| | | | | |
|---|---|---|---|---|
| Note: as compared with model control group, ^{∗}p<0.05, ^{∗∗}p<0.01. | | | | |

### 4. Conclusion

From the above experimental results, it could be seen that the polypeptides of the invention had explicit therapeutic effects on mouse models of type 2 diabetes after intravenous, subcutaneous and intramuscular injection, and the hypoglycemic effects following the three administration ways had no significant difference.

### Example 19 Comparative experiments of therapeutic effects, insulin resistance, oral glucose tolerance and repairing of pancreatic β cell of the polypeptide mixtures and metformin injected on mouse models of type 2 diabetes after administion by gavage

### 1. Materials and Instruments

Polypeptide: the fractions between 17.5 and 25 min in Example 1 were collected and freeze dried to give a polypeptide mixture.

Metformin hydrochloride: GLUMETZA metformin hydrochloride tablets, 0.5g/Tablet, Sino-American Shanghai Squibb Pharmaceutical Co., Ltd.

Insulin: Jiangsu Wanbang Biochemical Pharmaceutical Co., Ltd, 400 U/10mL.

Mouse models of type 2 diabetes: KK mice, Beijing Huafukang Biological Technology Co., Ltd., 60 mice, weighing 25 ± 2.7 g, male, had been fed with a high-fat and high-sugar diet for one month.

Normal control mice: Kunming white mice, from Hubei Epidemic Prevention Station, 20 mice, weighing 20 ± 2 g, male, had been fed with normal diet.

Blood glucose meter and test strips: Beijing Yicheng Bioelectronics Technology Co., Ltd.

### 2. Experimental method

2.1 Preparation of oral polypeptide hypoglycemic agents: the above polypeptide mixture was mixed with hydroxypropyl methylcellulose at ratio of 8:2 homogeneously, and then was compressed into tablets. Each tablet weighed 0.5 g and contained 392 mg of the total effective polypeptides. Before the administration by gavage, the tablets were prepared into a suspension containing 2 mg/ml of the polypeptides with normal saline.

Metformin hydrochloride was prepared into 20 mg/mL solution with normal saline for use.

2.2 The above mentioned mouse models of type 2 diabetes were randomly divided into three groups, 20 mice in each group. The 20 normal control mice were assigned as one group. Each of the mice was stained with picric acid as a marker and detected the blood glucose level by collecting tail vein blood. The mice in the model control group and normal control group were administered by gavage with normal saline at a dose of 0.10ml/10g once a day for 4 consecutive weeks; mice in Group A (metformin treatment group) were administered by gavage with metformin at a dose of 0.05ml/10g once a day for 4 consecutive weeks; and mice in Group B (polypeptide treatment group) were administered by gavage with the polypeptides at a dose of 0.05ml/10g once a day for 4 consecutive weeks. Blood samples were collected weekly from the tail vein, respectively, and blood glucose level in mmol/L was determined with the blood glucose meter.

2.3 Insulin tolerance test: one hour after the last administration, 10 mice in each of the diabetic model control group, metformin treatment group, polypeptide treatment group and normal control group were randomly selected and subcutaneously injected once with 0.5U/mL insulin at a dose of 0.05ml/10g. Blood samples were collected from the tail vein at 0 h, 0.5 h, 1 h, 1.5 h and 2 h, respectively, and blood glucose level in mmol/L was determined with the blood glucose meter.

2.4 Oral glucose tolerance test: one hour after the last administration, the remaining mice in the diabetic model control group, metformin treatment group, polypeptide treatment group and normal control group (10 mice in each group) after the selection in experiment 2.3 were administered by gavage with glucose at a dose of 2.5g/kg. Blood samples were collected from the tail vein at 0 h, 0.5 h, 1 h, 1.5 h and 2 h, respectively, and blood glucose level in mmol/L was determined with the blood glucose meter.

2.5 All mice were sacrificed after treatment for four weeks, and the pancreatic tissues were weighed, stained with H&E and detected by immunohistochemistry.

The experimental data was expressed in X±s, and the differences between groups were determined by t-test, with p<0.05 indicating a significant difference.

### 3. The experimental results are shown in the Tables below:

**Table 15. Blood glucose level of mice in each group during treatment**

| Mice group | Initial blood glucose level | Blood glucose level after one week | Blood glucose level after two weeks | Blood glucose level after three weeks | Blood glucose level after four weeks |
|---|---|---|---|---|---|
| Model control group | 9.1±1.8 | 10.3±0.9 | 11.8±1.3 | 11.6±1.1 | 12.4±0.8 |
| A | 9.3±1.1 | 8.1±1.1^{∗} | 7.2±0.9^{∗} | 7.3±1.2^{∗} | 6.8±0.8^{∗∗} |
| B | 9.2±0.9 | 7.4±0.7^{∗} | 6.4±0.8^{∗∗} | 6.5±1.3^{∗∗} | 6.3±0.7^{∗∗} |
| Normal control group | 6.7±0.4 | 6.9±0.3 | 6.8±0.4 | 6.6±0.2 | 6.5±0.1 |

| | | | | | |
|---|---|---|---|---|---|
| Note: as compared with model control group, ^{∗}p<0.05, ^{∗∗}p<0.01. | | | | | |

**Table 16. Insulin tolerance test**

| Mice group | 0min blood glucose level | 30min blood glucose level | 60min blood glucose level | 90min blood glucose level | 120min blood glucose level |
|---|---|---|---|---|---|
| Model control group | 12.4±0.8 | 8.6±2.8 | 9.5±4.2 | 10.2±1.7 | 12.1±2.5 |
| A | 6.8±0.8 | 4.1±1.1^{∗} | 4.8±1.2^{∗} | 5.4±0.7^{∗} | 6.2±1.3 |
| B | 6.3±0.7 | 3.2±0.7^{∗∗} | 4.3±0.5^{∗} | 4.6±0.6^{∗} | 5.7±0.7^{∗} |
| Normal control group | 6.2±0.7 | 4.7±0.4 | 3.1±0.5 | 2.4±0.3 | 2.1±0.2 |

| | | | | | |
|---|---|---|---|---|---|
| Note: as compared with model control group, ^{∗}p<0.05, ^{∗∗}p<0.01. | | | | | |

**Table 17. Oral glucose tolerance test**

| Mice group | 0min blood glucose level | 30min blood glucose level | 60min blood glucose level | 90min blood glucose level | 120min blood glucose level |
|---|---|---|---|---|---|
| Model control group | 12.4±0.8 | 28.2±3.7 | 22.1±2.8 | 18.5±4.2 | 13.1±2.5 |
| A | 6.8±0.8 | 16.8±4.1^{∗} | 13.4±2.4^{∗} | 10.6±3.2 | 8.2±1.7 |
| B | 6.3±0.7 | 15.6±2.9^{∗∗} | 11.2±1.7^{∗} | 8.3±2.1^{∗} | 6.7±1.3^{∗} |
| Normal control group | 6.4±0.9 | 16.1±3.9 | 10.5±2.3 | 7.8±1.6 | 6.5±0.9 |

| | | | | | |
|---|---|---|---|---|---|
| Note: as compared with model control group, ^{∗}p<0.05, ^{∗∗}p<0.01. | | | | | |

**Table 18. Pancreas weights of mice in each group**

| Mice group | Pancreas weight/body weight (%) |
|---|---|
| Model control group | 1.21±0.04 |
| A | 1.32±0.06 |
| B | 1.86±0.02^{∗∗} |
| Normal control group | 2.08±0.09 |

| | |
|---|---|
| Note: as compared with model control group, ^{∗}p<0.05, ^{∗∗}p<0.01. | |

### 4. Conclusion

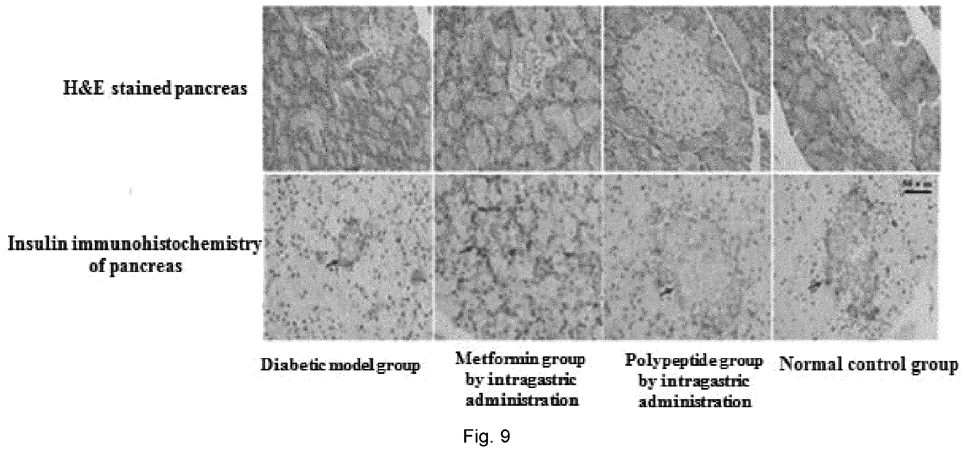

The hypoglycemic agent prepared from the polypeptide mixture could lower the blood glucose level of the mouse models of type 2 diabetes after oral administration and restore the blood glucose to normal level, indicating that the hypoglycemic agent could treat type 2 diabetes by oral administration. Moreover, the polypeptide mixtures could improve insulin tolerance and glucose tolerance of the mouse models of type 2 diabetes after administration by gavage. Meanwhile, the pancreas weights were increased in the mouse models of type 2 diabetes treated with the hypoglycemic agent. Fig. 9 showed that pancreatic β cells returned to normal, indicating that the hypoglycemic agent also had the effect of repairing pancreatic β cells in the mouse models of type 2 diabetes.

### Example 20 Liquid composition comprising the polypeptide mixture of the invention and amino acid

The fractions between 17.5 and 25 min in Example 1 were collected and freeze dried to give a polypeptide mixture, in which the total content of the 12 polypeptides with amino aicd sequence of SEQ ID No.1 to SEQ ID No.12 was ≥98%, abbreviated as polypeptide O.

A liquid composition comprising polypeptide O was prepared according to formulation ① as shown in Table 19 below.

**Table 19. Formulation ①**

| Material | Amount |
|---|---|
| Polypeptide O | 100 mg |
| Sodium acetate trihydrate | 200 mg |
| Acetic acid | adjusting pH to 4.5 |
| 85% Glycerol | 1500 mg |
| Water for injection | making up to 100 mL |

A liquid composition comprising polypeptide O was prepared according to formulation ② as shown in Table 20 below.

**Table 20. Formulation ②**

| Material | Amount |
|---|---|
| Polypeptide O | 100 mg |
| L-methionine | 10 mg |
| Sodium acetate trihydrate | 200 mg |
| Acetic acid | adjusting pH to 4.5 |
| 85% Glycerol | 1500 mg |
| Water for injection | making up to 100 mL |

The liquid composition as prepared according to formulations ① and ② were sealed in cillin bottles and stored at 40±2°C/75±5%RH, respectively.

### Example 21 Hypoglycemic activity of the liquid composition comprising the polypeptides of the invention and amino acid

### 1. Materials and Instruments

Polypeptide composition A: a liquid composition, which was prepared according to formulation ① in Example 20 and stored at 40±2°C/75±5%RH for 6 months.

Polypeptide composition B: a liquid composition, which was prepared according to formulation ② in Example 20 and stored at 40±2°C/75±5%RH for 6 months.

Polypeptide composition C: a liquid composition, which was currently prepared according to formulation ① in Example 20.

Experimental animals: Kunming white mice, male, weighing 20 ± 2 g, 40 mice, were purchased from Hubei Epidemic Prevention Station.

Blood glucose meter and test strips: Roche Accu-Chek^{®} Active Blood Glucose Meter and test stripes.

### 2. Experimental method

After being fasted, the above mentioned white mice were randomly divided into four groups, 10 mice in each group. Each of the mice was stained with picric acid as a marker and detected the blood glucose level by collecting tail vein blood. The mice in the blank control group were intravenously injected once with normal saline at a dose of 0.1ml/10g into the tail vein, and mice in the other groups were intravenously injected once with compositions A, B, and C at a dose of 0.10ml/10g into the tail vein, respectively. Blood samples were collected from the tail vein at 15 min, 30 min, 45 min and 60 min after injection, respectively, and blood glucose level in mmol/L was determined with the blood glucose meter.

The experimental data was expressed in X±s, and the differences between groups were determined by t-test, with p<0.05 and p<0.01 indicating a siginificant difference and a very significant difference, respectively.

### 3. The experimental results are shown in Table 21 below:

**Table 21. Hypoglycemic activity of the liquid composition comprising the polypeptide and amino aicd on normal mice after tail vein injection**

| Mice group | Blood glucose level prior to administration | Blood glucose level at 15 min after administrati on | Blood glucose level at 30 min after administra tion | Blood glucose level at 45 min after administr ation | Blood glucose level at 60 min after administrati on |
|---|---|---|---|---|---|
| Blank control group | 9.0±0.5 | 8.5±0.6 | 8.2±0.8 | 7.7±0.4 | 7.4±0.6 |
| Composition A group | 8.8±0.6 | 6.5±0.4 ## | 4.9±0.5 ## | 4.2±0.5 ## | 4.8±0.4 ## |
| Composition B group | 8.9±0.8 | 6.2±0.5 ## | 3.7±0.5 ## ^{∗∗}△ | 1.8±0.4 ## ^{∗∗}△ | 0.8±0.3 ## ^{∗∗} |
| Composition C group | 8.2±0.7 | 6.4±0.6 ## | 4.2±0.6 ## ^{∗} | 2.5±0.3 ## ^{∗∗} | 1.0±0.2 ## ^{∗∗} |

| | | | | | |
|---|---|---|---|---|---|
| Note: #, ## represents the comparison with black control group having significant difference (p<0.05) and very significant difference (p<0.01), respectively; ^{∗}, ^{∗∗} represents the comparison with composition A group having significant difference (p<0.05) and very significant difference (p<0.01), respectively; and △, △△ represents the comparison with composition B group and composition C group having significant difference (p<0.05) and very significant difference (p<0.01), respectively. | | | | | |

### 4. Conclusion

From the above experimental results, it could be seen that 15 minutes after the tail vein injection with composition A, B or C, respectively, the blood glucose level of all the normal mice began to drop. Mice in both Composition B group and Composition C group had especially significant decrease in blood glucose level, and showed hypoglycemia at 45 min. Mice in Composition A group did not show hypoglycemia, but showed a rebound of the blood glucose level at 60 min. The resultes demonstrated that the hypoglycemic activity of composition A without L-methionine decreased with the increasing storage duration, while that of composition B with L-methionine did not decrease with the increasing storage duration; moreover, the hypoglycemic activity of composition B was better than that of freshly prepared composition C, suggesting a synergistic effect of the components.

The above experiments demonstrated that the addition of L-methionine could improve the stability and hypoglycemic activity of polypeptide O.

### Example 22 Solid composition comprising the polypeptides of the invention and amino acid

A solution comprising polypeptide O was prepared according to formulation ③ in Table 22 below, distributed in cillin bottles, vacuum freeze dried, and stored at 40±2°C/75±5%RH after being sealed:

**Table 22. Formulation ③**

| Material | Amount |
|---|---|
| Polypeptide O | 10 g |
| Sodium Chloride | 100 mg |
| | |
| D-mannitol | 500 mg |
| EDTA disodium salt | 2 mg |
| Deionized water | making up to 100 mL |

A solution comprising polypeptide O and amino acide was prepared according to formulation ④ in Table 23 below, distributed in cillin bottles, vacuum freeze dried, and stored at 40±2°C/75±5%RH after being sealed:

**Table 23. Formulation ④**

| Material | Amount |
|---|---|
| Polypeptide O | 10 g |
| DL-methionine | 500 mg |
| D-mannitol | 100 mg |
| EDTA disodium salt | 2 mg |
| Deionized water | making up to 100 mL |

### Example 23 Hypoglycemic activity of the solid composition comprising the polypeptides of the invention and amino acid

### 1. Materials and Instruments

Polypeptide composition D: a solid composition was prepared according to formulation ③ in Example 22, stored at 40±2°C/75±5%RH for 6 months, and was then prepared into a solution containing 2 mg/mL polypeptide O with normal saline for use.

Polypeptide composition E: a solid composition was prepared according to formulation ④ in Example 22, stored at 40±2°C/75±5%RH for 6 months, and was then prepared into a solution containing 2 mg/mL polypeptide O with normal saline for use.

Polypeptide composition F: a solid composition was currently prepared according to formulation ③ in Example 23, and then prepared into a solution containing 2 mg/mL polypeptide O with normal saline for use.

Metformin: GLUMETZA metformin hydrochloride tablets, 0.5g/Tablet, Sino-American Shanghai Squibb Pharmaceutical Co., Ltd., was prepared into 20 mg/mL suspension with normal saline for use.

High-fat diet: 30% fat, 20% protein, and 50% carbohydrate.

Experimental animals: C57BL/6, male, weighing 20 ± 2 g, 100 mice, were purchased from Hubei Epidemic Prevention Station.

Blood glucose meter and test strips: Roche Accu-Chek^{®} Active Blood Glucose Meter and test stripes.

### 2. Experimental method

Male C57BL/6 mice at 4 weeks of age, after adapting to the environment for 5 days, were randomly divided into normal control group (10 mice) and model-to-be established groups (90 mice). The mice in the normal control group were fed with normal diet. The mice in the model-to-be established groups were fed with high-fat diet and intraperitoneally injected with streptozotocin (STZ) in citrate buffer at a dose of 45 mg/kg. After injection, the mice were fasted for 1.5 hours and injected for 5 consecutive days, and then the diabetic mouse model was established. After intraperitoneal injection of streptozotocin for four weeks, the mice were fasted from 8 pm the day before, blood samples were collected from the tail vein at 8 am in the morning of the day, and then the fasting blood glucose level was measured. The mice with the fasting blood glucouse level between 10.0 and 20.0 mmol/L and at the same time having polydipsia and polyuria were selected out as the diabetic mice. Then, the selected diabetic mice were randomly divided into five groups: model control group, metformin group, Composition D group, Composition E group, and Composition E group, 10 mice in each group. The mice were administered by gavage with the corresponding drug: normal saline (100 ·L for each mouse), metformin (0.05 ml/10g), composition D (0.05 ml/10g), composition E (0.05 ml/10g) and composition F (0.05 ml/10g), respectively, once a day for 4 weeks. The mice in normal control group were administered with normal saline (100 □L for each mouse). The diabetic mice were fed with high-fat diet continuously and drank water freely, and the mice in normal control group were fed with normal diet and drank water freely.

Blood samples of mice in each group were collected from the tail vein weekly at regular intervals, respectively, and fasting blood glucose level in mmol/L was determined with the blood glucose meter once.

The experimental data was expressed in X±s, and the differences between groups were determined by t-test, with p<0.05 and p<0.01 indicating a siginificant difference and a very significant difference, respectively.

### 3. The experimental results are shown in Table 24 below:

**Table 24. Hypoglycemic acitivity of the solid composition comprising the polypeptides and amino aicd on mouse models of type 2 diabetes after administration by gavage**

| Mice group | Blood glucose level after one week | Blood glucose level after two weeks | Blood glucose level after three weeks | Blood glucose level after four weeks |
|---|---|---|---|---|
| Normal control group | 4.8±0.3 | 5.1±0.2 | 4.7±0.2 | 4.6±0.3 |
| Model control group | 12.5±0.7 | 13.6±0.8 | 15.1±0.3 | 16.5±0.4 |
| Metformin group | 11.9±0.6 | 13.2±0.5 | 11.3±1.2 ## | 9.2±0.9 ## |
| Composition D group | 12.2±0.4 | 10.4±1.0 ## ^{∗∗} | 7.8±0.5 ## ^{**} | 6.9±0.6 ## ^{∗∗} |
| Composition E group | 13.6±0.8 | 8.5±0.5 ## ^{∗∗}★ | 6.6±0.7 ## ^{∗∗}★ | 4.8±0.5 ## ^{∗∗}★ |
| Composition F group | 12.5±0.9 | 9.7±0.8 ## ^{∗∗} | 7.6±0.7 ## ^{∗∗} | 5.2±0.5 ## ^{∗∗}△ |

| | | | | |
|---|---|---|---|---|
| Note: #, ## represents the comparison with model control group having significant difference (p<0.05) and very significant difference (p<0.01), respectively; ^{∗}, ^{∗∗} represents the comparison with metformin group having significant difference (p<0.05) and very significant difference (p<0.01), respectively; and ★, ★★ represents the comparison with Composition E group and Composition F group having significant difference (p<0.05) and very significant difference (p<0.01), respectively. | | | | |

### 4. Conclusion

The above experimental results showed that, the hypoglycemic activity of the solid composition comprising polypeptide O (composition D) after storage for 6 months decreased as compared with that of Composition F as freshly prepared, but was better than that of metformin (100mg/Kg) at a conventional dose. The solid composition comprising polypeptide O and an amino acid (Composition E) of the invention could effectively control blood glucose in type 2 diabetic mice fed with STZ in combination with high-fat and high-sugar diet. After four weeks of treatment, there was no significant difference in the blood glucose levels between the mice treated with Composition E and those in normal control group. The therapeutic effect of Composition E was remarkable, better than that of Composition F without an amino acid, and much better than that of metformin. Therefore, DL-methionine not only improved the stability of polypeptide O, but also had a synergistic effect on the hypoglycemic effect.

### Example 24 Improvement in glycolipid metabolism in diabetic mice treated with the composition comprising the polypeptides of the invention and amino acid

### 1. Materials and Instruments

Polypeptide composition F: a solid composition was currently prepared according to formulation ③ in Example 22, and then prepared into a solution containing 2 mg/mL polypeptide O with normal saline for use.

Polypeptide composition E: a solid composition was prepared according to formulation ④ in Example 22, stored at 40±2°C/75±5%RH for 6 months, and then prepared into a solution containing 2 mg/mL polypeptide O with normal saline for use.

High-fat diet: 30% fat, 20% protein, and 50% carbohydrate.

Experimental animals: BALB/c, male, weighing 20 ± 2 g, 80 mice, were purchased from Hubei Epidemic Prevention Station.

Metformin: GLUMETZA metformin hydrochloride tablets, 0.5g/Tablet, Sino-American Shanghai Squibb Pharmaceutical Co., Ltd., were prepared into 200 mg/mL suspension with normal saline for use.

Blood glucose meter and test strips: Roche Accu-Chek^{®} Active Blood Glucose Meter and test stripes.

Biochemical analyzer and assay kits: cholesterol (TC) assay kits, triglyceride (TG) assay kits and high-density lipoprotein cholesterol (HDL-C) assay kits were purchased from Mindray Bio-medical Electronics Co., Ltd. and tested with Mindray BS-220 Automatic Biochemical Analyzer.

### 2. Experimental method

Male BALB/c mice at 7 weeks of age, after adapting to the environment for 2 days, were randomly divided into normal control group (10 mice) and model-to-be established group (70 mice). The mice in the normal control group were fed with normal diet. The mice in the model-to-be established group were fed with high-fat diet, intraperitoneally injected with streptozotocin (STZ) in citrate buffer at a dose of 100 mg/kg, and then the diabetic mouse model was established. Three days later, the mice were fasted for 4 hours (free access to water), blood samples were collected from the tail vein and the blood glucose level were measured. The mice with the blood glucouse level higher than 11.0 mmol/L and at the same time having polydipsia and polyuria were selected out as the diabetic mice. Then the selected diabetic mice were randomly divided into four groups: model control group, metformin group, Composition E group and Composition F group, 10 mice in each group. The mice were intragastric administered with the corresponding drugs: normal saline (100 □L for each mouse), metformin (0.05 ml/10g), Composition E (0.05 ml/10g) and Composition F (0.05 ml/10g), respectively, once a day for 4 weeks. The mice in normal control group were administered with normal saline (100 □L for each mouse). The diabetic mice were fed with high-fat diet continuously and drank water freely, and the mice in normal control group were fed with normal diet and drank water freely.

Blood samples of the mice in each group were collected from the tail vein weekly at regular intervals, respectively, and fasting blood glucose level in mmol/L was determined with the blood glucose meter once.

After the end of experiment, blood samples were collected from the eyeballs of the mice, serum was separated and the serum levels of TC and TG were detected.

The experimental data was expressed in X±s, and the differences between groups were determined by t-test, with p<0.05 and p<0.01 indicating a siginificant difference and a very significant difference, respectively.

### 3. The experimental results are shown in Table 25 and Table 26 below:

**Table 25. Hypoglycemic acitivity of the composition of the polypeptides and amino aicd**

| Mice group | Blood glucose level after one week | Blood glucose level after two weeks | Blood glucose level after three weeks | Blood glucose level after four weeks |
|---|---|---|---|---|
| Normal control group | 5.3±0.2 | 5.0±0.3 | 5.2±0.2 | 5.2±0.2 |
| Model control group | 13.1±0.6 | 14.1±0.6 | 14.7±0.4 | 15.8±0.9 |
| Metformin group | 12.6±0.7 | 13.5±0.7 | 12.3±1.0 ## | 8.0±0.7 ## |
| Composition F group | 12.4±0.8 | 9.1±0.9 ## ^{∗∗} | 8.7±0.5 ## ^{∗∗} | 7.2±0.6 ## |
| Composition E group | 13.9±1.0 # | 8.5±0.5 ##^{∗∗}△ | 5.7±0.5 ## ^{∗∗}△△ | 4.9±0.3 ## ^{∗∗}△△ |

| | | | | |
|---|---|---|---|---|
| Note: #, ## represents the comparison with model control group having significant difference (p<0.05) and very significant difference (p<0.01), respectively; ^{∗}, ^{∗∗} represents the comparisons with metformin group having significant difference (p<0.05) and very significant difference (p<0.01), respectively; and △, △△ represents the comparison with composition E group and composition F group having significant difference (p<0.05) and very significant difference (p<0.01), respectively. | | | | |

**Table 26. Hypolipidemic acitivity of the composition of the polypeptides and amino aicd**

| Group | TC (mmol/L) | TG (mmol/L) |
|---|---|---|
| Normal control group | 2.21±1.22 | 2.11±0.95 |
| Model control group | 2.96±2.31 | 3.78±1.24 |
| Metformin group | 2.32±0.78 | 1.87±1.26 ** |
| Composition F group | 2.53±1.80 | 1.95±1.38 ^{∗∗} |
| Composition E group | 1.84±0.72 ^{∗∗} △ | 1.48±0.37 ^{∗∗} △ |

| | | |
|---|---|---|
| Note: ^{∗} represents the comparisons with metformin group having significant difference (p<0.05) and very significant difference (p<0.01), respectively; and △, △△ represent the comparisons with composition E group and composition F group having significant difference (p<0.05) and very significant difference (p<0.01), respectively. | | |

### 4. Conclusion

The above results showed that for the diabetic mouse models with pancreatic damage induced by STZ, metformin at ultra-high-dose (1000 mg/kg, a dose 10 times of the commonly used dose) could not control blood glucose effectively, but the polypeptides of the invention when used alone had better effect than that of metformin. Moreover, the compositions comprising the polypeptides of the invention and amino acids enabled the mice to reach normal blood glucose level after being treated for four weeks. The compositions comprising the polypeptides of the invention and amino acids exhibited synergistic effect, and could controll blood glucose in diabetic mice with impaired pancreases more effectively than the polypeptides of the invention alone.

Meanhwile, the polypeptides of the invention could significantly lower blood lipid level in the diabetic mouse models with pancreatic damage. Furthermore, the compositions comprising the polypeptides of the invention and amino acid had a better effect of lowering blood lipid level in the diabetic mouse models with pancreatic damage than the polypeptides alone, indicating a synergistic effect of the combination of the polypeptides of the invention and amino acid.

### Example 25 Hypolipidemic acitivity of the composition comprising the polypeptides of the invention and amino aicd

### 1. Materials and Instruments

Polypeptide composition E: a solid composition was prepared according to formulation ④ in Example 23, stored at 40±2°C/75±5%RH for 6 months, and then prepared into a solution containing 2 mg/mL polypeptide O with normal saline for use.

High-fat diet: 78.8% basic diet, 1% cholesterol, 10% egg yolk powder, 10% lard, and 0.2% bile salt.

Experimental animals: male SD rats, 50 rats, weighing 200 ± 2 g, were purchased from Hubei Epidemic Prevention Station.

Biochemical analyzer and assay kits: cholesterol (TC) assay kits, triglyceride (TG) assay kits and high-density lipoprotein cholesterol (HDL-C) assay kits were purchased from Mindray Bio-medical Electronics Co., Ltd. and tested with Mindray BS-220 Automatic Biochemical Analyzer.

### 2. Experimental method

After adapting to the environment for 3 days, the rats were fed with normal diet for 5 days. After being fasted for 16 hours, blood samples were collected from the tail and centrifuged (3000 rpm) to give serum, and then TC, TG and HDL-C in serum were measured. According to the TC level, the rats were randomly divided into five groups, 10 rats in each group, i.e., three treatment groups (administered with 10, 20 and 30 mg/kg, respectively), one high-fat diet control group and one normal diet control group. During the experimental period, the rats of the three treatment groups were given high-fat diet and administered by gavage with the solid composition of the invention comprising the polypeptides and amino acid at different doses. The rats of the high-fat diet control group were given high-fat diet and water, and the rats of the normal diet control group were given normal diet and water. On day 30 of the experiment, after being fasted for 16 hours, blood samples were collected from the tail and centrifuged (3000 rpm) to give serum, and then TC, TG and HDL-C in serum were measured.

The experimental data was expressed in X±s, and the differences between groups were determined by t-test, with p<0.05 and p<0.01 indicating a siginificant difference and a very significant difference, respectively.

### 3. The experimental results are shown in Table 27 below:

**Table 27. Hypolipidemic acitivity of the composition comprising the polypeptides and amino aicd**

| Group | TC (mmol/L) | | TG(mmol/L) | | HDL-C(mmol/L) | |
|---|---|---|---|---|---|---|
| | Pre-test | Post-test | Pre-test | Post-test | Pre-test | Post-test |
| Normal control group | 1.72±0.24 | 1.88±0.32 | 1.32±0.21 | 1.23±0.38 | 0.56±0.09 | 0.52±0.13 |
| High fat control group | 1.75±0.32 | 2.14±0.12 # | 1.29±0.16 | 1.88±0.13 # | 0.63±0.14 | 0.35±0.08 # |
| High-dose group | 1.74±0.19 | 0.91±0.17 ## ^{∗∗} | 1.22±0.09 | 0.65±0.24 ## ^{∗∗} | 0.55±0.09 | 0.88±0.11 ## ^{∗∗} |
| Middle-dose group | 1.73±0.27 | 1.54±0.35 # ^{∗∗} | 1.27±0.12 | 0.86±0.15 # ^{∗∗} | 0.58±0.21 | 0.73±0.10 ## ^{∗∗} |
| Low-dose group | 1.77±0.26 | 1.68±0.26 * | 1.26±0.33 | 1.12±0.28 * | 0.57±0.12 | 0.62±0.14 * |

| | | | | | | |
|---|---|---|---|---|---|---|
| Note: #, ## represents the comparison with normal control group having significant difference (p<0.05) and very significant difference (p<0.01), respectively; and ^{∗}, ^{∗∗} represents the comparison with high-fat diet control group having significant difference (p<0.05) and very significant difference (p<0.01), respectively. | | | | | | |

### 4. Conclusion

From the above results, it could be seen that the rats of the three treatment groups were fed with high-fat diet and at the same time administered with the composition comprising the polypeptides of the invention and amino acid at different doses. As compared with the rats of the high-fat diet group, the SD rats of the high, medium and low dose groups had significantly lower total serum cholesterol (TC) and triglyceride (TG) level, and had significantly higher high density lipoprotein cholesterol (HDL-C) level. The above results indicated that the compositions comprising the polypeptides of the invention and amino acid had explicit improving effect on the blood lipids in hyperlipidemia rats.

### Example 26 Improvement in blood glucose level and glycosylated hemoglobin (HbA1c) level in type 2 diabetic patients after the single oral administration of the composition comprising the polypeptides of the invention and amino aciding

A female patient, Ms. Jiang, 58 years old, was diagnosed with diabetes in hospital on January 19, 2018, with a fasting blood glucose level up to 15.32 mmol/L and glycosylated hemoglobin of 9.7%. From January 25, 2018, she started orally taking the single solid composition as prepared according to formulation ④ in Example 22, before meal, once a day, 100 mg every time, not taking other hypoglycemic agents. On February 25, 2018, she went to the hospital for a check, and the fasting blood glucose decreased to 7.3 mmol/L and the glycosylated hemoglobin decreased to 6.7%. In only one month, the blood glucose level was effectively controlled and the glycosylated hemoglobin level was essentially returned to normal.

The example showed that for patients with early diabetes, blood glucose level could be effectively controlled, and glycosylated hemoglobin levels could be returned to normal by orally taking the single composition comprising the polypeptides of the invention and amino acid.

### Example 27 Improvement in glycosylated hemoglobin (HbA1c) level in type 2 diabetic patients after the administration of the composition comprising the polypeptides of the invention and amino acid in combination with metformin

A male patient, Mr. Wang, 40 years old, with a family history of diabetes, was diagnosed with diabetes in hospital two years ago, and took a tablet of 0.5 g metformin orally every day. His fasting blood glucose level remained between 8.0 and 10.0 for a long time, but the blood glucose level was not effectively controlled. On January 7, 2018, his glycosylated hemoglobin level was 7.5% as shown in a test in the hospital. From January 8, 2018, he started orally taking the solid composition as prepared according to formulation ④ in Example 22, before meal, three times a day, 100 mg every time, without changing the dosage regimen of metformin. On March 1, 2018, he went to the hospital for a check, and the glycosylated hemoglobin level was 6.9%, which was essentially normal.

The example showed that when the composition comprising the polypeptides of the invention and amino acid was used in combination with metformin, the glycosylated hemoglobin level of the patient was obviously improved and tended to be normal and the effect was better than that of metformin when used alone.

### Example 28 Improvement in glycosylated hemoglobin (HbA1c) level in type 2 diabetic patients after the administration of the composition comprising the polypeptides of the invention and amino acid in combination with metformin, insulin

A male patient, Mr. Yuan, 66 years old, with a history of diabetes for 20 years, had been injected with insulin from year to year, 50 units of short-acting insulin per day, and took a tablet of 0.5 g metformin orally every night. However, his blood glucose level could not be effectively controlled, and the fasting blood glucose level remained between 12.0 and 15.0 for a long time. On November 22, 2017, his glycosylated hemoglobin level was 12.9% as shown in a test in the hospital. From November 22, 2017, he started orally taking the solid composition as prepared according to formulation ④ in Example 22, before meal, three times a day, 100 mg every time, without changing the dosage regimen of metformin and insulin. After continuous administration for two months, the fasting blood glucose level fluctuated between 5.0 and 7.0. Then he stopped taking the above composition comprising the polypeptides and amino acid and changed the injection dose of insulin to 25 units, and the fasting blood glucose level remained stable between 5.0 and 7.0. On March 3, 2018, he went to the hospital for a check, and the glycosylated hemoglobin level was 7.0%, which was essentially normal.

The example showed that for patients whose blood glucose level could still not be effectively controlled by using metformin and insulin, the composition comprising the polypeptides of the invention and amino acid in combination with metformin and insulin could effectively improve the blood glucose level, return the glycosylated hemoglobin level to normal, reduce the dosage of insulin, and achieve an effect better than that of the use of metformin and insulin alone.

After taking the composition comprising the polypeptides of the invention and amino acid for some time, even if the patient stopped it and reduced the insulin dose by half, his fasting blood glucose and glycosylated hemoglobin level could still be controlled within a normal range. It indicated that the composition comprising the polypeptides of the invention and amino acid had the function of repairing the damaged pancreases of diabetic patients and promoting the recovery of insulin-secretion, and thus could reverse type 2 diabetes.

### Example 29 Improvement in glycolipid metabolism in type 2 diabetic patients after the administration of the composition comprising the polypeptides of the invention and amino aicd

A male patient, Mr. Xu, 49 years old, was diagnosed with diabetes in hospital on April 1, 2018, with a fasting blood glucose level up to 14.61 mmol/L and glycosylated hemoglobin of 10.5%. From January 5, 2018, he started orally taking the single solid composition as prepared according to formulation ④ in Example 22, before meal, three times a day, 100 mg every time, not taking other hypoglycemic agents. On March 17, 2018, he went to the hospital for a check, the fasting blood glucose decreased to 8.32 mmol/L, the glycosylated hemoglobin decreased to 7.7%, and the triglyceride (TG) decreased to 2.6 mmol/L. In two months, the fasting blood glucose level, glycosylated hemoglobin level and triglyceride (TG) level were reduced by 43%, 27% and 42%, respectively, which changed significantly.

The example showed that the composition comprising the polypeptides and amino aicd could significantly improve glycolipid metabolism in type 2 diabetic patients.

The embodiments of the invention are described above.

## Claims

1. One polypeptide of the sequence as shown in the following general formula (I) or a mixture of two or more polypeptides of the sequences as shown in the following general formula (I) in any proportion for use in lowering blood glucose and/or blood lipid level in a mammal, or preventing and/or treating diabetes and/or hyperlipidemia in a mammal:
**X₁SCNGVCSPFX₂X₃PPCGX₄X₅X₆CRCIPX₇GLX₈X₉GX₁₀CRNPX₁₁G** (I)
wherein, X₁-X₁₁ is independently selected from the group consisting of A, C, D, E, F, G, H, I, K, L, M, N, P, Q, R, S, T, V, W, Y and Mₒₓ, whereby
said mammal is preferably a human being, and
said diabetes is preferably type 2 diabetes.

2. One polypeptide of the sequence as shown in the following general formula (I) or a mixtures comprising two or more polypeptides of the sequences as shown in the following general formula (I) in any proportion, in combination with other hypoglycemic agents and/or hypolipidemic agents, for use in lowering blood glucose and/or blood lipid level in a mammal, or preventing and/or treating diabetes and/or hyperlipidemia in a mammal:
**X₁SCNGVCSPFX₂X₃PPCGX₄X₅X₆CRCIPX₇GLX₈X₉GX₁₀CRNPX₁₁G** (I)
wherein, X₁-X₁₁ is independently selected from the group consisting of A, C, D, E, F, G, H, I, K, L, M, N, P, Q, R, S, T, V, W, Y and Mₒₓ, wherein
said mammal is preferably a human being, and
said diabetes is preferably type 2 diabetes.

3. An oral pharmaceutical composition or formulation, food, health food or formula food for special medical purposes for use in medicine, comprising one polypeptide of the sequence as shown in the following general formula (I) or a mixture of two or more polypeptides of the sequences as shown in the following general formula (I) in any proportion, and pharmaceutically acceptable carriers:
**X₁SCNGVCSPFX₂X₃PPCGX₄X₅X₆CRCIPX₇GLX₈X₉GX₁₀CRNPX₁₁G** (I)
wherein, X₁-X₁₁ is independently selected from the group consisting of A, C, D, E, F, G, H, I, K, L, M, N, P, Q, R, S, T, V, W, Y and Mₒₓ, wherein
the oral pharmaceutical composition or formulation, food, health food or formula food for special medical purposes preferably further comprises other hypoglycemic agents and/or hypolipidemic agents,
said other hypoglycemic agents and/or hypolipidemic agents preferably are selected from oral hypoglycemic agents and/or hypolipidemic agents.

4. A pharmaceutical composition or preparation for intravenous, intramuscular or subcutaneous injection for use in medicine, comprising one polypeptide of the sequence as shown in the following general formula (I) or a mixture of two or more polypeptides of the sequences as shown in the following general formula (I) in any proportion, and pharmaceutically acceptable carriers:
**X₁SCNGVCSPFX₂X₃PPCGX₄X₅X₆CRCIPX₇GLX₈X₉GX₁₀CRNPX₁₁G** (I)
wherein, X₁-X₁₁ is independently selected from the group consisting of A, C, D, E, F, G, H, I, K, L, M, N, P, Q, R, S, T, V, W, Y and Mₒₓ, wherein
the pharmaceutical composition or preparation preferably further comprises other hypoglycemic agents and/or hypolipidemic agents,
said other hypoglycemic agents preferably are selected from insulin and/or incretin.

5. A composition, comprising one polypeptide of the sequence as shown in general formula (I) or a mixture of two or more polypeptides of the sequences as shown in general formula (I) in any proportion, and methionine:
**X₁SCNGVCSPFX₂X₃PPCGX₄X₅X₆CRCIPX₇GLX₈X₉GX₁₀CRNPX₁₁G** (I)
wherein, X₁-X₁₁ is independently selected from the group consisting of A, C, D, E, F, G, H, I, K, L, M, N, P, Q, R, S, T, V, W, Y and Mₒₓ, wherein
the methionine preferably is in D-form, L-form or DL-form, more preferably L-form,
the polypeptide and methionine preferably are at a weight ratio in the range of 100:1-1:100, more preferably 50:1-1:50, further preferably 50:1-1:2, most preferably 20:1-10:1,
said composition preferably further comprises recombinant human serum albumin and/or bovine serum albumin with the content thereof in the range of 0.5-50 mg/ml, more preferably 1-20 mg/ml, most preferably 5-10 mg/ml, in a liquid composition, or in the range of 0.5-50 mg/g, more preferably 1-20 mg/g, most preferably 5-10 mg/g, in a solid composition,
said composition preferably further comprises sodium chloride with the content thereof in the range of 1-20 mg/ml, more preferably 5-15 mg/ml, most preferably 8-12 mg/ml, in a liquid composition, or in the range of 1-20 mg/g, more preferably 5-15 mg/g, most preferably 8-12 mg/g, in a solid composition,
said composition preferably further comprises ethylenediaminetetraacetic acid disodium salt with the content thereof in the range of 0.05-0.1 mg/ml in a liquid composition or in the range of 0.05-0.2 mg/g in a solid composition,
said composition preferably further comprises sodium acetate and acetic acid with the content of sodium acetate in the range of 0.5-3 mg/ml, more preferably 1-2.5 mg/ml, most preferably 1.5-2 mg/ml, in a liquid composition,
said composition preferably further comprises one, two or more selected from the group consisting of glycerol, mannitol and sorbitol with the content thereof in the range of 0.5-100 mg/ml, more preferably 1-50 mg/ml, most preferably 8-15 mg/ml, in a liquid composition, or in the range of 0.5-100 mg/g, more preferably 1-50 mg/g, most perferably 8-15 mg/g, in a solid composition,
said composition preferably is a liquid composition with the pH in the range of 3.5-6.5, more preferably 4-5, most preferably 4.5,
said composition preferably is a solid composition with the pH of a 5% solution of the composition in the range of 3.5-6.5, more preferably 4-5, most preferably 4.5,
said composition preferably is a liquid composition, comprising:
(a) one polypeptide of the sequence as shown in general formula (I) or a mixture of two or more polypeptides of the sequences as shown in general formula (I) in any proportion;
(b) methionine;
(c) sodium acetate;
(d) acetic acid;
(e) glycerol; and
(f) water for injection, wherein
said composition preferably is a solid composition, comprising:
(a) one polypeptide of the sequence as shown in general formula (I) or a mixture of two or more polypeptides of the sequences as shown in general formula (I) in any proportion;
(b) methionine;
(c) D-mannitol; and
(d) ethylenediaminetetraacetic acid disodium salt. Preferably, said solid composition further comprises sodium chloride.

6. The composition of claim 5 for use in lowering blood glucose and/or blood lipid level in a mammal, or preventing and/or treating diabetes and/or hyperlipidemia in a mammal, wherein
said mammal preferably is a human being, and
said diabetes preferably is type 2 diabetes.

7. The composition of claim 5, in combination with other hypoglycemic agents and/or hypolipidemic agents, for use in lowering blood glucose and/or blood lipid level in a mammal, or preventing and/or treating diabetes and/or hyperlipidemia in a mammal, wherein
said mammal preferably is a human being, and
said diabetes preferably is type 2 diabetes.

8. An oral pharmaceutical preparation, food, health food or formula food for special medical purposes for use in medicine, comprising the composition of claim 5 and pharmaceutically acceptable carriers, wherein
the oral pharmaceutical preparation, food, health food or formula food for special medical purposes preferably further comprises other hypoglycemic agents and/or hypolipidemic agents,
said other hypoglycemic agents and/or hypolipidemic agents preferably are selected from oral hypoglycemic agents and/or hypolipidemic agents.

9. A pharmaceutical composition or preparation for intravenous, intramuscular or subcutaneous injection for use in medicine, comprising the composition of claim 5 and pharmaceutically acceptable carriers, wherein
said pharmaceutical composition or preparation preferably further comprises other hypoglycemic agents and/or hypolipidemic agents,
said other hypoglycemic agents preferably are selected from insulin and/or incretin.

10. The polypeptide, pharmaceutical composition or preparation, food, health food or formula food for special medical purposes, composition of any one of claims 1-9, wherein the blood glucose level of said mammal cannot be effectively controlled by using metformin, sulfonylurea secretion enhancers, thiazolidinediones, α-glucodidase inhibitors, insulin or derivatives thereof, insulin or derivatives or combinations thereof and/or said mammal has a glycosylated hemoglobin value >7.0% and preferably >7.5% before administration and a glycosylated hemoglobin value <7.5% and preferably <7.0% after administration.

11. The polypeptide, pharmaceutical composition or preparation, food, health food or formula food for special medical purposes, composition of any one of claims 1-10, **characterized in that**, said polypeptide has the sequence as shown in general formula (I), wherein
X₁ preferably is a hydrophobic amino acid, selected from the group consisting of A, L, V, I, P, F, M, W, and Mₒₓ;
X₂ is an acidic amino acid, selected from E and D;
X₃ is a hydrophobic amino acid, selected from the group consisting of A, L, V, I, P, F, M, W, and Mₒₓ;
X₄ is a polar neutral amino acid except cysteine, selected from the group consisting of G, S, T, Y, N, and Q;
X₅ is a neutral amino acid except cysteine, selected from the group consisting of G, S, T, Y, N, Q, A, L, V, I, P, F, M, and W;
X₆ is a hydrophobic amino acid, selected from the group consisting of A, L, V, I, P, F, M, W, and Mₒₓ;
X₇ is a neutral amino acid except cysteine, selected from the group consisting of G, S, T, Y, N, Q, A, L, V, I, P, F, M, and W;
X₈ is a hydrophobic amino acid, selected from the group consisting of A, L, V, V, I, P, F, M, W, and Mₒₓ;
X₉ is a hydrophobic amino acid, selected from the group consisting of A, L, V, I, P, F, M, W, and Mₒₓ;
X₁₀ is selected from the group consisting of Y, K, and N;
X₁₁ is a polar neutral amino acid except cysteine, selected from the group consisting of G, S, T, Y, N, and Q,
more preferably, X₁ is A, I or V; X₂ is E or D; X₃ is M, Mₒₓ or I; X₄ is S or T; X₅ is S or P; X₆ is A or L; X₇ is V, A or Y; X₈ is V, F or L; X₉ is V or I; X₁₀ is Y, K or N; X₁₁ is S, T or Y,
more preferably, X₂X₃ is one selected from the group consisting of DI, EI, DM, EM, DMₒₓ, and EMₒₓ; X₄X₅X₆ is one selected from the group consisting of SPL, SPA, SSL, SSA, TPL, TPA, TSL and TSA; X₈X₉ is one selected from the group consisting of VI, VV, FI, FV, LI, and LV,
more preferably, said polypeptide is one having the following sequence or a mixture of two or more polypeptides having the following sequences in any proportion:
ASCNGVCSPFEMPPCGTSACRCIPVGLVVGYCRNPSG,
ASCNGVCSPFEMₒₓPPCGTSACRCIPVGLVVGYCRNPSG,
ASCNGVCSPFEMPPCGTSACRCIPVGLVIGYCRNPSG,
ASCNGVCSPFEMₒₓPPCGTSACRCIPVGLVIGYCRNPSG,
ASCNGVCSPFEMPPCGSSACRCIPVGLLIGYCRNPSG,
ASCNGVCSPFEMₒₓPPCGSSACRCIPVGLLIGYCRNPSG,
ASCNGVCSPFEMPPCGTSACRCIPVGLFIGYCRNPSG,
ASCNGVCSPFEMₒₓPPCGTSACRCIPVGLFIGYCRNPSG,
ASCNGVCSPFEMPPCGTSACRCIPYGLFIGYCRNPSG,
ASCNGVCSPFDIPPCGSPLCRCIPVGLVIGKCRNPYG,
ISCNGVCSPFDIPPCGSPLCRCIPAGLVIGNCRNPYG,
VSCNGVCSPFDIPPCGSPLCRCIPAGLVIGKCRNPYG,
ASCNGVCSPFDIPPCGTPLCRCIPVGLVIGNCRNPYG,
ASCNGVCSPFDMPPCGS SACRCIPVGLFIGNCRNPYG,
ASCNGVCSPFDMPPCGSSACRCIPVGLVIGYCRNPYG,
ASCNGVCSPFDMPPCGSSLCRCIPAGLVVGYCRNPSG,
ASCNGVCSPFDMPPCGTSACRCIPAGLFIGKCRNPYG,
ASCNGVCSPFDMPPCGTSACRCIPVGLVVGYCRNPSG,
ASCNGVCSPFDMPPCGTSLCRCIPAGLFIGYCRNPSG,
ASCNGVCSPFDMPPCGTSLCRCIPAGLVIGYCRNPYG,
ASCNGVCSPFEIPPCGSPLCRCIPVGLVIGNCRNPYG,
ASCNGVCSPFEIPPCGTPLCRCIPAGLVIGKCRNPYG,
ASCNGVCSPFEIPPCGTPLCRCIPVGLFIGKCRNPSG,
ASCNGVCSPFEMPPCGSSACRCIPAGLFIGKCRNPYG,
ASCNGVCSPFEMPPCGSSACRCIPVGLFVGYCRNPYG,
ASCNGVCSPFEMPPCGSSACRCIPVGLVVGYCRNPSG,
ASCNGVCSPFEMPPCGSSLCRCIPAGLFIGYCRNPSG,
ASCNGVCSPFEMPPCGSSLCRCIPAGLVIGYCRNPSG,
ASCNGVCSPFEMPPCGTSACRCIPAGLFIGNCRNPYG,
ASCNGVCSPFEMPPCGTSACRCIPAGLVIGYCRNPYG,
ISCNGVCSPFDIPPCGSPACRCIPVGLVIGKCRNPYG,
ISCNGVCSPFDIPPCGSPLCRCIPAGLVIGNCRNPSG,
ISCNGVCSPFDIPPCGSPLCRCIPVGLFIGNCRNPSG,
ISCNGVCSPFDIPPCGSPLCRCIPVGLVIGYCRNPSG,
ISCNGVCSPFDIPPCGTPACRCIPVGLVIGNCRNPYG,
ISCNGVCSPFDIPPCGTPLCRCIPAGLFVGKCRNPYG,
ISCNGVCSPFDIPPCGTPLCRCIPAGLFIGKCRNPSG,
ISCNGVCSPFDMPPCGSPACRCIPAGLLIGYCRNPSG,
ISCNGVCSPFDMPPCGTSACRCIPAGLVIGYCRNPSG,
ISCNGVCSPFEIPPCGSPLCRCIPAGLFIGKCRNPSG,
ISCNGVCSPFEIPPCGTPACRCIPVGLFIGKCRNPSG,
ISCNGVCSPFEIPPCGTPACRCIPAGLVIGKCRNPYG,
ISCNGVCSPFEIPPCGSPACRCIPVGLVIGNCRNPYG,
ISCNGVCSPFEIPPCGSSLCRCIPAGLLVGKCRNPSG,
ISCNGVCSPFEIPPCGTPLCRCIPAGLFIGNCRNPSG,
ISCNGVCSPFEIPPCGTPLCRCIPAGLVIGYCRNPSG,
ISCNGVCSPFEIPPCGTSACRCIPAGLVIGYCRNPSG,
ISCNGVCSPFEMPPCGSSACRCIPAGLVIGYCRNPSG,
ISCNGVCSPFEMPPCGSSACRCIPAGLFIGYCRNPSG,
ISCNGVCSPFEMPPCGTSLCRCIPAGLVVGYCRNPSG,
VSCNGVCSPFDIPPCGTPLCRCIPYGLFVGNCRNPYG,
VSCNGVCSPFDIPPCGTPACRCIPYGLFVGNCRNPYG,
VSCNGVCSPFDIPPCGTPLCRCIPAGLVIGNCRNPYG,
VSCNGVCSPFDIPPCGTPLCRCIPVGLFIGNCRNPSG,
VSCNGVCSPFDIPPCGTPLCRCIPVGLVIGYCRNPSG,
VSCNGVCSPFDIPPCGSPLCRCIPVGLFIGKCRNPSG,
VSCNGVCSPFDMPPCGS SACRCIP AGLFIGNCRNPYG,
VSCNGVCSPFDMPPCGSSACRCIPAGLVIGYCRNPYG,
VSCNGVCSPFDMPPCGSSACRCIPVGLVIGYCRNPSG,
VSCNGVCSPFDMPPCGSSACRCIPVGLFIGYCRNPSG,
VSCNGVCSPFDMPPCGTSACRCIPAGLFIGYCRNPSG,
VSCNGVCSPFEIPPCGSPACRCIPVGLVIGKCRNPYG,
VSCNGVCSPFEIPPCGSPLCRCIP AGLVIGNCRNPYG,
VSCNGVCSPFEIPPCGSPLCRCIPVGL VIGYCRNPSG,
VSCNGVCSPFEIPPCGTPACRCIPVGLVIGNCRNPYG,
VSCNGVCSPFEIPPCGSPLCRCIPVGLFIGNCRNPSG,
VSCNGVCSPFEIPPCGTPLCRCIP AGLFIGKCRNPSG,
VSCNGVCSPFEMPPCGSSACRCIPYGL VVGNCRNPSG,
VSCNGVCSPFEMPPCGTPLCRCIPYGLLIGKCRNPYG,
VSCNGVCSPFEMPPCGTSACRCIPAGLVIGYCRNPSG,
VSCNGVCSPFEMPPCGTSACRCIPAGLFIGYCRNPSG,
more preferably, said polypeptide comprises three pairs of disulfide bonds in the peptide chain, with one disulfide bond formed between two cysteines at positions 3 and 20, one disulfide bond formed between two cysteines at positions 7 and 22, and one disulfide bond formed between two cysteines at positions 15 and 32 in the amino acid sequence,
more preferably, said mixture of polypeptides is an extract of the seeds of a leguminous plant or the by-products of the seeds after processing - bean meal,
said leguminous plant preferably is one selected from the group consisting of soybean, mung bean, pea, faba bean, red bean, cowpea, green bean, and lentil, or a mixture of two or more selected from said group in any proportion; said leguminou plant preferably is one selected from the group consisting of soybean, mung bean, and pea, or a mixture of two or more selected from the group in any proportion, more preferably said extract comprises the polypeptides having the following sequences:
ASCNGVCSPFEMPPCGTSACRCIPVGLVVGYCRNPSG,
ASCNGVCSPFEMₒₓPPCGTSACRCIPVGLVVGYCRNPSG,
ASCNGVCSPFEMPPCGTSACRCIPVGLVIGYCRNPSG,
ASCNGVCSPFEMₒₓPPCGTSACRCIPVGLVIGYCRNPSG,
ASCNGVCSPFEMPPCGSSACRCIPVGLLIGYCRNPSG,
ASCNGVCSPFEMₒₓPPCGSSACRCIPVGLLIGYCRNPSG,
ASCNGVCSPFEMPPCGTSACRCIPVGLFIGYCRNPSG,
ASCNGVCSPFEMₒₓPPCGTSACRCIPVGLFIGYCRNPSG,
ASCNGVCSPFEMPPCGTSACRCIPYGLFIGYCRNPSG,
ASCNGVCSPFDIPPCGSPLCRCIPVGL VIGKCRNPYG,
ISCNGVCSPFDIPPCGSPLCRCIPAGL VIGNCRNPYG,
VSCNGVCSPFDIPPCGSPLCRCIPAGLVIGKCRNPYG,
more preferably the total content of said polypeptides having said sequences is at least 5%, preferably at least 8%, more preferably at least 10%, more preferably at least 20%, more preferably at least 30%, more preferably at least 50%, more preferably at least 80%, further preferably at least 90%, further preferably at least 95%, further preferably at least 97%, and most preferably at least 98%.

## Patentansprüche

1. Ein Polypeptid der Sequenz wie in der folgenden allgemeinen Formel (I) gezeigt oder eine Mischung von zwei oder mehr Polypeptiden der Sequenzen wie in der folgenden allgemeinen Formel (I) gezeigt in beliebigem Verhältnis zur Verwendung beim Senken von Blutzucker- und/oder Blutfettspiegel bei einem Säugetier oder Prävention und/oder Behandlung von Diabetes und/oder Hyperlipidämie bei einem Säugetier:
**X₁SCNGVCSPFX₂X₃PPCGX₄X₅X₆CRCIPX₇GLX₈X₉GX₁₀CRNPX₁₁G** (I)
worin X₁-X₁₁ unabhängig ausgewählt aus der Gruppe bestehend aus A, C, D, E, F, G H, I, K, L, M, N, P, Q, R, S, T, V, W, Y und Mₒₓ ist, wobei
das Säugetier vorzugsweise ein Mensch ist, und
der Diabetes vorzugsweise Typ-2-Diabetes ist.

2. Ein Polypeptid der Sequenz wie in der folgenden allgemeinen Formel (I) gezeigt oder eine Mischung umfassend zwei oder mehr Polypeptide der Sequenzen wie in der folgenden allgemeinen Formel (I) gezeigt in beliebigem Verhältnis, in Kombination mit anderen hypoglykämischen Mitteln und/ oder hypolipidämische Mittel zur Verwendung beim Senken von Blutzucker- und/oder Blutfettspiegel bei einem Säugetier oder zur Prävention und/oder Behandlung von Diabetes und/oder Hyperlipidämie bei einem Säugetier:
**X₁SCNGVCSPFX₂X₃PPCGX₄X₅X₆CRCIPX₇GLX₈X₉GX₁₀CRNPX₁₁G** (I)
worin X₁-X₁₁ unabhängig ausgewählt aus der Gruppe bestehend aus A, C, D, E, F, G H, I, K, L, M, N, P, Q, R, S, T, V, W, Y und Mₒₓ ist, wobei
das Säugetier vorzugsweise ein Mensch ist, und
der Diabetes vorzugsweise Typ-2-Diabetes ist.

3. Eine orale pharmazeutische Zusammensetzung oder Formulierung, Lebensmittel, Reformkost oder Formelnahrung für besondere medizinische Zwecke zur Verwendung in der Medizin, umfassend ein Polypeptid der Sequenz wie in der folgenden allgemeinen Formel (I) gezeigt, oder eine Mischung aus zwei oder mehr Polypeptiden die Sequenzen wie in der folgenden allgemeinen Formel (I) gezeigt in beliebigem Verhältnis, und pharmazeutisch verträgliche Träger:
**X₁SCNGVCSPFX₂X₃PPCGX₄X₅X₆CRCIPX₇GLX₈X₉GX₁₀CRNPX₁₁G** (I)
worin X₁-X₁₁ unabhängig ausgewählt aus der Gruppe bestehend aus A, C, D, E, F, G H, I, K, L, M, N, P, Q, R, S, T, V, W, Y und Mₒₓ ist, wobei
die orale pharmazeutische Zusammensetzung oder Formulierung, Lebensmittel, Reformkost oder Formelnahrung für besondere medizinische Zwecke vorzugsweise weiterhin andere hypoglykämische Mittel und/oder hypolipidämische Mittel umfasst,
die anderen hypoglykämischen Mittel und/oder hypolipidämischen Mittel vorzugsweise ausgewählt aus oralen hypoglykämischen Mitteln und/oder hypolipidämischen Mitteln sind.

4. Eine pharmazeutische Zusammensetzung oder Zubereitung zur intravenösen, intramuskulären oder subkutanen Injektion zur Verwendung in der Medizin, umfassend ein Polypeptid der Sequenz wie in der folgenden allgemeinen Formel (I) gezeigt oder eine Mischung aus zwei oder mehr Polypeptiden der Sequenzen wie in der gezeigt der folgenden allgemeinen Formel (I) in beliebigem Verhältnis, und pharmazeutisch verträgliche Träger:
**X₁SCNGVCSPFX₂X₃PPCGX₄X₅X₆CRCIPX₇GLX₈X₉GX₁₀CRNPX₁₁G** (I)
worin X₁-X₁₁ unabhängig ausgewählt aus der Gruppe bestehend aus A, C, D, E, F, G H, I, K, L, M, N, P, Q, R, S, T, V, W, Y und Mₒₓ ist, wobei
die pharmazeutische Zusammensetzung oder Zubereitung vorzugsweise weiterhin andere hypoglykämische Mittel und/oder hypolipidämische Mittel umfasst,
die anderen hypoglykämischen Mittel vorzugsweise ausgewählt aus Insulin und/oder Inkretin sind.

5. Eine Zusammensetzung, umfassend ein Polypeptid der Sequenz wie in der allgemeinen Formel (I) gezeigt oder eine Mischung aus zwei oder mehr Polypeptiden der Sequenz wie in der allgemeinen Formel (I) gezeigt in beliebigem Verhältnis und Methionin:
**X₁SCNGVCSPFX₂X₃PPCGX₄X₅X₆CRCIPX₇GLX₈X₉GX₁₀CRNPX₁₁G** (I)
worin X₁-X₁₁ unabhängig ausgewählt aus der Gruppe bestehend aus A, C, D, E, F, G H, I, K, L, M, N, P, Q, R, S, T, V, W, Y und Mₒₓ ist, wobei
das Methionin liegt vorzugsweise in D-Form, L-Form oder DL-Form vor, stärker bevorzugt in L-Form,
das Polypeptid und Methionin liegen vorzugsweise in einem Gewichtsverhältnis im Bereich von 100:1-1:100, stärker bevorzugt 50:1-1:50, weiter bevorzugt 50:1-1:2, am meisten bevorzugt 20:1-10:1 vor,
die Zusammensetzung vorzugsweise weiterhin rekombinantes menschliches Serumalbumin und/oder Rinderserumalbumin mit einem Gehalt davon im Bereich von 0,5-50 mg/ml, stärker bevorzugt 1-20 mg/ml, am meisten bevorzugt 5-10 mg/ml in a flüssige Zusammensetzung oder im Bereich von 0,5-50 mg/g, stärker bevorzugt 1-20 mg/g, am meisten bevorzugt 5-10 mg/g in einer festen Zusammensetzung umfasst,
die Zusammensetzung vorzugsweise fernes Natriumchlorid mit einem Gehalt davon im Bereich von 1-20 mg/ml, stärker bevorzugt 5-15 mg/ml, am meisten bevorzugt 8-12 mg/ml in einer flüssigen Zusammensetzung oder im Bereich von 1-20 mg/g, stärker bevorzugt 5-15 mg/g, am stärksten bevorzugt 8-12 mg/g in einer festen Zusammensetzung umfasst,
die Zusammensetzung vorzugsweise ferner Ethylendiamintetraessigsäure-Dinatriumsalz mit einem Gehalt davon im Bereich von 0,05-0,1 mg/ml in einer flüssigen Zusammensetzung oder im Bereich von 0,05-0,2 mg/g in einer festen Zusammensetzung umfasst,
die Zusammensetzung vorzugsweise ferner Natriumacetat und Essigsäure mit einem Gehalt an Natriumacetat im Bereich von 0,5-3 mg/ml, stärker bevorzugt 1-2,5 mg/ml, am meisten bevorzugt 1,5-2 mg/ml in einer flüssigen Zusammensetzung umfasst,
die Zusammensetzung vorzugsweise ferner ein, zwei oder mehr ausgewählt aus der Gruppe bestehend aus Glycerol, Mannit und Sorbit, mit einem Gehalt davon im Bereich von 0,5-100 mg/ml, stärker bevorzugt 1-50 mg/ml, am meisten bevorzugt 8-15 mg/ml in einer flüssigen Zusammensetzung oder im Bereich von 0,5-100 mg/g, stärker bevorzugt 1-50 mg/g, am meisten bevorzugt 8-15 mg/g in einer festen Zusammensetzung umfasst,
die Zusammensetzung vorzugsweise eine flüssige Zusammensetzung mit einem pH-Wert im Bereich von 3,5-6,5, stärker bevorzugt 4-5, am meisten bevorzugt 4,5 ist,
die Zusammensetzung vorzugsweise eine feste Zusammensetzung mit dem pH-Wert einer 5%igen Lösung der Zusammensetzung im Bereich von 3,5-6,5, stärker bevorzugt 4-5, am meisten bevorzugt 4,5 ist,
die Zusammensetzung vorzugsweise eine flüssige Zusammensetzung ist, umfassend:
(a) ein Polypeptid der Sequenz wie in der allgemeinen Formel (I) gezeigt oder eine Mischung aus zwei oder mehr Polypeptiden der Sequenz wie in der allgemeinen Formel (I) gezeigt in beliebigem Verhältnis;
(b) Methionin;
(c) Natriumacetat;
(d) Essigsäure;
(e) Glycerin; und
(f) Wasser zur Injektion, wobei
die Zusammensetzung vorzugsweise eine feste Zusammensetzung ist, umfassend:
(a) ein Polypeptid der Sequenz wie in der allgemeinen Formel (I) gezeigt oder eine Mischung aus zwei oder mehr Polypeptiden der Sequenz wie in der allgemeinen Formel (I) gezeigt in beliebigem Verhältnis;
(b) Methionin;
(c) D-Mannit; und
(d) Ethylendiamintetraessigsäure-Dinatriumsalz, vorzugsweise die feste Zusammensetzung ferner Natriumchlorid umfasst.

6. Zusammensetzung nach Anspruch 5 zur Verwendung beim Senken von Blutzucker- und/oder Blutfettspiegel bei einem Säugetier oder zur Prävention und/oder Behandlung von Diabetes und/oder Hyperlipidämie bei einem Säugetier, wobei
das Säugetier vorzugsweise ein Mensch ist, und
der Diabetes vorzugsweise Typ-2-Diabetes ist.

7. Zusammensetzung nach Anspruch 5 in Kombination mit anderen hypoglykämischen Mitteln und/oder hypolipidämischen Mitteln zur Verwendung beim Senken von Blutzucker- und/oder Blutfettspiegel bei einem Säuger oder zur Prävention und/oder Behandlung von Diabetes und/oder Hyperlipidämie bei einem Säuger, wobei
das Säugetier vorzugsweise ein Mensch ist, und
der Diabetes vorzugsweise Typ-2-Diabetes ist.

8. Eine orale pharmazeutische Zubereitung, Lebensmittel, Reformkost oder Formelnahrung für besondere medizinische Zwecke zur Verwendung in der Medizin, umfassend die Zusammensetzung nach Anspruch 5 und pharmazeutisch verträgliche Träger, wobei
das orale pharmazeutische Zubereitung, Lebensmittel, Reformkost oder Formelnahrung für besondere medizinische Zwecke vorzugsweise weiterhin andere hypoglykämische Mittel und/oder hypolipidämische Mittel umfasst,
die anderen hypoglykämischen Mittel und/oder hypolipidämischen Mittel vorzugsweise ausgewählt aus oralen hypoglykämischen Mitteln und/oder hypolipidämischen Mitteln sind.

9. Pharmazeutische Zusammensetzung oder Zubereitung zur intravenösen, intramuskulären oder subkutanen Injektion zur Verwendung in der Medizin, umfassend die Zusammensetzung nach Anspruch 5 und pharmazeutisch verträgliche Träger, wobei
die pharmazeutische Zusammensetzung oder Zubereitung vorzugsweise ferner andere hypoglykämische Mittel und/oder hypolipidämische Mittel umfasst,
die anderen hypoglykämischen Mittel vorzugsweise ausgewählt aus Insulin und/oder Inkretin sind.

10. Polypeptid, pharmazeutische Zusammensetzung oder Zubereitung, Nahrungsmittel, Reformkost oder Formelnahrung für spezielle medizinische Zwecke, Zusammensetzung nach einem der Ansprüche 1-9, wobei der Blutzuckerspiegel des Säugers nicht wirksam durch die Verwendung von Metformin, Sulfonylharnstoff-Sekretionsverstärker, Thiazolidindione, α-Glukodidase-Inhibitoren, Insulin oder Derivate davon, Insulin oder Derivate oder Kombinationen davon kontrolliert werden kann und/oder der Säuger einen glykosylierten Hämoglobinwert von > 7,0 % und vorzugsweise > 7,5 % vor der Verabreichung und einen glykosylierten Hämoglobinwert von < 7,5 % und vorzugsweise aufweist < 7,0 % nach Verabreichung.

11. Polypeptid, pharmazeutische Zusammensetzung oder Zubereitung, Lebensmittel, Reformkost oder Formelnahrung für besondere medizinische Zwecke, Zusammensetzung nach einem der Ansprüche 1-10, **dadurch gekennzeichnet, dass** das Polypeptid die in der allgemeinen Formel (I) gezeigte Sequenz aufweist, worin
X₁ vorzugsweise eine hydrophobe Aminosäure, ausgewählt aus der Gruppe bestehend aus A, L, V, I, P, F, M, W und Mₒₓ, ist;
X₂ eine saure Aminosäure, ausgewählt aus E und D, ist;
X₃ eine hydrophobe Aminosäure, ausgewählt aus der Gruppe bestehend aus A, L, V, I, P, F, M, W und Mₒₓ, ist;
X₄ eine polare neutrale Aminosäure außer Cystein, ausgewählt aus der Gruppe bestehend aus G S, T, Y, N und Q, ist;
X₅ eine neutrale Aminosäure außer Cystein, ausgewählt aus der Gruppe bestehend aus G S, T, Y, N, Q, A, L, V, I, P, F, M und W, ist;
X₆ eine hydrophobe Aminosäure, ausgewählt aus der Gruppe bestehend aus A, L, V, I, P, F, M, W und Mₒₓ, ist;
X₇ eine neutrale Aminosäure außer Cystein, ausgewählt aus der Gruppe bestehend aus G S, T, Y, N, Q, A, L, V, I, P, F, M und W, ist;
X₈ eine hydrophobe Aminosäure, ausgewählt aus der Gruppe bestehend aus A, L, V, V, I, P, F, M, W und Mₒₓ, ist;
X₉ eine hydrophobe Aminosäure, ausgewählt aus der Gruppe bestehend aus A, L, V, I, P, F, M, W und Mₒₓ, ist;
X₁₀ ausgewählt aus der Gruppe bestehend aus Y, K und N ist;
X₁₁ eine polare neutrale Aminosäure außer Cystein, ausgewählt aus der Gruppe bestehend aus G S, T, Y, N und Q, ist,
stärker bevorzugt, X₁ A, I oder V ist; X₂ E oder D ist; X₃ M, M_{OX} oder I ist; X₄ S oder T ist; X₅ S oder P ist; X₆ A oder L ist; X₇ V, A oder Y ist; X₈ V, F oder L ist; X₉ V oder I ist; X₁₀ Y, K oder N ist; X₁₁ S, T oder Y ist,
stärker bevorzugt, X₂X₃ eines ausgewählt aus der Gruppe bestehend aus DI, EI, DM, EM, DMₒₓ und EMₒₓ ist; X₄X₅X₆ eines ausgewählt aus der Gruppe bestehend aus SPL, SPA, SSL, SSA, TPL, TPA, TSL und TSA ist; X₈X₉ eines ausgewählt aus der Gruppe bestehend aus VI, VV, FI, FV, LI und LV ist,
stärker bevorzugt, das Polypeptid eines mit der folgenden Sequenz oder eine Mischung aus zwei oder mehr Polypeptiden mit den folgenden Sequenzen in beliebigem Verhältnis ist:
ASCNGVCSPFEMPPCGTSACRCIPVGL VVGYCRNPSG,
ASCNGVCSPFEMₒₓPPCGTSACRCIPVGLVVGYCRNPSG,
ASCNGVCSPFEMPPCGTSACRCIPVGLVIGYCRNPSG,
ASCNGVCSPFEMₒₓPPCGTSACRCIPVGLVIGYCRNPSG,
ASCNGVCSPFEMPPCGSSACRCIPVGLLIGYCRNPSG,
ASCNGVCSPFEMₒₓPPCGSSACRCIPVGLLIGYCRNPSG,
ASCNGVCSPFEMPPCGTSACRCIPVGLFIGYCRNPSG,
ASCNGVCSPFEMₒₓPPCGTSACRCIPVGLFIGYCRNPSG,
ASCNGVCSPFEMPPCGTSACRCIPYGLFIGYCRNPSQ
ASCNGVCSPFDIPPCGSPLCRCIPVGLVIGKCRNPYQ
ISCNGVCSPFDIPPCGSPLCRCIPAGLVIGNCRNPYQ
VSCNGVCSPFDIPPCGSPCCRCIPAGLVIGKCRNPYG,
ASCNGVCSPFDIPPCGTPCCRCIPVGLVIGNCRNPYG,
ASCNGVCSPFDMPPCGSSACRCIPVGLFIGNCRNPYQ
ASCNGVCSPFDMPPCGSSACRCIPVGLVIGYCRNPYQ,
ASCNGVCSPFDMPPCGSSLCRCIPAGLVVGYCRNPSG,
ASCNGVCSPFDMPPCGTSACRCIPAGLFIGKCRNPYQ,
ASCNGVCSPFDMPPCGTSACRCIPVGLVVGYCRNPSQ,
ASCNGVCSPFDMPPCGTSLCRCIPAGLFIGYCRNPSG,
ASCNGVCSPFDMPPCGTSLCRCIPAGLVIGYCRNPYQ,
ASCNGVCSPFEIPPCGSPLCRCIPVGLVIGNCRNPYQ,
ASCNGVCSPFEIPPCGTPLCRCIPAGLVIGKCRNPYQ,
ASCNGVCSPFEIPPCGTPLCRCIPVGLFIGKCRNPSG,
ASCNGVCSPFEMPPCGSSACRCIPAGLFIGKCRNPYG,
ASCNGVCSPFEMPPCGSSACRCIPVGLFVGYCRNPYG,
ASCNGVCSPFEMPPCGSSACRCIPVGLVVGYCRNPSG,
ASCNGVCSPFEMPPCGSSLCRCIPAGLFIGYCRNPSG,
ASCNGVCSPFEMPPCGSSLCRCIPAGLVIGYCRNPSG,
ASCNGVCSPFEMPPCGTSACRCIPAGLFIGNCRNPYQ,
ASCNGVCSPFEMPPCGTSACRCIPAGLVIGYCRNPYG,
ISCNGVCSPFDIPPCGSPACRCIPVGLVIGKCRNPYQ,
ISCNGVCSPFDIPPCGSPLCRCIPAGLVIGNCRNPSQ,
ISCNGVCSPFDIPPCGSPLCRCIPVGLFIGNCRNPSQ,
ISCNGVCSPFDIPPCGSPLCRCIPVGLVIGYCRNPSG,
ISCNGVCSPFDIPPCGTPACRCIPVGLVIGNCRNPYG,
ISCNGVCSPFDIPPCGTPLCRCIPAGLFVGKCRNPYQ,
ISCNGVCSPFDIPPCGTPLCRCIPAGLFIGKCRNPSQ,
ISCNGVCSPFDMPPCGSPACRCIPAGLLIGYCRNPSG,
ISCNGVCSPFDMPPCGTSACRCIPAGLVIGYCRNPSG,
ISCNGVCSPFEIPPCGSPLCRCIPAGLFIGKCRNPSQ,
ISCNGVCSPFEIPPCGTPACRCIPVGLFIGKCRNPSG,
ISCNGVCSPFEIPPCGTPACRCIPAGLVIGKCRNPYG,
ISCNGVCSPFEIPPCGSPACRCIPVGLVIGNCRNPYQ,
ISCNGVCSPFEIPPCGSSLCRCIPAGLLVGKCRNPSG,
ISCNGVCSPFEIPPCGTPLCRCIPAGLFIGNCRNPSQ,
ISCNGVCSPFEIPPCGTPLCRCIPAGLVIGYC,
ISCNGVCSPFEIPPCGTSACRCIPAGLVIGYC,
ISCNGVCSPFEMPPCGSSACRCIPAGLVIGYCRNPSQ,
ISCNGVCSPFEMPPCGSSACRCIPAGLFIGYCRNPSG,
ISCNGVCSPFEMPPCGTSLCRCIPAGLVVGYCRNPSG,
VSCNGVCSPFDIPPCGTPLCRCIPYGLFVGNCRNPYG,
VSCNGVCSPFDIPPCGTPACRCIPYGLFVGNCRNPYG,
VSCNGVCSPFDIPPCGTPCCRCIPAGLVIGNCRNPYG,
VSCNGVCSPFDIPPCGTPLCRCIPVGLFIGNCRNPSQ,
VSCNGVCSPFDIPPCGTPLCRCIPVGLVIGYCRNPSQ,
VSCNGVCSPFDIPPCGSPLCRCIPVGLFIGKC,
VSCNGVCSPFDMPPCGSSACRCIPAGLFIGNCRNPYG,
VSCNGVCSPFDMPPCGSSACRCIPAGLVIGYCRNPYG,
VSCNGVCSPFDMPPCGSSACRCIPVGLVIGYCRNPSG,
VSCNGVCSPFDMPPCGSSACRCIPVGLFIGYCRNPSG,
VSCNGVCSPFDMPPCGTSACRCIPAGLFIGYCRNPSG,
VSCNGVCSPFEIPPCGSPACRCIPVGLVIGKCRNPYG,
VSCNGVCSPFEIPPCGSPCCRCIPAGLVIGNCRNPYG,
VSCNGVCSPFEIPPCGSPLCRCIPVGLVIGYCRNPSQ,
VSCNGVCSPFEIPPCGTPACRCIPVGLVIGNCRNPYG,
VSCNGVCSPFEIPPCGSPLCRCIPVGLFIGNCRNPSQ,
VSCNGVCSPFEIPPCGTPLCRCIPAGLFIGKCRNPSQ,
VSCNGVCSPFEMPPCGSSACRCIPYGLVVGNCRNPSG,
VSCNGVCSPFEMPPCGTPLCRCIPYGLLIGKCRNPYQ,
VSCNGVCSPFEMPPCGTSACRCIPAGLVIGYCRNPSG,
VSCNGVCSPFEMPPCGTSACRCIPAGLFIGYCRNPSG,
stärker bevorzugt, das Polypeptid drei Disulfidbindungspaare in der Peptidkette umfasst, wobei eine Disulfidbindung zwischen zwei Cysteinen an den Positionen 3 und 20 gebildet wird, eine Disulfidbindung zwischen zwei Cysteinen an den Positionen 7 und 22 gebildet wird und eine Disulfidbindung zwischen zwei Cysteine an den Positionen 15 und 32 in der Aminosäuresequenz gebildet wird,
stärker bevorzugt, die Mischung von Polypeptiden ein Extrakt der Samen einer Leguminosenpflanze oder der Nebenprodukte der Samen nach der Verarbeitung - Bohnenmehl ist,
die Leguminosenpflanze vorzugsweise eine ausgewählt aus der Gruppe bestehend aus Sojabohne, Mungobohne, Erbse, Ackerbohne, rote Bohne, Kuherbse, grüne Bohne und Linse, oder eine Mischung aus zwei oder mehreren ausgewählt aus der Gruppe in beliebigem Verhältnis ist; die Leguminosenpflanze vorzugsweise eine ausgewählt aus der Gruppe bestehend aus Sojabohne, Mungobohne und Erbse oder eine Mischung aus zwei oder mehreren ausgewählt aus der Gruppe in beliebigem Verhältnis ist, stärker bevorzugt der Extrakt die Polypeptide mit den folgenden Sequenzen umfasst:
ASCNGVCSPFEMPPCGTSACRCIPVGLVVGYCRNPSQ,
ASCNGVCSPFEMₒₓPPCGTSACRCIPVGLVVGYCRNPSG,
ASCNGVCSPFEMPPCGTSACRCIPVGLVIGYCRNPSQ,
ASCNGVCSPFEMₒₓPPCGTSACRCIPVGLVIGYCRNPSG,
ASCNGVCSPFEMPPCGSSACRCIPVGLLIGYCRNPSG,
ASCNGVCSPFEMₒₓPPCGSSACRCIPVGLLIGYCRNPSG,
ASCNGVCSPFEMPPCGTSACRCIPVGLFIGYCRNPSG,
ASCNGVCSPFEMₒₓPPCGTSACRCIPVGLFIGYCRNPSG,
ASCNGVCSPFEMPPCGTSACRCIPYGLFIGYCRNPSQ,
ASCNGVCSPFDIPPCGSPLCRCIPVGLVIGKCRNPYQ,
ISCNGVCSPFDIPPCGSPLCRCIPAGLVIGNCRNPYQ,
VSCNGVCSPFDIPPCGSPCCRCIPAGLVIGKCRNPYG,
stärker bevorzugt, der Gesamtgehalt der Polypeptide mit den Sequenzen mindestens 5 %, vorzugsweise mindestens 8 %, stärker bevorzugt mindestens 10 %, stärker bevorzugt mindestens 20 %, stärker bevorzugt mindestens 30 %, stärker bevorzugt mindestens 50 %, stärker bevorzugt mindestens 80 %, stärker bevorzugt mindestens 90 %, stärker bevorzugt mindestens 95 %, stärker bevorzugt mindestens 97 % und am meisten bevorzugt mindestens 98 % beträgt.

## Revendications

1. Un polypeptide de la séquence indiquée dans la formule générale (I) suivante ou un mélange de deux ou plusieurs polypeptides des séquences indiquées dans la formule générale (I) suivante dans n'importe quelle proportion destiné à être utilisé pour abaisser la glycémie et/ou le taux de lipides sanguins chez un mammifère, ou prévenir et/ou traiter le diabète et/ou l'hyperlipidémie chez un mammifère:
**X₁SCNGVCSPFX₂X₃PPCGX₄X₅X₆CRCIPX₇GLX₈X₉GX₁₀CRNPX₁₁G** (I)
où, X₁-X₁₁ sont sélectionnés indépendamment dans le groupe constitué de A, C, D, E, F, G H, I, K, L, M, N, P, Q, R, S, T, V, W, Y et Mₒₓ, dans lequel
ledit mammifère est de préférence un être humain, et
ledit diabète est de préférence un diabète de type 2.

2. Un polypeptide de la séquence indiquée dans la formule générale (I) suivante ou un mélange comprenant deux ou plusieurs polypeptides des séquences indiquées dans la formule générale (I) suivante dans n'importe quelle proportion, en combinaison avec d'autres agents hypoglycémiants et/ ou des agents hypolipidémiants, destiné à être utilisé pour abaisser la glycémie et/ou le taux de lipides sanguins chez un mammifère, ou prévenir et/ou traiter le diabète et/ou l'hyperlipidémie chez un mammifère:
**X₁SCNGVCSPFX₂X₃PPCGX₄X₅X₆CRCIPX₇GLX₈X₉GX₁₀CRNPX₁₁G** (I)
où, X₁-X₁₁ sont sélectionnés indépendamment dans le groupe constitué de A, C, D, E, F, G H, I, K, L, M, N, P, Q, R, S, T, V, W, Y et Mₒₓ, dans lequel
ledit mammifère est de préférence un être humain, et
ledit diabète est de préférence un diabète de type 2.

3. Une composition ou formulation pharmaceutique orale, un aliment, un aliment diététique ou un aliment de formule à des fins médicales spéciales destinée à être utilisée en médecine, comprenant un polypeptide de la séquence indiquée dans la formule générale (I) suivante ou un mélange de deux ou plusieurs polypeptides des séquences indiquées dans la formule générale (I) suivante dans n'importe quelle proportion, et des supports pharmaceutiquement acceptables:
**X₁SCNGVCSPFX₂X₃PPCGX₄X₅X₆CRCIPX₇GLX₈X₉GX₁₀CRNPX₁₁G** (I)
où, X₁-X₁₁ sont sélectionnés indépendamment dans le groupe constitué de A, C, D, E, F, G H, I, K, L, M, N, P, Q, R, S, T, V, W, Y et Mₒₓ, dans laquelle
la composition ou formulation pharmaceutique orale, l'aliment, l'aliment diététique ou l'aliment de formule à des fins médicales spéciales de préférence comprend en outre d'autres agents hypoglycémiants et/ou agents hypolipidémiants,
lesdits autres agents hypoglycémiants et/ou agents hypolipidémiants sont de préférence choisis parmi les agents hypoglycémiants et/ou les agents hypolipidémiants oraux.

4. Une composition ou préparation pharmaceutique pour injection intraveineuse, intramusculaire ou sous-cutanée destinée à être utilisée en médecine, comprenant un polypeptide de la séquence indiquée dans la formule générale (I) suivante ou un mélange de deux ou plusieurs polypeptides des séquences indiquées dans la suivant la formule générale (I) suivante dans n'importe quelle proportion, et les supports pharmaceutiquement acceptables:
**X₁SCNGVCSPFX₂X₃PPCGX₄X₅X₆CRCIPX₇GLX₈X₉GX₁₀CRNPX₁₁G** **(I)**
où, X₁-X₁₁ sont sélectionnés indépendamment dans le groupe constitué de A, C, D, E, F, G H, I, K, L, M, N, P, Q, R, S, T, V, W, Y et Mₒₓ, dans laquelle
la composition ou préparation pharmaceutique de préférence comprend en outre d'autres agents hypoglycémiants et/ou agents hypolipidémiants,
lesdits autres agents hypoglycémiants sont de préférence choisis parmi l'insuline et/ou l'incrétine.

5. Une composition comprenant un polypeptide de la séquence indiquée dans la formule générale (I) ou un mélange de deux ou plusieurs polypeptides des séquences indiquées dans la suivant la formule générale (I) dans n'importe quelle proportion, et de la méthionine :
**X₁SCNGVCSPFX₂X₃PPCGX₄X₅X₆CRCIPX₇GLX₈X₉GX₁₀CRNPX₁₁G** **(I)**
où, X₁-X₁₁ sont sélectionnés indépendamment dans le groupe constitué de A, C, D, E, F, G H, I, K, L, M, N, P, Q, R, S, T, V, W, Y et Mₒₓ, dans laquelle
la méthionine est de préférence sous forme D, sous forme L ou sous forme DL, plus préférablement sous forme L,
le polypeptide et la méthionine sont de préférence à un rapport pondéral dans la gamme de 100:1-1:100, plus préférablement 50:1-1:50, encore plus préférablement 50:1-1:2, le plus préférablement 20:1-10:1,
ladite composition de préférence comprend en outre de l'albumine sérique humaine recombinante et/ou de l'albumine sérique bovine dont la teneur se situe dans la gamme de 0,5 à 50 mg/ml, plus préférablement 1 à 20 mg/ml, le plus préférablement 5 à 10 mg/ml, dans une composition liquide, ou dans la gamme de 0,5 à 50 mg/g, plus préférablement 1 à 20 mg/g, le plus préférablement 5 à 10 mg/g, dans une composition solide,
ladite composition de préférence comprend en outre du chlorure de sodium dont la teneur se situe dans la gamme de 1-20 mg/ml, plus préférablement 5-15 mg/ml, le plus préférablement 8-12 mg/ml, dans une composition liquide, ou dans la gamme de 1-20 mg/g, plus préférablement 5-15 mg/g, le plus préférablement 8-12 mg/g, dans une composition solide,
ladite composition de préférence comprend en outre un sel disodique d'acide éthylènediaminetétraacétique dont la teneur se situe dans la gamme de 0,05 à 0,1 mg/ml dans une composition liquide ou dans la gamme de 0,05 à 0,2 mg/g dans une composition solide,
ladite composition de préférence comprend en outre de l'acétate de sodium et de l'acide acétique avec une teneur en acétate de sodium dans la gamme de 0,5 à 3 mg/ml, plus préférablement 1 à 2,5 mg/ml, le plus préférablement 1,5 à 2 mg/ml, dans une composition liquide.
ladite composition de préférence comprend en outre un, deux ou plus choisis dans le groupe constitué de glycérol, de mannitol et de sorbitol avec leur teneur dans la gamme de 0,5 à 100 mg/ml, plus préférablement 1 à 50 mg/ml, le plus préférablement 8 à 15 mg/ml, dans une composition liquide, ou dans la gamme de 0,5 à 100 mg/g, plus préférablement 1 à 50 mg/g, le plus préférablement 8 à 15 mg/g, dans une composition solide,
ladite composition est de préférence une composition liquide avec un pH dans la gamme de 3,5 à 6,5, plus préférablement 4 à 5, le plus préférablement 4,5,
ladite composition est de préférence une composition solide avec le pH d'une solution à 5 % de la composition dans la gamme de 3,5 à 6,5, plus préférablement 4 à 5, le plus préférablement 4,5,
ladite composition est de préférence une composition liquide, comprenant:
(a) un polypeptide de la séquence indiquée dans la formule générale (I) ou un mélange de deux ou plusieurs polypeptides des séquences indiquées dans la formule générale (I) dans n'importe quelle proportion;
(b) méthionine;
(c) acétate de sodium;
(d) acide acétique;
(e) glycérol; et
(f) de l'eau pour injection, dans laquelle
ladite composition est de préférence une composition solide, comprenant :
(a) un polypeptide de la séquence indiquée dans la formule générale (I) ou un mélange de deux ou plusieurs polypeptides des séquences indiquées dans la formule générale (I) dans n'importe quelle proportion;
(b) méthionine;
(c) D-mannitol; et
(d) le sel disodique de l'acide éthylènediaminetétracétique, de préférence, ladite composition solide comprend en outre du chlorure de sodium.

6. La composition selon la revendication 5 destinée à être utilisée pour pour abaisser la glycémie et/ou le taux de lipides sanguins chez un mammifère, ou prévenir et/ou traiter le diabète et/ou l'hyperlipidémie chez un mammifère, dans laquelle:
ledit mammifère est de préférence un être humain, et
ledit diabète est de préférence un diabète de type 2.

7. La composition selon la revendication 5, en combinaison avec d'autres agents hypoglycémiants et/ ou des agents hypolipidémiants, destiné à être utilisé pour abaisser la glycémie et/ou le taux de lipides sanguins chez un mammifère, ou prévenir et/ou traiter le diabète et/ou l'hyperlipidémie chez un mammifère, dans laquelle
ledit mammifère est de préférence un être humain, et
ledit diabète est de préférence un diabète de type 2.

8. Une préparation pharmaceutique orale, un aliment, un aliment diététique ou un aliment de formule à des fins médicales spéciales destinée à être utilisée en médecine, comprenant la composition de la revendication 5 et des supports pharmaceutiquement acceptables, dans laquelle
la préparation pharmaceutique orale, l'aliment, l'aliment diététique ou l'aliment de formule à des fins médicales spéciales de préférence comprend en outre d'autres agents hypoglycémiants et/ou agents hypolipidémiants,
lesdits autres agents hypoglycémiants et/ou agents hypolipidémiants sont de préférence choisis parmi les agents hypoglycémiants et/ou les agents hypolipidémiants oraux.

9. Une composition ou préparation pharmaceutique pour injection intraveineuse, intramusculaire ou sous-cutanée destinée à être utilisée en médecine, comprenant la composition de la revendication 5 et des supports pharmaceutiquement acceptables, dans laquelle
la composition ou préparation pharmaceutique de préférence comprend en outre d'autres agents hypoglycémiants et/ou agents hypolipidémiants,
lesdits autres agents hypoglycémiants sont de préférence choisis parmi l'insuline et/ou l'incrétine.

10. Le polypeptide, la composition ou préparation pharmaceutique, l'aliment, l'aliment diététique ou l'aliment de formule à des fins médicales spéciales, la composition selon l'une quelconque des revendications 1 à 9, dans lequel la glycémie dudit mammifère ne peut pas être efficacement contrôlé en utilisant metformine, promoteurs de sécrétion de sulfonylurée, thiazolidinediones, inhibiteurs de l'a-glucodidase, l'insuline ou ses dérivés, l'insuline ou ses dérivés ou leurs combinaisons, et/ou ledit mammifère a une valeur d'hémoglobine glycosylée > 7,0 % et de préférence > 7,5 % avant administration et une valeur d'hémoglobine glycosylée < 7,5 % et de préférence <7,0 % après administration.

11. Le polypeptide, la composition ou préparation pharmaceutique, l'aliment, l'aliment diététique ou l'aliment de formule à des fins médicales spéciales, la composition selon l'une quelconque des revendications 1 à 10, **caractérisé en ce que** ledit polypeptide a la séquence indiquée dans la formule générale (I), dans lequel
X₁ est de préférence un acide aminé hydrophobe, choisi dans le groupe constitué de A, L, V, I, P, F, M, W et Mₒₓ;
X₂ est un acide aminé acide, choisi parmi E et D;
X₃ est un acide aminé hydrophobe, choisi dans le groupe constitué de A, L, V, I, P, F, M, W et Mₒₓ;
X₄ est un acide aminé neutre polaire à l'exception de la cystéine, choisi dans le groupe constitué de G S, T, Y, N et Q;
X₅ est un acide aminé neutre à l'exception de la cystéine, choisi dans le groupe constitué de G S, T, Y, N, Q, A, L, V, I, P, F, M et W;
X₆ est un acide aminé hydrophobe, choisi dans le groupe constitué de A, L, V, I, P, F, M, W et Mₒₓ;
X₇ est un acide aminé neutre à l'exception de la cystéine, choisi dans le groupe constitué de G S, T, Y, N, Q, A, L, V, I, P, F, M et W;
X₈ est un acide aminé hydrophobe, choisi dans le groupe constitué de A, L, V, V, I, P, F, M, W et Mₒₓ;
X₉ est un acide aminé hydrophobe, choisi dans le groupe constitué de A, L, V, I, P, F, M, W et Mₒₓ ;
X₁₀ est choisi dans le groupe constitué de Y, K et N ;
X₁₁ est un acide aminé neutre polaire à l'exception de la cystéine, choisi dans le groupe constitué de G S, T, Y, N et Q,
plus préférablement, X₁ est A, I ou V; X₂ est E ou D; X₃ est M, Mₒₓ ou I; X₄ est S ou T; X₅ est S ou P; X₆ est A ou L; X₇ est V, A ou Y; X₈ est V, F ou L; X₉ est V ou I; X₁₀ est Y, K ou N; X₁₁ est S, T ou Y,
plus préférablement, X₂X₃ est choisi dans le groupe constitué de DI, EI, DM, EM, DMₒₓ et EMₒₓ; X₄X₅X₆ est sélectionné dans le groupe constitué de SPL, SPA, SSL, SSA, TPL, TPA, TSL et TSA; X₈X₉ est sélectionné dans le groupe constitué de VI, VV, FI, FV, LI et LV,
plus préférablement, ledit polypeptide est un polypeptide ayant la séquence suivante ou un mélange de deux ou plusieurs polypeptides ayant les séquences suivantes dans n'importe quelle proportion:
ASCNGVCSPFEMPPCGTSACRCIPVGLVVGYCRNPSQ
ASCNGVCSPFEMₒₓPPCGTSACRCIPVGLVVGYCRNPSG,
ASCNGVCSPFEMPPCGTSACRCIPVGL VIGYCRNPSG,
ASCNGVCSPFEMₒₓPPCGTSACRCIPVGLVIGYCRNPSG,
ASCNGVCSPFEMPPCGSSACRCIPVGLLIGYCRNPSG,
ASCNGVCSPFEMₒₓPPCGSSACRCIPVGLLIGYCRNPSG,
ASCNGVCSPFEMPPCGTSACRCIPVGLFIGYCRNPSG,
ASCNGVCSPFEMₒₓPPCGTSACRCIPVGLFIGYCRNPSG,
ASCNGVCSPFEMPPCGTSACRCIPYGLFIGYCRNPSQ
ASCNGVCSPFDIPPCGSPLCRCIPVGLVIGKCRNPYG,
ISCNGVCSPFDIPPCGSPLCRCIPAGLVIGNCRNPYQ,
VSCNGVCSPFDIPPCGSPCCRCIPAGLVIGKCRNPYG,
ASCNGVCSPFDIPPCGTPCCRCIPVGLVIGNCRNPYG,
ASCNGVC SPFDMPPCGSSACRCIPVGLFIGNCRNPYQ,
ASCNGVC SPFDMPPCGSSACRCIPVGLVIGYCRNPYQ,
ASCNGVCSPFDMPPCGSSLCRCIPAGLVVGYCRNPSG,
ASCNGVCSPFDMPPCGTSACRCIPAGLFIGKCRNPYG,
ASCNGVCSPFDMPPCGTSACRCIPVGLVVGYCRNPSG,
ASCNGVCSPFDMPPCGTSLCRCIPAGLFIGYCRNPSG,
ASCNGVCSPFDMPPCGTSLCRCIPAGLVIGYCRNPYG,
ASCNGVCSPFEIPPCGSPLCRCIPVGLVIGNCRNPYG,
ASCNGVCSPFEIPPCGTPCCRCIPAGLVIGKCRNPYG,
ASCNGVCSPFEIPPCGTPLCRCIPVGLFIGKCRNPSG,
ASCNGVC SPFEMPPCGSSACRCIPAGLFIGKCRNPYG,
ASCNGVC SPFEMPPCGSSACRCIPVGLFVGYCRNPYG,
ASCNGVCSPFEMPPCGSSACRCIPVGLVVGYCRNPSG,
ASCNGVCSPFEMPPCGSSLCRCIPAGLFIGYCRNPSG,
ASCNGVCSPFEMPPCGSSLCRCIPAGLVIGYCRNPSG,
ASCNGVCSPFEMPPCGTSACRCIPAGLFIGNCRNPYG,
ASCNGVCSPFEMPPCGTSACRCIPAGLVIGYCRNPYG,
ISCNGVCSPFDIPPCGSPACRCIPVGLVIGKCRNPYG,
ISCNGVCSPFDIPPCGSPLCRCIPAGLVIGNCRNPSG,
ISCNGVCSPFDIPPCGSPLCRCIPVGLFIGNCRNPSG,
ISCNGVCSPFDIPPCGSPLCRCIPVGLVIGYCRNPSG,
ISCNGVCSPFDIPPCGTPACRCIPVGLVIGNCRNPYG,
ISCNGVCSPFDIPPCGTPLCRCIPAGLFVGKCRNPYG,
ISCNGVCSPFDIPPCGTPLCRCIPAGLFIGKCRNPSG,
ISCNGVCSPFDMPPCGSPACRCIPAGLLIGYCRNPSG,
ISCNGVCSPFDMPPCGTSACRCIPAGLVIGYCRNPSG,
ISCNGVCSPFEIPPCGSPLCRCIPAGLFIGKCRNPSG,
ISCNGVCSPFEIPPCGTPACRCIPVGLFIGKC,
ISCNGVCSPFEIPPCGTPACRCIPAGLVIGKCRNPYG,
ISCNGVCSPFEIPPCGSPACRCIPVGLVIGNCRNPYG,
ISCNGVCSPFEIPPCGSSLCRCIPAGLLVGKCRNPSG,
ISCNGVCSPFEIPPCGTPLCRCIPAGLFIGNCRNPSG,
ISCNGVCSPFEIPPCGTPLCRCIPAGLVIGYCRNPSG,
ISCNGVCSPFEIPPCGTSACRCIPAGLVIGYCRNPSG,
ISCNGVCSPFEMPPCGSSACRCIPAGLVIGYCRNPSG,
ISCNGVCSPFEMPPCGSSACRCIPAGLFIGYCRNPSG,
ISCNGVCSPFEMPPCGTSLCRCIPAGLVVGYCRNPSG,
VSCNGVCSPFDIPPCGTPLCRCIPYGLFVGNCRNPYG,
VSCNGVCSPFDIPPCGTPACRCIPYGLFVGNCRNPYG,
VSCNGVCSPFDIPPCGTPCCRCIPAGLVIGNCRNPYG,
VSCNGVCSPFDIPPCGTPLCRCIPVGLFIGNCRNPSG,
VSCNGVCSPFDIPPCGTPLCRCIPVGLVIGYCRNPSG,
VSCNGVCSPFDIPPCGSPLCRCIPVGLFIGKCRNPSG,
VSCNGVCSPFDMPPCGSSACRCIPAGLFIGNCRNPYG,
VSCNGVCSPFDMPPCGSSACRCIPAGLVIGYCRNPYG,
VSCNGVCSPFDMPPCGSSACRCIPVGLVIGYCRNPSG,
VSCNGVCSPFDMPPCGSSACRCIPVGLFIGYCRNPSG,
VSCNGVCSPFDMPPCGTSACRCIPAGLFIGYCRNPSG,
VSCNGVCSPFEIPPCGSPACRCIPVGLVIGKCRNPYG,
VSCNGVCSPFEIPPCGSPCCRCIPAGLVIGNCRNPYG,
VSCNGVCSPFEIPPCGSPLCRCIPVGLVIGYCRNPSG,
VSCNGVCSPFEIPPCGTPACRCIPVGLVIGNCRNPYG,
VSCNGVCSPFEIPPCGSPLCRCIPVGLFIGNCRNPSG,
VSCNGVCSPFEIPPCGTPLCRCIPAGLFIGKCRNPSG,
VSCNGVCSPFEMPPCGSSACRCIPYGLVVGNCRNPSG,
VSCNGVCSPFEMPPCGTPLCRCIPYGLLIGKCRNPYG,
VSCNGVCSPFEMPPCGTSACRCIPAGLVIGYCRNPSG,
VSCNGVCSPFEMPPCGTSACRCIPAGLFIGYCRNPSG,
plus préférentiellement, ledit polypeptide comprend trois paires de ponts disulfure dans la chaîne peptidique, avec un pont disulfure formé entre deux cystéines aux positions 3 et 20, un pont disulfure formé entre deux cystéines en positions 7 et 22, et un pont disulfure formé entre deux cystéines aux positions 15 et 32 dans la séquence d'acides aminés,
plus préférentiellement, ledit mélange de polypeptides est un extrait des graines d'une plante légumineuse ou les sous-produits des graines après traitement - tourteau de haricot,
ladite plante légumineuse est de préférence choisie dans le groupe constitué de le soja, le haricot mungo, le pois, la féverole, le haricot rouge, le niébé, le haricot vert et la lentille, ou un mélange de deux ou plus choisis dans ledit groupe dans n'importe quelle proportion; ladite plante légumineuse est de préférence choisie dans le groupe constitué de le soja, le haricot mungo et le pois, ou un mélange de deux ou plus choisis dans le groupe dans n'importe quelle proportion, plus préférablement ledit extrait comprend les polypeptides ayant les séquences suivantes :
ASCNGVCSPFEMPPCGTSACRCIPVGLVVGYCRNPSG,
ASCNGVCSPFEMₒₓPPCGTSACRCIPVGLVVGYCRNPSG,
ASCNGVCSPFEMPPCGTSACRCIPVGL VIGYCRNPSG,
ASCNGVCSPFEMₒₓPPCGTSACRCIPVGLVIGYCRNPSG,
ASCNGVCSPFEMPPCGSSACRCIPVGLLIGYCRNPSG,
ASCNGVCSPFEMₒₓPPCGSSACRCIPVGLLIGYCRNPSG,
ASCNGVCSPFEMPPCGTSACRCIPVGLFIGYCRNPSG,
ASCNGVCSPFEMₒₓPPCGTSACRCIPVGLFIGYCRNPSG,
ASCNGVCSPFEMPPCGTSACRCIPYGLFIGYCRNPSQ
ASCNGVCSPFDIPPCGSPLCRCIPVGLVIGKCRNPYQ
ISCNGVCSPFDIPPCGSPLCRCIPAGLVIGNCRNPYQ
VSCNGVCSPFDIPPCGSPCCRCIPAGLVIGKCRNPYG,
plus préférablement la teneur totale desdits polypeptides ayant lesdites séquences est d'au moins 5%, de préférence d'au moins 8%, plus préférablement d'au moins 10%, plus préférablement d'au moins 20%, plus préférablement d'au moins 30%, plus préférablement d'au moins 50%, plus préférablement au moins 80%, encore plus préférablement au moins 90%, encore plus préférablement au moins 95%, encore plus préférablement au moins 97%, et le plus préférablement au moins 98%.
